# EUROPEAN PATENT APPLICATION

(11) **EP 4 286 408 A1**
(43) Date of publication of application: **06.12.2023**
(21) Application number: 21915801.1
(22) Date of filing: 29.12.2021
(51) Int. Cl.: C07K 16/00

(54) **BI- OR MULTI-SPECIFIC ANTIBODY**

(30) Priority: 29.12.2020 KR 20200185664
(71) Applicant: Samsung Biologics Co., Ltd., Incheon 21987 (KR)
(72) Inventor: LEE, Dongheon, Incheon 22021 (KR); YANG, Wonjun, Yongin-si Gyeonggi-do 16866 (KR); KIM, Jungwon, Incheon 21986 (KR); JUNG, Woosuk, Seoul 05698 (KR); PARK, Juhyun, Namyangju-si Gyeonggi-do 12233 (KR); MOON, Seungtae, Gunpo-si Gyeonggi-do 15862 (KR); KIM, Jina, Incheon 21316 (KR); LEE, Sangho, Incheon 21036 (KR); KIM, Migyeom, Seoul 01775 (KR); SONG, Hyundong, Incheon 22009 (KR); CHOI, Hyungseok, Yongin-si Gyeonggi-do 17000 (KR)
(74) Representative: Mewburn Ellis LLP
(86) International application number: PCT/KR2021/020103
(87) International publication number: WO 2022/146003

(57) **Abstract**

The present invention relates to a bi- or multi-specific antibody in a novel format comprising a polypeptide in which a CH3 dimer has been introduced into a portion of a Fab region. The bi- or multi-specific antibody in the novel format forms heterodimers while exhibiting almost no non-specific binding between heavy chains and light chains, and produces almost no homodimers, and thus can be highly expressed through animal cells. In addition, the bi- or multi-specific antibody can be obtained through a purification process of an existing monoclonal antibody, and has stability equal to or higher than that of the monoclonal antibody.

## Description

### [Technical Field]

The present invention relates to a novel bispecific or multispecific antibody and relates to a bispecific or multispecific antibody including a polypeptide having a CH3 dimer introduced into a portion of a Fab region.

### [Background Art]

As various causes and mechanisms of one indication have recently been identified, approaches from one type of target to multiple types of targets are emerging in the development of therapeutic agents. Accordingly, in the development of therapeutic agents using antibodies, various studies have been conducted for several decades to impart the characteristics of monospecific antibodies to bispecific or multispecific mechanisms that can specifically bind to two or more antigenic proteins.

Most bioantibody drugs are composed of a single target antibody backbone, such as IgG1 or IgG4. However, such bioantibody drugs have limitations such as efficacy, convenience, and side effects as antibody therapeutic agents for a single target, since mechanisms for various targets act on diseases.

At present, various clinical stages focus on combination therapy of two monoclonal antibodies. Accordingly, there is a need to develop antibodies that allow one protein to bind to two or more targets.

Several research papers associated therewith have been published in the last 20 years or so. Currently, there are a small number of licensed bispecific antibodies, a large number of candidate materials are researched/developed in the clinical stage, and examples of licensed bispecific antibodies include Removab, Blincyto (BiTE^{™}), and Hemlibra.

However, there is still a need for improvement of production yield, productivity, solubility, aggregation, and stability of the developed bi- or multi-target antibodies.

Under this technical background, as a result of extended efforts to develop a novel format of bispecific or multispecific antibodies, the present inventors developed a bispecific or multispecific antibody including a polypeptide having a CH3 dimer introduced into the Fab region. Based thereon, the present invention was completed.

### [Disclosure]

### [Technical Problem]

Therefore, the present invention has been made in view of the above problems, and it is an object of the present invention to provide a novel bi- or multi-specific antibody platform.

### [Technical Solution]

In accordance with one aspect of the present invention, the above and other objects can be accomplished by the provision of a bispecific antibody including a first arm binding to a first antigen and including VH1-CHa-Fc1 and VL1-CLb, and a second arm binding to a second antigen and including VH2-CH1-Fc2 and VL2-CL,
wherein the VH1 and the VH2 are each heavy-chain variable regions including the same or different antigen-binding regions,
the VL1 and the VL2 are each light-chain variable regions including the same or different antigen-binding regions,
the CHa includes i) an IgG heavy-chain constant region or an IgD heavy-chain constant region CH1, and an IgG heavy-chain constant region CH2 or CH3, or ii) an IgM heavy-chain constant region CH3,
the CLb includes i) a CL1 including an IgG light-chain constant region λ or x, and at least one selected from the group consisting of IgG heavy-chain constant regions CH1, CH2, and CH3, or ii) an IgM heavy-chain constant region CH3,
the CHa and the CLb are linked to each other to form a dimer,
the CH1 is an IgG heavy-chain constant region CH1 and the CL is an IgG light-chain constant region CL, and
the Fc1 of the first arm is linked to the Fc2 of the second arm to form a heavy-chain constant region dimer.

In accordance with another aspect of the present invention, provided is a bispecific antibody including a first arm binding to a first antigen and including VH1-CHa-Fc1 and VL1-CLb, and a second arm binding to a second antigen and including VH2-CH1-Fc2 and VL2-CL,
wherein the VH1 and the VH2 are each heavy-chain variable regions including the same or different antigen-binding regions,
the VL1 and the VL2 are each light-chain variable regions including the same or different antigen-binding regions,
the CHa includes an IgG heavy-chain constant region CH3 and an IgG heavy-chain constant region CH1,
the CLb includes a CL1 including an IgG light-chain constant region λ or x, and an IgG heavy-chain constant region CH3,
the CHa and the CLb are linked to each other to form a dimer,
the CH1 is an IgG heavy-chain constant region CH1 and the CL is an IgG light-chain constant region CL, and
the Fc1 of the first arm is linked to the Fc2 of the second arm to form a heavy-chain constant region dimer.

In accordance with another aspect of the present invention, provided is a multispecific antibody including the bispecific antibody.

### [Description of Drawings]

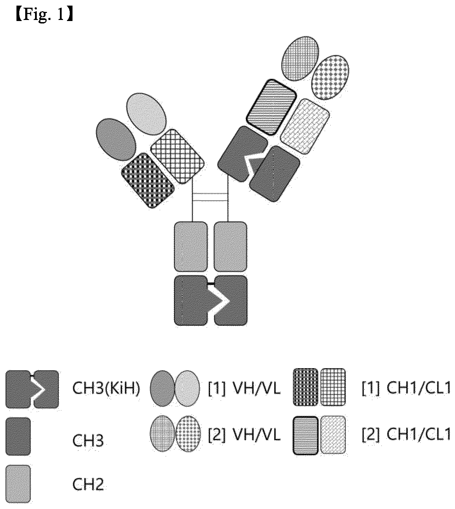

FIG. 1 illustrates the structure of a first bispecific antibody candidate.

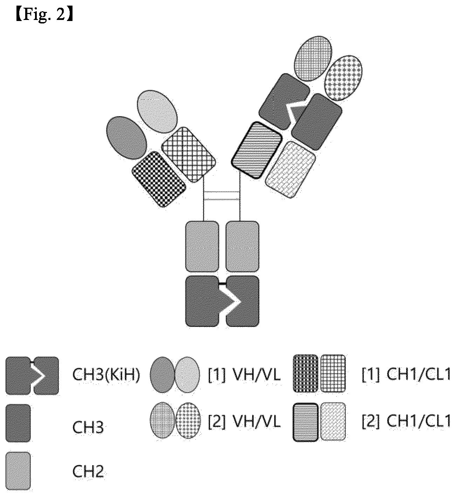

FIG. 2 illustrates the structure of a second bispecific antibody candidate.

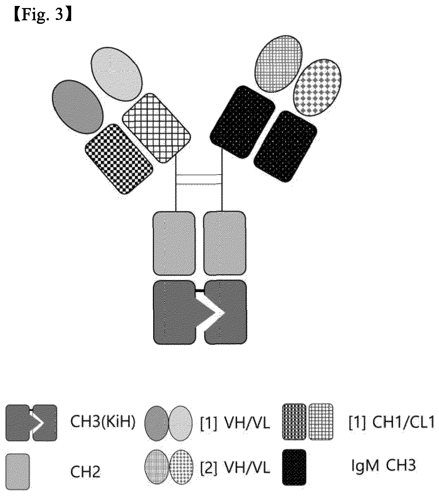

FIG. 3 illustrates the structure of a third bispecific antibody candidate.

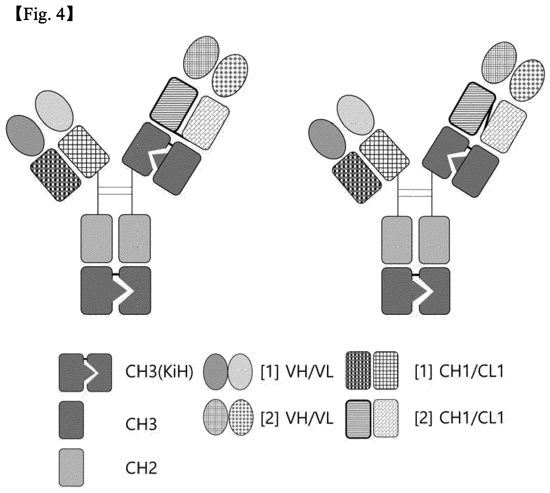

FIG. 4 illustrates the structure of a fourth bispecific antibody candidate.

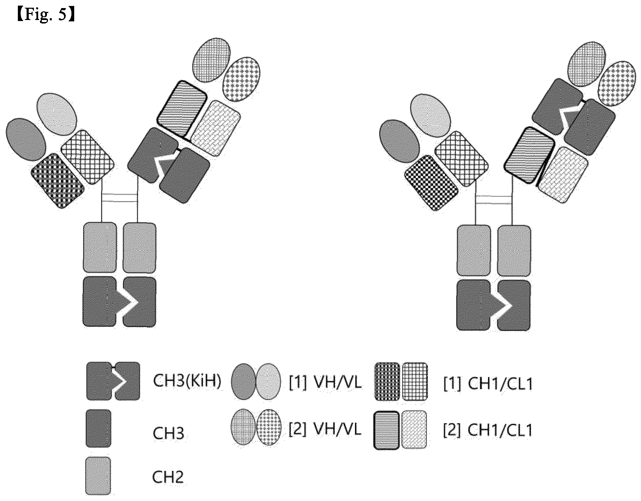

FIG. 5 illustrates the structure of a fifth bispecific antibody candidate.

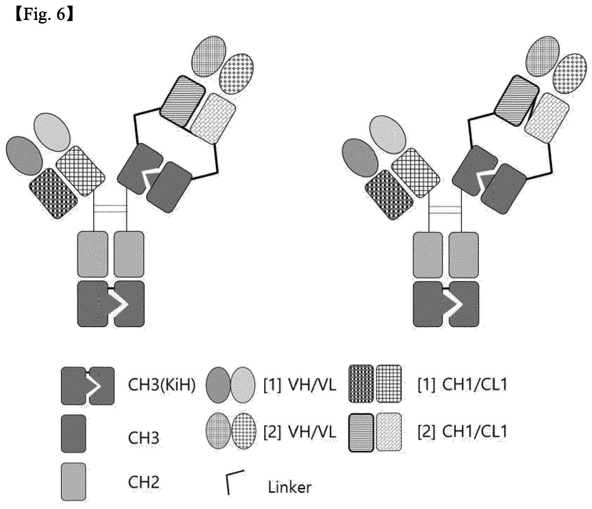

FIG. 6 illustrates the structure of a sixth bispecific antibody candidate.
FIG. 7 illustrates the structure of a seventh bispecific antibody candidate.
FIG. 8 illustrates the structure of a multispecific antibody that binds to three targets.

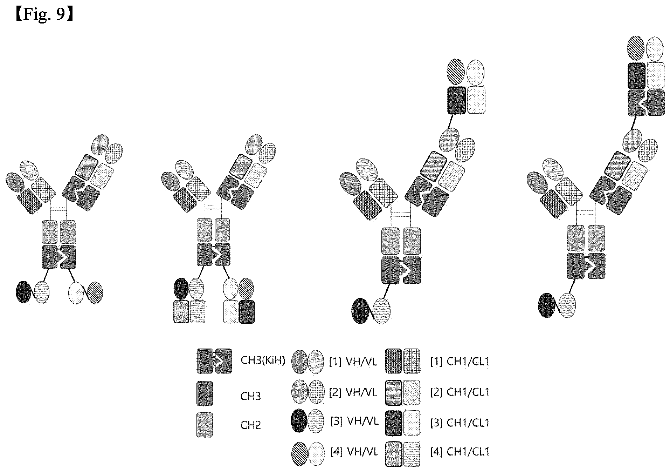

FIG. 9 illustrates the structure of a multispecific antibody that binds to four targets.
FIG. 10 illustrates an exemplary bivalent bispecific antibody format.
FIG. 11 is a schematic diagram illustrating bispecific antibody formats of Q-SBL1 and R-SBL1 constructs, respectively.
FIGS. 12A to 12B are schematic diagrams illustrating various bispecific antibody formats of Q-SBL1 (FIG. 12A) and R-SBL1 (FIG. 12B) depending on the number of linkers.
FIGS. 13A to 13B are schematic diagrams illustrating various bispecific antibody formats of Q-SBL1 (FIG. 13A) and R-SBL1 (FIG. 13B) depending on hinge region.
FIG. 14 is a schematic diagram illustrating various bispecific antibody formats of Q-SBL1 upon interchange of knob-CH3/hole-CH3 domains.
FIG. 15 is a schematic diagram illustrating the bispecific antibody format of each Q-SBL9.
FIG. 16 illustrates an exemplary trivalent bispecific antibody format.
FIG. 17 illustrates an exemplary tetravalent bispecific antibody format.

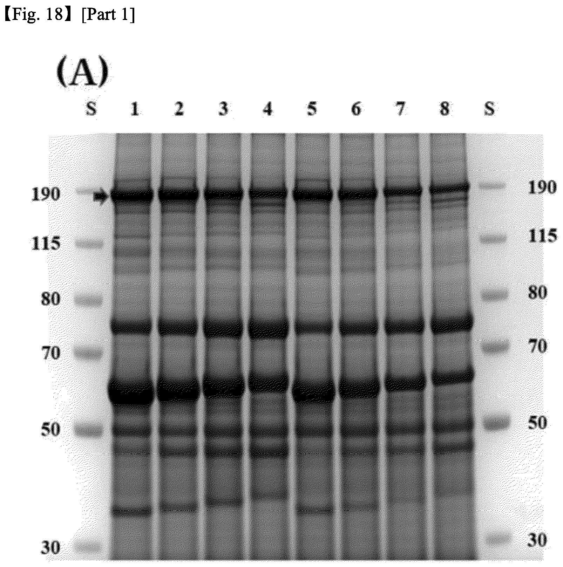

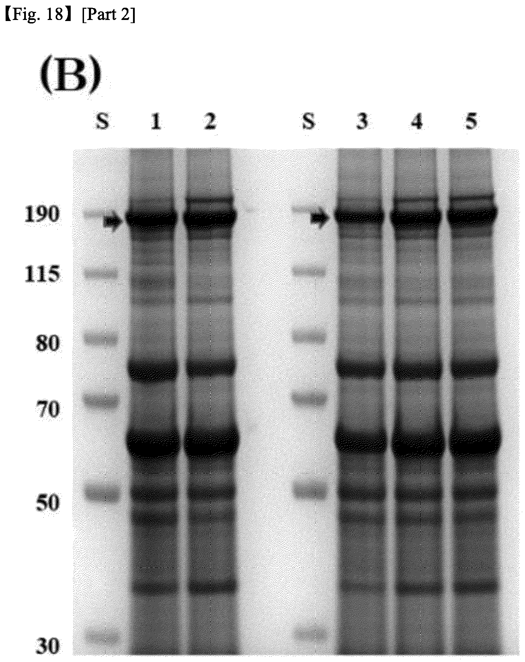

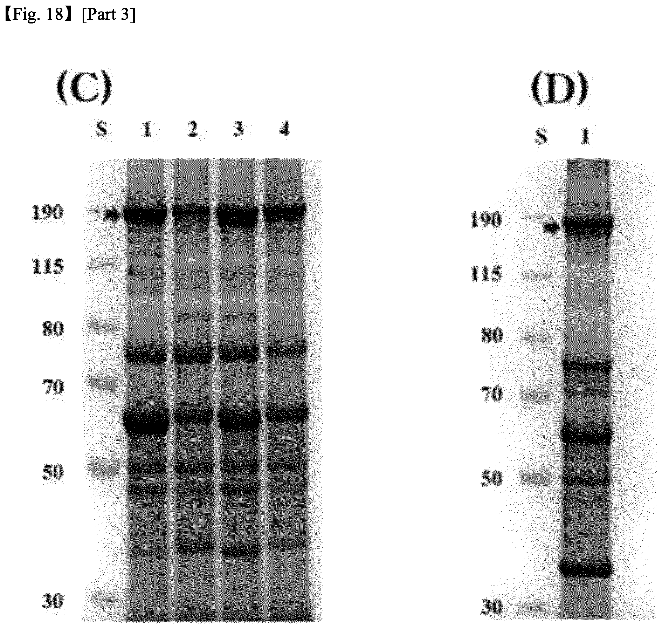

FIG. 18 illustrates non-reducing SDS-PAGE of bispecific antibody candidates obtained by transient production of ExpiCHO-S culture supernatants, wherein (A) is SDS-PAGE gel images of Q-SBL-1,2,3,4 and R-SBL-1,2,3,4 (S: size marker (kDa), Lane 1: Q-SBL-2, Lane 2: Q-SBL-1, Lane 3: Q-SBL-3, Lane 4: Q-SBL-4, Lane 5: R-SBL-2, Lane 6: R-SBL-1, Lane 7: R-SBL-3, Lane 8: R-SBL-4), (B) is an SDS-PAGE gel image of Q-SBL-1, Q-SBL-5, R-SBL-1, R-SBL-5, and R-SBL-6 (S: size marker (kDa), Lane 1: Q-SBL-1, Lane 2: Q-SBL-5, Lane 3: R-SBL-1, Lane 4: R-SBL-5, Lane 5: R-SBL-6), (C) is SDS-PAGE gel images of Q-SBL-1, Q-SBL-6, Q-SBL-7, Q-SBL-8 (S: size marker (kDa), Lane 1: Q-SBL-1, Lane 2: Q-SBL-6, Lane 3: Q-SBL-7, Lane 4: Q-SBL-8), and (D) is an SDS-PAGE gel image of Q-SBL9 (S: size marker (kDa), Lane 1: Q-SBL9).
FIG. 19 illustrates the result of non-reducing SDS-PAGE analysis of the bispecific antibody product obtained by three-step purification.
FIGS. 20A to 20D illustrate the SEC-HPLC profile of the bispecific antibody product obtained in each purification step (FIG. 20A: Q-SBL1, 2, 3, and 4, FIG. 20B: R-SBL1, 2, 3, and 4, FIG. 20C: Q-SBL5, R-SBL5 and 6, and FIG. 20D: Q-SBL6, 7, 8 and 9).
FIG. 21 illustrates the result of CE-SDS analysis of antibodies obtained by the three-step purification under non-reduction conditions.
FIG. 22 illustrates differential scanning calorimetry (DSC) thermograms of Q-SBL2 (A) and Q-SBL9 (B) obtained by the three-step purification.

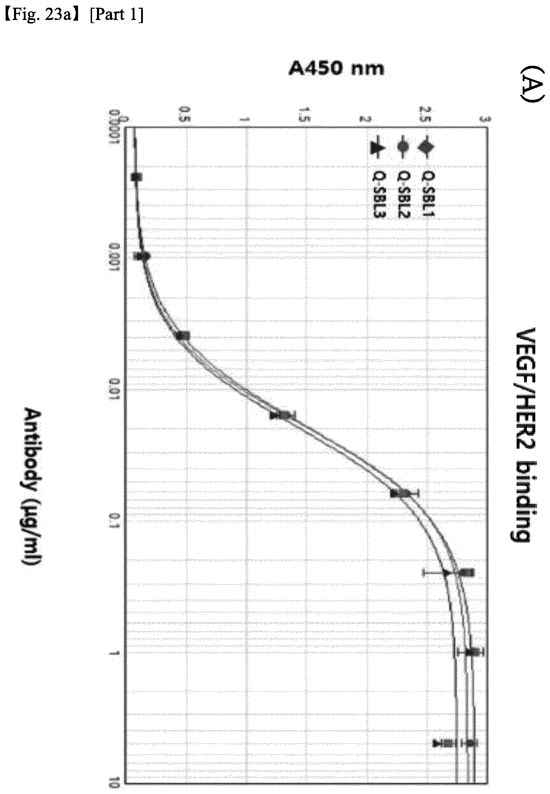

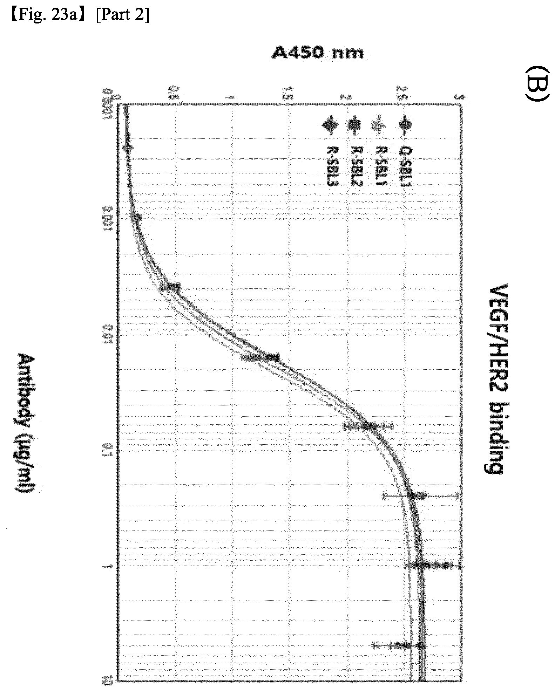

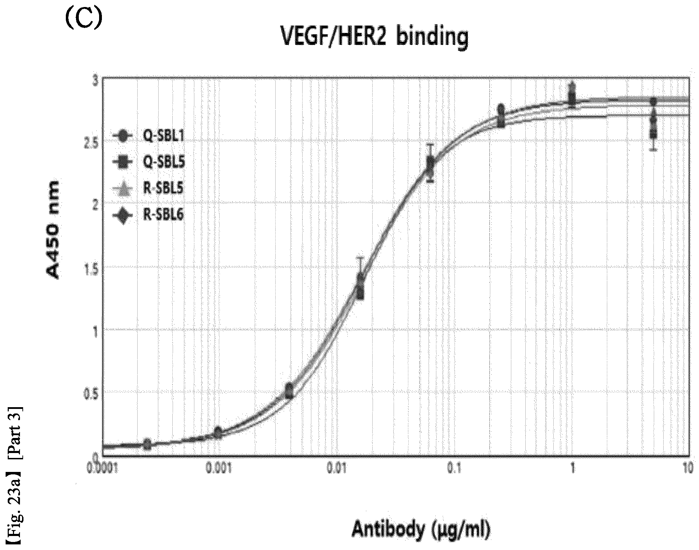

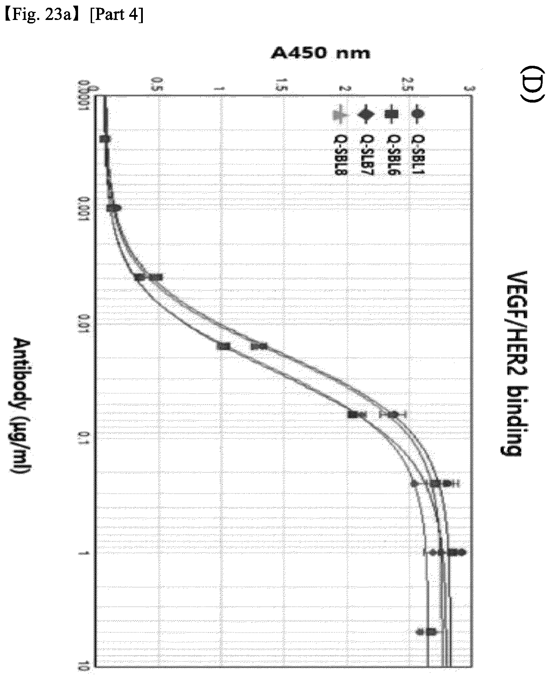

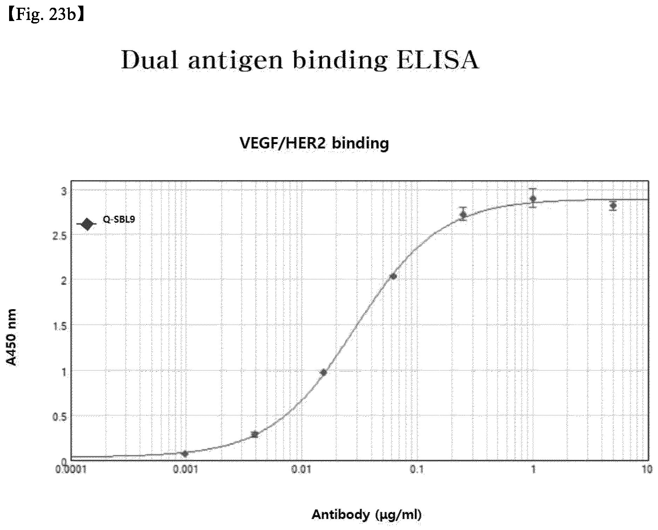

FIGS. 23A and 23B illustrates simultaneous dual binding of bispecific antibodies to VEGF and HER2 (FIG. 23A-A): ◆: Q-SBL1, •: Q-SBL2, ▲: Q-SBL3, (FIG. 23A-B) •: Q-SBL1, ■: R-SBL1, ▲: R-SBL2, ◆: R-SBL3, (FIG. 23A-C) •: Q-SBL1, ■: Q-SBL5, ▲:R-SBL5, ◆: R-SBL6, (FIG. 23A-D) •: Q-SBL1, ■: Q-SBL6, ◆: Q-SBL7, ▲: Q-SBL8, (FIG. 23B) ◆: Q-SBL9.
FIG. 24 illustrates the inhibitory effect of bispecific antibodies on human umbilical vein endothelial cells (HUVEC) using Q-SBL9, wherein different concentrations of the bispecific antibody are incubated for 3 days and then treated with a CCK-8 solution and each sample was run in triplicate.
FIG. 25 illustrates the results of assay of binding of bispecific antibodies to the C1q protein; ■: Trastuzumab, •: Q-SBL2, ▲: Q-SBL9.

### [Best Mode]

Unless defined otherwise, all technical and scientific terms used herein have the same meanings as appreciated by those skilled in the field to which the present invention pertains. In general, the nomenclature used herein is well-known in the art and is ordinarily used.

In one aspect, the present invention is directed to a bispecific antibody including a first arm binding to a first antigen and including VH1-CHa-Fc1 and VL1-CLb, and a second arm binding to a second antigen and including VH2-CH1-Fc2 and VL2-CL,
wherein the VH1 and the VH2 are each heavy-chain variable regions including the same or different antigen-binding regions,
the VL1 and VL2 are each light-chain variable regions including the same or different antigen-binding regions,
the CHa includes i) an IgG heavy-chain constant region or an IgD heavy-chain constant region CH1, and an IgG heavy-chain constant region CH2 or CH3, or ii) an IgM heavy-chain constant region CH3,
the CLb includes i) a CL1 including an IgG light-chain constant region λ or x, and at least one selected from the group consisting of IgG heavy-chain constant regions CH1, CH2, and CH3, or ii) an IgM heavy-chain constant region CH3,
the CH1 is an IgG heavy-chain constant region CH1 and the CL is an IgG light-chain constant region CL, and
the Fc1 of the first arm is linked to the Fc2 of the second arm to form a heavy-chain constant region dimer.

As used herein, the term "bispecific" refers to an ability of a binding protein to specifically bind to two different types of targets to control the activity of the targets. For example, bispecificity can be obtained by conjugation of a monoclonal antibody or fragment thereof that specifically binds to each target, possesses two distinct antigen-binding arms (arm: specific for two targets) and has monovalence for each bound antigen.

VH1 and VH2 are each heavy-chain variable regions including the same or different antigen-binding regions. VL1 and VL2 are each light-chain variable regions including the same or different antigen-binding regions.

The term "polypeptide" refers to any polymeric chain of amino acids. The terms "peptide" and "protein" are used interchangeably with the term "polypeptide", which also refer to any polymeric chain of amino acids. The term "polypeptide" includes natural or synthetic proteins, protein fragments, and polypeptide analogs of protein sequences. Polypeptides may be monomeric or polymeric.

The term "specific binding" or "specifically binding" with respect to the interaction of an antibody, polypeptide, protein or peptide means that the interaction depends on the presence of a specific construct (e.g., antigenic determinant or epitope) of a chemical species. For example, antibodies generally recognize and bind to specific protein constructs rather than proteins. If an antibody is specific for epitope "A", the presence of a molecule containing epitope A (or released unlabeled A) in the reaction involving labeled "A" and the antibody reduces the amount of labeled A bound to the antibody.

An antibody refers to any immunoglobulin (Ig) molecule consisting of four polypeptide chains, two heavy (H) chains and two light (L) chains, or any functional fragment, mutant, variant or derivative having the indispensable epitope-binding property of such an Ig molecule. Specific examples of such a mutant, variant or derivative are described below, but are not limited thereto.

The term "monoclonal antibody" refers to an identical antibody, which is obtained from a population of substantially homogeneous antibodies, that is, each antibody constituting the population, excluding possible naturally occurring mutations that may be present in trivial amounts. Monoclonal antibodies are highly specific and are thus induced against a single antigenic site. Unlike conventional (polyclonal) antibody preparations that typically include different antibodies directed against different determinants (epitopes), each monoclonal antibody is directed against a single determinant on the antigen.

The term "epitope" refers to a protein determinant to which an antibody can specifically bind. Epitopes usually consist of a group of chemically active surface molecules, such as amino acid or sugar side chains, and generally have not only specific three-dimensional structural characteristics but also specific charge characteristics. Three-dimensional epitopes are distinguished from non-three-dimensional epitopes in that a bond to the former is broken in the presence of a denatured solvent, while a bond to the latter is not broken.

The non-human (e.g., murine) antibody of the "humanized" form is a chimeric antibody including minimal sequences derived from non-human immunoglobulins. In most cases, the humanized antibody is a human immunoglobulin (receptor antibody) in which a residue from the hypervariable region of a receptor is replaced with a residue from the hypervariable region of a non-human species (donor antibody) such as a mouse, rat, rabbit or non-human primate having the desired specificity, affinity and ability.

As used herein, the term "human antibody" refers to a molecule derived from human immunoglobulin, in which all of the amino acid sequences constituting the antibody including a complementarity-determining region and a structural region are composed of human immunoglobulins.

In a complete antibody, each heavy-chain consists of a heavy-chain variable region (HCVR or VH) and a heavy-chain constant region. The heavy-chain constant region is composed of three domains, CH1, CH2 and CH3. Each light-chain is composed of a light-chain variable region and a light-chain constant region. The light-chain constant region consists of one domain, CL. The VH and VL regions may be subdivided into hypervariable regions called "complementarity-determining regions" (CDRs) and further conserved regions called "framework regions" (FRs) are distributed in the CDRs. As used herein, the term "variable domain" refers to the light- and heavy-chain regions of an antibody molecule including the amino acid sequences of a complementarity-determining region (CDR; i.e., CDR1, CDR2, and CDR3) and a framework region (FR). VH refers to a variable domain of the heavy-chain. VL refers to a variable domain of the light-chain.

The term "complementarity-determining region" (CDR; i.e., CDR1, CDR2, and CDR3) refers to an amino acid residue of the antibody variable domain, which is necessary for antigen binding. Each variable domain typically has three CDR regions, identified as CDR1, CDR2, and CDR3.

The term "framework region" (FR) refers to a variable domain residue other than a CDR residue. Each variable domain typically has four FRs, identified as FR1, FR2, FR3, and FR4.

They are arranged from amino terminus to carboxy terminus in the following order: FR1, CDR1, FR2, CDR2, FR3, CDR3, and FR4. An immunoglobulin molecule may be of any type (e.g., IgG, IgE, IgM, IgD, IgA, or IgY), class (e.g., IgG1, IgG2, IgG3, IgG4, IgA1, or IgA2) or subclass.

A Fab fragment refers to a structure including a variable region of each of the heavy-chain and the light-chain, the constant region of the light-chain, and the first constant domain (CH1) of the heavy-chain, each having one antigen-binding site. Fab' is different from Fab in that it further includes a hinge region including at least one cysteine residue at the C-terminus of the CH1 domain of the heavy-chain. F(ab')2 is created by a disulfide bond between cysteine residues in the hinge region of Fab'. Fv is the minimal antibody fragment having only a heavy-chain variable region and a light-chain variable region. Two-chain Fv is a fragment in which the variable region of the heavy-chain and the variable region of the light-chain are linked by a non-covalent bond, and single-chain Fv (scFv) is a fragment in which the variable region of the heavy-chain and the variable region of the light-chain are generally linked by a covalent bond via a peptide linker therebetween, or are directly linked at the C-terminal, forming a dimer-shaped structure, like the two-chain Fv. Such antibody fragments may be obtained using proteases (e.g., Fab can be obtained by restriction-cleaving the complete antibody with papain, and the F(ab')2 fragment may be obtained by restriction-cleaving the complete antibody with pepsin), and may be produced using genetic recombination techniques.

The antibody according to the present invention includes "antigen-binding portion", which includes at least one antibody fragment having the ability to specifically bind to antigens and may specifically bind to other antigens, and thus may be bispecific or multispecific. In the present invention, the antigen-binding portion is included in the heavy-chain variable region of VH1 or VH2 and the light-chain variable region of VL1 or VL2, and each includes the same or different antigen-binding regions.

Two different variable regions can bind to two antigens. In order to minimize mispairing of the light-chain, an IgG CH3 dimer may be further introduced into either the C-terminal portion of the Fab or between the variable and constant regions of the Fab in one of the two Fab regions. By further introducing a CH3 dimer into one of the Fab regions, the two arms of the Fab region may be asymmetric to each other. Various types of CH1 domains may be introduced into the CH1/CL portion in one of Fab regions. As a result, it is possible to produce a bispecific or multispecific antibody having excellent productivity and stability.

According to the present invention, the antibody does not greatly deviate from the IgG-like structure. Structures that greatly deviate from the IgG structure are highly likely to make proteins unstable.

Moreover, a constant region of an immunoglobulin structure is further introduced into the mis-paired light-chain portion to greatly reduce the frequency of mispairing. Light-chain mispairing was prevented by introducing a known heavy-chain constant region domain into one of the two Fab arms.

The first bispecific antibody candidate has no structural modification in the left arm (second arm) in the two Fab parts that bind to the target and may have an Ig constant domain (CH3) further introduced into the right arm (first arm), may have IgG1 CH1 and various types of CH1 introduced instead of IgG1 CH1, and may have a knob-in-hole structure as a heterodimer structure of the heavy-chain.

In the second candidate bispecific antibody, the right Fab arm portion in the two Fab portions that bind to the target may be changed. The changed part may change the positions of the CH3 domain and the CH1 domain in the first bispecific antibody candidate.

In some cases, the third bispecific antibody candidate may have a configuration in which CH1/CL is substituted with CH3 of IgM.

The CHa includes i) an IgG heavy-chain constant region or an IgD heavy-chain constant region CH1, and an IgG heavy-chain constant region CH2 or CH3, or ii) an IgM heavy-chain constant region CH3.

The CHa may be derived from IgG1, IgG2, IgG3, IgG4, IgD or IgM. Specifically, CHa may include at least one selected from the group consisting of heavy-chain constant regions CH1, CH2, and CH3 derived from IgG1, IgG2, IgG3, IgG4, IgD or IgM. For example, CHa may include heavy-chain constant region CH1 derived from IgG1, IgG2, IgG3, IgG4, IgD or IgM, and include heavy-chain constant region CH3 derived from IgG1, IgG2, IgG3, IgG4, IgD and IgM in order from N-terminus to C-terminus in the first arm. For example, CHa may include heavy-chain constant region CH3 derived from IgG1, IgG2, IgG3, IgG4, IgD or IgM, and include heavy-chain constant region CH1 derived from IgG1, IgG2, IgG3, IgG4, IgD and IgM in order from N-terminus to C-terminus in the first arm. In some cases, CHa may include an IgM-derived heavy-chain constant region CH3.

The CLb includes i) CL1 including an IgG light-chain constant region λ or x, and at least one selected from the group consisting of IgG heavy-chain constant regions CH1, CH2, and CH3, or ii) an IgM heavy-chain constant region CH3.

The CLb may be derived from IgG1, IgG2, IgG3, IgG4, IgD or IgM. Specifically, CLb may include CL1 including light-chain constant region λ or κ derived from IgG1, IgG2, IgG3, IgG4, IgD or IgM, and at least one selected from the group consisting of heavy-chain constant regions CH1, CH2, and CH3 derived from IgG1, IgG2, IgG3, IgG4, IgD or IgM. For example, CLb may include CL1 including light-chain constant region λ or κ derived from IgG1, IgG2, IgG3, IgG4, IgD or IgM, and a heavy-chain constant region CH3 derived from IgG1, IgG2, IgG3, IgG4, IgD or IgM in order from N-terminus to C-terminus in the first arm. For example, CLb may include a heavy-chain constant region CH3 derived from IgG1, IgG2, IgG3, IgG4, IgD or IgM and CL1 including light-chain constant region λ or κ derived from IgG1, IgG2, IgG3, IgG4, IgD or IgM in order from N-terminus to C-terminus in the first arm. In some cases, CLb may include a heavy-chain constant region CH3 derived from IgM.

The CH1 is an IgG heavy-chain constant region CH1 and the CL is an IgG light-chain constant region CL. Each of CH1 and CL may be derived from IgG1, IgG2, IgG3, IgG4 or IgD. The CH1 and CL may have a Fab fragment-like structure through a non-covalent interaction.

As used herein, the term "Fc region" is used to define the C-terminal region of an immunoglobulin heavy-chain that can be produced by papain digestion of an intact antibody. The Fc region may be a native sequence Fc region or a variant Fc region. The Fc region of an immunoglobulin generally includes two constant domains, a CH2 domain and a CH3 domain, and optionally includes a CH4 domain.

In one embodiment, each of CHa and CLb may include CH3. Specifically, each of CHa and CLb may include CH3 derived from IgG1, IgG2, IgG3, or IgG4. The CHa and CLb may form a dimer through covalent or non-covalent interaction.

The CHa and CLb may be linked through chains via or without a disulfide bond. Specifically, the dimer formed through CH3 included in each of CHa and CLb of the first arm, and the CH3 dimer in the Fab region including CH1 and CL1 included in CHa and CLb, respectively, are linked through a disulfide bond, and CH1 and CL1 may be linked without a disulfide bond.

In some cases, the CH3 domain and the CH1 domain may be linked through a linker. The linker may be a peptide linker and may include about 5-25 aa residues, or specifically about 5-10 aa residues. For example, the linker may include hydrophilic amino acids such as glycine and/or serine, but are not limited thereto.

Specifically, the linker, for example, includes a glycine linker (G, Gly)p (p is 1 to 10), or a GS linker (GₙS)ₘ (n and m are each 1 to 10) in order to impart structural flexibility. Specifically, the linker may include GGGGS or (GGGGS)2, or (G, Gly)p 5-10 aa glycine wherein p is 5 to 10.

Fc₁ of the first arm and Fc₂ of the second arm each include CH2 and CH3 monomers of the heavy-chain constant region. The monomer means one domain among dimers formed through two constant domains CH2-CH3 having the same amino acid sequence of the heavy-chain constant region Fc. Fc₁ of the first arm and Fc₂ of the second arm are linked to each other to form a heavy-chain constant region dimer.

The dimer may include a homodimer formed by bonding between constant domains CH3 having the same amino acid sequence or a heterodimer formed by bonding between constant domains CH3 having different amino acid sequences.

Optionally, a disulfide bond may be present between CH3 of CHa and CH3 of CLb or a pair of CH3 of Fc, or an interchain linkage may be formed therebetween without a disulfide bond.

In one embodiment, the CH1 of CHa and CL1 of CLb may be linked via a disulfide bond or without a disulfide bond. Specifically, CH1 of CHa and CL1 of CLb may be linked without a disulfide bond.

The CHa and CLb each include CH3, and the CH3 of the CHa and the CH3 of the CLb are linked to form a dimer. The CHa and CLb each include CH3 and CH3 may form a dimer through a disulfide bond.

The CHa and CLb each include CH3, and the CH3 of the CHa and the CH3 of the CLb are linked to form a dimer. The CHa and CLb each include CH3, and CH3 may form a dimer through a disulfide bond.

In one embodiment, one of the dimers formed by bonding CH3 of CHa to CH3 of CLb includes at least one selected from the group consisting of Y349C, S354C, T366S, T366W, L368A, and Y407V, and at least one selected from the group consisting of S354C, Y349C, T366W, T366S, L368A and Y407V, and thus may have a knob-in-hole structure.

One of the dimers includes at least one selected from the group consisting of T366W, S354C, and Y349C, and the other includes at least one selected from the group consisting of S354C, Y349C, T366S, L368A, and Y407V, to form a knob-in-hole structure. Specifically, one of the dimers formed by bonding CH3 of CHa to CH3 of CLb includes S354C, T366S, L368A, and Y407V, and the other includes Y349C and T366W, to form a knob-in-hole structure.

Specifically, the CH1 of CHa and the CL1 of CLb are linked without a disulfide bond, i) any one of CH3 of CHa and CH3 of CLb includes at least one selected from the group consisting of Y349C, T366S, L368A and Y407V, and the other includes S354C and/or T366W, or ii) any one of CH3 of CHa and CH3 of CLb includes at least one selected from the group consisting of S354C, T366S, L368A and Y407V, and the other includes Y349C and/or T366W.

The CH1 of CHa and CL1 of CLb are linked without a disulfide bond, i) any one of CH3 of CHa and CH3 of CLb includes Y349C, T366S, L368A and Y407V, and the other includes S354C and T366W, or ii) one of CH3 of CHa and CH3 of CLb includes S354C, T366S, L368A, and Y407V, and the other includes Y349C and T366W.

In one embodiment, one of the CH3 dimers of the Fc includes at least one selected from the group consisting of Y349C, S354C, T366S, T366W, L368A and Y407V, and at least one selected from the group consisting of S354C, Y349C, T366W, T366S, L368A and Y407V, to form a knob-in-hole structure among dimers.

By forming a mutation in the Fc domain of IgG (immunoglobulin G), an asymmetric heterodimer is stably formed. In addition, the heterodimer structure of the heavy-chain may be a known knob-in-hole structure.

The knob-in-hole, which was first published by Genentech in a paper in 1997, is currently the most widely adopted structure by various developers. Although the knob-in-hole structure is introduced, 100% heterodimer is not formed in the knob-in-hole structure. Therefore, a high heterodimer ratio is obtained by establishing optimal transfection conditions to improve production yield.

By inducing mutations in the CH3 domains of two different Ig heavy-chains, a hole structure is created in the CH3 domain of one Ig heavy-chain and a knob structure is created in the CH3 domain of the other Ig heavy-chain, so that the two Ig heavy-chains form a heterodimer.

The knob-into-hole technology converts hydrophobic amino acid residues with large side chains into hydrophobic amino acids with small side chains for residues located in the hydrophobic core of the CH3 domain interaction site in one heavy-chain CH3 domain, to form a hole structure. In the other heavy-chain CH3 domain, hydrophobic amino acid residues with small side chains are substituted with hydrophobic amino acids with large side chains to form a knob structure. As a result, a heavy-chain constant region mutation pair introduced with two pairs of mutations is co-expressed to form a heterodimer heavy-chain constant region.

In one embodiment, to form the knob structure, CH3 of CHa and CLb or CH3 of Fc may include at least one selected from the group consisting of S354C, Y349C, T366W, T366S, L368A, and Y407V. For example, to form the hole structure, CH3 of CHa and CLb or CH3 of Fc may include at least one selected from the group consisting of Y349C, S354C, T366S, T366W, L368A, and Y407V.

In another embodiment, for example, CH3 of CHa and CLb or CH3 of Fc may include T366W to form the knob structure. In order to form the hole structure, for example, CH3 of CHa and CLb or CH3 of Fc may include at least one selected from the group consisting of T366S, L368A, and Y407V. Specifically, to form the knob structure, for example, Y349C and T366W may be included. In order to form the hole structure, for example, CH3 of CHa and CLb or CH3 of Fc may include at least one selected from the group consisting of S354C, T366S, L368A, and Y407V.

The VH/VL amino acid sequences of bevacizumab and trastuzumab may be used as two types of VH/VL amino acid sequences in various types of bispecific or multispecific antibodies. Amino acid numbering may be based on the IMGT numbering system (based on Eu index according to http://www.imgt.org/IMGTScientificChart/Numbering/Hu_IGHGnber.ht ml#refs).

The specific sequences used in each candidate are as follows.

**[Table 1]**

| Config uratio n | Sequence | SEQ ID No. |
|---|---|---|
| Bevaci zumab VH | | 1 |
| Bevaci zumab VL | | 2 |
| Trastu zumab VH | | 3 |
| Trastu zumab VL | | 4 |

**[Table 2]**

| Confi gurat ion | Sequence | SEQ ID No. |
|---|---|---|
| IgG1 CH1 | ASTKGPSVFPLAPSSKSTSGGTAALGCLVKDYFPEPVTVSWNSGALTSG VHTFPAVLQSSGLYSLSSVVTVPSSSLGTQTYICNVNHKPSNTKVDKKV | 5 |
| IgG1 CH2 | APELLGGPSVFLFPPKPKDTLMISRTPEVTCVVVDVSHEDPEVKFNWYV DGVEVHNAKTKPREEQYNSTYRVVSVLTVLHQDWLNGKEYKCKVSNKAL PAPIEKTISKAK | 6 |
| IgG1 CH3 | GQPREPQVYTLPPSRDELTKNQVSLTCLVKGFYPSDIAVEWESNGQPEN NYKTTPPVLDSDGSFFLYSKLTVDKSRWQQGNVFSCSVMHEALHNHYTQ KSLSLSPGK | 7 |
| IgG1 CH3-Knob | GQPREPQVYTLPPSREEMTKNQVSLWCLVKGFYPSDIAVEWESNGQPEN NYKTTPPVLDSDGSFFLYSKLTVDKSRWQQGNVFSCSVMHEALHNHYTQ KSLSLSPGK | 8 |
| IgG1 CH3 - Hole | GQPREPQVYTLPPSREEMTKNQVSLSCAVKGFYPSDIAVEWESNGQPEN NYKTTPPVLDSDGSFFLVSKLTVDKSRWQQGNVFSCSVMHEALHNHYTQ KSLSLSPGK | 9 |
| IgG1 CH3-Knob (S354 C) | GQPREPQVYTLPPCREEMTKNQVSLWCLVKGFYPSDIAVEWESNGQPEN NYKTTPPVLDSDGSFFLYSKLTVDKSRWQQGNVFSCSVMHEALHNHYTQ KSLSLSPGK | 10 |
| IgG1 CH3-Hole (Y349 C) | GQPREPQVCTLPPSREEMTKNQVSLSCAVKGFYPSDIAVEWESNGQPEN NYKTTPPVLDSDGSFFLVSKLTVDKSRWQQGNVFSCSVMHEALHNHYTQ KSLSLSPGK | 11 |
| IgG1 CH3-Knob (Y349 C) | GQPREPQVCTLPPSREEMTKNQVSLWCLVKGFYPSDIAVEWESNGQPEN NYKTTPPVLDSDGSFFLYSKLTVDKSRWQQGNVFSCSVMHEALHNHYTQ KSLSLSPGK | 12 |
| IgG1 CH3-Hole (S354 C) | GQPREPQVYTLPPCREEMTKNQVSLSCAVKGFYPSDIAVEWESNGQPEN NYKTTPPVLDSDGSFFLVSKLTVDKSRWQQGNVFSCSVMHEALHNHYTQ KSLSLSPGK | 13 |
| CL-Kappa | | 14 |
| CL-Kappa C214S | RTVAAPSVFIFPPSDEQLKSGTASVVCLLNNFYPREAKVQWKVDNALQS GNSQESVTEQDSKDSTYSLSSTLTLSKADYEKHKVYACEVTHQGLSSPV TKSFNRGES | 15 |
| CL-Lambd a | GQPKANPTVTLFPPSSEELQANKATLVCLISDFYPGAVTVAWKADGSPV KAGVETTKPSKQSNNKYAASSYLSLTPEQWKSHRSYSCQVTHEGSTVEK TVAPTECS | 16 |
| CL-Lambd a-C214S | GQPKANPTVTLFPPSSEELQANKATLVCLISDFYPGAVTVAWKADGSPV KAGVETTKPSKQSNNKYAASSYLSLTPEQWKSHRSYSCQVTHEGSTVEK TVAPTESS | 17 |
| IgG4 CH1 | ASTKGPSVFPLAPCSRSTSESTAALGCLVKDYFPEPVTVSWNSGALTSG VHTFPAVLQSSGLYSLSSVVTVPSSSLGTKTYTCNVDHKPSNTKVDKRV | 18 |
| IgG4 CH1-C131S | ASTKGPSVFPLAPSSRSTSESTAALGCLVKDYFPEPVTVSWNSGALTSG VHTFPAVLQSSGLYSLSSVVTVPSSSLGTKTYTCNVDHKPSNTKVDKRV | 19 |
| IgD CH1 | APTKAPDVFPIISGCRHPKDNSPVVLACLITGYHPTSVTVTWYMGTQSQ PQRTFPEIQRRDSYYMTSSQLSTPLQQWRQGEYKCVVQHTASKSKKEIF | 20 |
| IgG2 CH1 | ASTKGPSVFPLAPCSRSTSESTAALGCLVKDYFPEPVTVSWNSGALTSG VHTFPAVLQSSGLYSLSSVVTVPSSNFGTQTYTCNVDHKPSNTKVDKTV | 66 |
| IgM CH1 | GSASAPTLFPLVSCENSPSDTSSVAVGCLAQDFLPDSITFSWKYKNNSD ISSTRGFPSVLRGGKYAATSQVLLPSKDVMQGTDEHVVCKVQHPNGNKE KNVPLPV | 67 |
| IgM CH3 | TAIRVFAIPPSFASIFLTKSTKLTCLVTDLTTYDSVTISWTRQNGEAVK THTNISESHPNATFSAVGEASICEDDWNSGERFTCTVTHTDLPSPLKQT ISRPKG | 68 |

**[Table 3]**

| Configuration | Sequence | SEQ ID No. |
|---|---|---|
| IgG1 Hinge -wild type | EPKSCDKTHTCPPCP | 21 |
| IgG1 Hinge- del EPKSC | DKTHTCPPCP | 22 |
| IgG1 Hinge - EPKSS | EPKSSDKTHTCPPCP | 23 |
| IgG4 Hinge | ESKYGPPCPPCP | 24 |
| Elbow sequence-1 | AS | 25 |
| Elbow sequence-2 | RT | 26 |
| Linker-1 | GGGGS | 27 |
| Linker-2 | GGGGSGGGGS | 28 |
| Linker-3 | GGGGSGGGGSGGGGS | 29 |
| Linker-4 | GGGGSGGGGSGGGGSGGGGS | 30 |

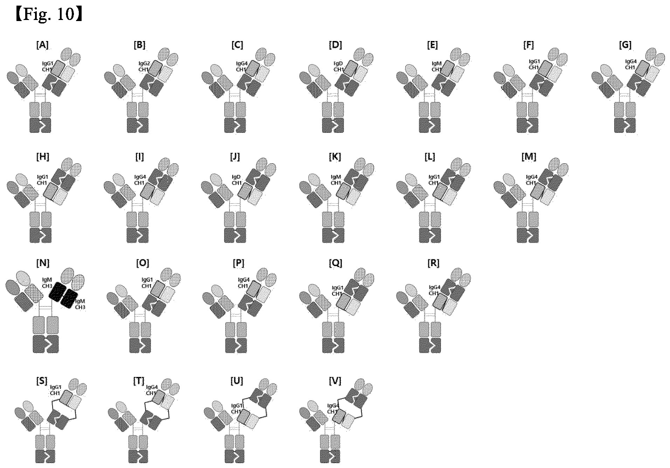

As can be seen from FIG. 10, [A] has a knob structure in the CH3 domain of the heavy-chain region that binds to one (second arm on the left) epitope, and has S354C and T366W mutations to introduce a disulfide bond. In some cases, disulfide bonds may not be introduced.

In the heavy-chain region (first arm on the right) binding to another epitope, the CH3 domain linked to the C-terminus of the CH1 domain has a hole structure and has T366S, L368A, and Y407V mutations. In some cases, a disulfide bond may be introduced. At this time, the CH3 domain has the Y349C mutation. The CH3 domain bonded to the C-terminus of the CH2 domain of the heavy-chain region has a hole structure and has Y349C, T366S, L368A, and Y407V mutations through a disulfide bond. In some cases, disulfide bonds may not be introduced. The CH3 domain linked to the C-terminus of the CL domain of the light-chain region has a knob structure and has a T366W mutation. In some cases, a disulfide bond may be introduced. At this time, the CH3 domain has the S354C mutation. Various types of CH1 domains may be introduced into the heavy-chain region CH1 domain.

E216, P217, K218, S219, and C220 may be added to the C-terminus of the IgG1 CH1 domain of the heavy-chain region (first arm on the right) that binds to another epitope. This aims at forming a disulfide bond with the CL domain. The CH3 domain sequence is based on the Uniprot site (https://www.uniprot.org/uniprot/P01857). The IgD CH1 domain sequence corresponds to the amino acid sequence from amino acids 1 to 98 in https://www.uniprot.org/uniprot/P01880. The IgM CH1 domain sequence corresponds to the amino acid sequence from amino acids 1 to 105 in https://www.uniprot.org/uniprot/P01871.

[H] has a knob structure in the CH3 domain of the heavy-chain region that binds to one (second arm on the left) epitope, and has S354C and T366W mutations to introduce a disulfide bond.

In some cases, a disulfide bond may not be introduced and S354 may be maintained.

The CH3 domain linked to the C-terminus of the VH domain in the heavy-chain region (first arm on the right) that binds to another epitope has a hole structure and has T366S, L368A, and Y407V mutations. In some cases, a disulfide bond may be introduced. The CH3 domain has the Y349C mutation. The CH3 domain linked to the C-terminus of the CH2 domain of the heavy-chain region has a hole structure and has Y349C, T366S, L368A, and Y407V mutations to introduce a disulfide bond. In some cases, a disulfide bond may not be introduced and Y349 may be maintained. The CH3 domain linked to the C-terminus of the VL domain of the light-chain region has a knob and has a T366W mutation. In some cases, a disulfide bond may be introduced. The CH3 domain has a S354C mutation. Various types of CH1 domains may be introduced into the heavy-chain region CH1 domain.

Elbow sequences A118 and S119 are added to the C-terminus of the VH domain of the heavy-chain region (first arm on the right) that binds to another epitope and various types of CH3 domains are bonded thereto next. Here, the sequence of the CH3 domain includes from P343 to K447, without the elbow sequences G341 and Q342, from the sequence shown in https://www.uniprot.org/uniprot/P01857. Elbow sequences R108 and T109 are added to the C-terminus of the VL domain of the light-chain region and various types of CH3 domains are bonded thereto next.

[N] has a knob structure in the CH3 domain of the heavy-chain region that binds to one (second arm on the left) epitope, and has S354C and T366W mutations to introduce a disulfide bond.

Elbow sequences A118 and S119 are added to the C-terminus of the VH domain of the heavy-chain region (first arm on the right) that binds to another epitope and an IgM CH3 domain is bonded thereto next. Elbow sequences R108 and T109 are added to the C-terminus of the VL domain of the light-chain region and an IgM CH3 domain is bonded thereto next. The IgM CH3 domain sequence corresponds to the amino acid sequence from amino acids 220 to 323 in https://www.uniprot.org/uniprot/P01871.

[S, U] has a knob structure in the CH3 domain of the heavy-chain region that binds to one (second arm on the left) epitope, and has S354C and T366W mutations to introduce a disulfide bond. Elbow sequences A118 and S119 are added to the C-terminus of the VH domain of the heavy-chain region (first arm on the right) that binds to another epitope and a GGGGS linker is bonded thereto next. In addition, various types of CH3 domains are bonded thereto. The GGGGS linker includes one to five GGGGS repeat sequences with various lengths.

The specific sequence for each candidate is as follows.

**[Table 4]**

| | **Heavy chain-1** | **Light chain-1** | **Heavy chain-2** | **Light chain-2** |
|---|---|---|---|---|
| [A] | EVQLVESGGGLVQPGGSLRL SCAASGYTFTNYGMNWVR QAPGKGLEWVGWINTYTGE PTYAADFKRRFTFSLDTSKST AYLQMNSLRAEDTAVYYCAK YPHYYGSSHWYFDVWGQG TLVTVSSASTKGPSVFPLAPS SKSTSGGTAALGCLVKDYFPE PVTVSWNSGALTSGVHTFPA VLQSSGLYSLSSVVTVPSSSL GTQTYICNVNHKPSNTKVDK KVEPKSCDKTHTCPPCPAPEL LGGPSVFLFPPKPKDTLMISR TPEVTCVVVDVSHEDPEVKF NWYVDGVEVHNAKTKPREE QYNSTYRVVSVLTVLHQDW LNGKEYKCKVSNKALPAPIEK TISKAKGQPREPQVYTLPPCR EEMTKNQVSLWCLVKGFYPS DIAVEWESNGQPENNYKTT PPVLDSDGSFFLYSKLTVDKS RWQQGNVFSCSVMHEALH NHYTQKSLSLSPGK | DIQMTQSPSSLSASVGDR VTITCSASQDISNYLNWY QQKPGKAPKVLIYFTSSLH SGVPSRFSGSGSGTDFTLT ISSLQPEDFATYYCQQYST VPWTFGQGTKVEIKRTVA APSVFIFPPSDEQLKSGTA SVVCLLNNFYPREAKVQ WKVDNALQSGNSQESVT EQDSKDSTYSLSSTLTLSK ADYEKHKVYACEVTHQGL SSPVTKSFNRGEC | EVQLVESGGGLVQPGGSLRLSCAASGF NIKDTYIHWVRQAPGKGLEWVARIYPT NGYTRYADSVKGRFTISADTSKNTAYLQ MNSLRAEDTAVYYCSRWGGDGFYAM DYWGQGTLVTVSSASTKGPSVFPLAPS SKSTSGGTAALGCLVKDYFPEPVTVSW NSGALTSGVHTFPAVLQSSGLYSLSSVV TVPSSSLGTQTYICNVNHKPSNTKVDK KVEPKSCGQPREPQVYTLPPSREEMTK NQVSLSCAVKGFYPSDIAVEWESNGQ PENNYKTTPPVLDSDGSFFLVSKLTVDK SRWQQGNVFSCSVMHEALHNHYTQ KSLSLSPGKDKTHTCPPCPAPELLGGPS VFLFPPKPKDTLMISRTPEVTCVVVDVS HEDPEVKFNWYVDGVEVHNAKTKPRE EQYNSTYRVVSVLTVLHQDWLNGKEY KCKVSNKALPAPIEKTISKAKGQPREPQ VCTLPPSREEMTKNQVSLSCAVKGFYP SDIAVEWESNGQPENNYKTTPPVLDS DGSFFLVSKLTVDKSRWQQGNVFSCS VMHEALHNHYTQKSLSLSPGK** | DIQMTQSPSSLSASVGDRVT ITCRASQDVNTAVAWYQQK PGKAPKLLIYSASFLYSGVPSR FSGSRSGTDFTLTISSLQPEDF ATYYCQQHYTTPPTFGQGTK VEIKRTVAAPSVFIFPPSDEQL KSGTASVVCLLNNFYPREAK VQWKVDNALQSGNSQESV TEQDSKDSTYSLSSTLTLSKA DYEKHKVYACEVTHQGLSSP VTKSFNRGECGQPREPQVYT LPPSREEMTKNQVSLWCLVK GFYPSDIAVEWESNGQPEN NYKTTPPVLDSDGSFFLYSKL TVDKSRWQQGNVFSCSVM HEALHNHYTQKSLSLSPGK** |

**[Table 5]**

| | | | | |
|---|---|---|---|---|
| [B] | EVQLVESGGGLVQPGGSLRL SCAASGYTFTNYGMNWVR QAPGKGLEWVGWINTYTGE PTYAADFKRRFTFSLDTSKST AYLQMNSLRAEDTAVYYCAK YPHYYGSSHWYFDVWGQG TLVTVSSASTKGPSVFPLAPS SKSTSGGTAALGCLVKDYFPE PVTVSWNSGALTSGVHTFPA VLQSSGLYSLSSVVTVPSSSL GTQTYICNVNHKPSNTKVDK KVEPKSCDKTHTCPPCPAPEL LGGPSVFLFPPKPKDTLMISR TPEVTCVVVDVSHEDPEVKF NWYVDGVEVHNAKTKPREE QYNSTYRVVSVLTVLHQDW LNGKEYKCKVSNKALPAPIEK TISKAKGQPREPQVYTLPPCR EEMTKNQVSLWCLVKGFYPS DIAVEWESNGQPENNYKTT PPVLDSDGSFFLYSKLTVDKS RWQQGNVFSCSVMHEALH NHYTQKSLSLSPGK | DIQMTQSPSSLSASVGDR VTITCSASQDISNYLNWY QQKPGKAPKVLIYFTSSLH SGVPSRFSGSGSGTDFTLT ISSLQPEDFATYYCQQYST VPWTFGQGTKVEIKRTVA APSVFIFPPSDEQLKSGTA SVVCLLNNFYPREAKVQ WKVDNALQSGNSQESVT EQDSKDSTYSLSSTLTLSK ADYEKHKVYACEVTHQGL SSPVTKSFNRGEC | EVQLVESGGGLVQPGGSLRLSCAASGF NIKDTYIHWVRQAPGKGLEWVARIYPT NGYTRYADSVKGRFTISADTSKNTAYLQ MNSLRAEDTAVYYCSRWGGDGFYAM DYWGQGTLVTVSSASTKGPSVFPLAPC SRSTSESTAALGCLVKDYFPEPVTVSW NSGALTSGVHTFPAVLQSSGLYSLSSVV TVPSSNFGTQTYTCNVDHKPSNTKVD KTVGQPREPQVYTLPPSREEMTKNQV SLSCAVKGFYPSDIAVEWESNGQPENN YKTTPPVLDSDGSFFLVSKLTVDKSRW QQGNVFSCSVMHEALHNHYTQKSLSL SPGKDKTHTCPPCPAPELLGGPSVFLFP PKPKDTLMISRTPEVTCVVVDVSHEDP EVKFNWYVDGVEVHNAKTKPREEQY NSTYRVVSVLTVLHQDWLNGKEYKCK VSNKALPAPIEKTISKAKGQPREPQVCT LPPSREEMTKNQVSLSCAVKGFYPSDIA VEWESNGQPENNYKTTPPVLDSDGSF FLVSKLTVDKSRWQQGNVFSCSVMHE ALHNHYTQKSLSLSPGK** | DIQMTQSPSSLSASVGDRVT ITCRASQDVNTAVAWYQQK PGKAPKLLIYSASFLYSGVPSR FSGSRSGTDFTLTISSLQPEDF ATYYCQQHYTTPPTFGQGTK VEIKRTVAAPSVFIFPPSDEQL KSGTASVVCLLNNFYPREAK VQWKVDNALQSGNSQESV TEQDSKDSTYSLSSTLTLSKA DYEKHKVYACEVTHQGLSSP VTKSFNRGECGQPREPQVYT LPPSREEMTKNQVSLWCLVK GFYPSDIAVEWESNGQPEN NYKTTPPVLDSDGSFFLYSKL TVDKSRWQQGNVFSCSVM HEALHNHYTQKSLSLSPGK** |

**[Table 6]**

| | | | | |
|---|---|---|---|---|
| [C] | EVQLVESGGGLVQPGGSLRL SCAASGYTFTNYGMNWVR QAPGKGLEWVGWINTYTGE PTYAADFKRRFTFSLDTSKST AYLQMNSLRAEDTAVYYCAK YPHYYGSSHWYFDVWGQG TLVTVSSASTKGPSVFPLAPS SKSTSGGTAALGCLVKDYFPE PVTVSWNSGALTSGVHTFPA VLQSSGLYSLSSVVTVPSSSL GTQTYICNVNHKPSNTKVDK KVEPKSCDKTHTCPPCPAPEL LGGPSVFLFPPKPKDTLMISR TPEVTCVVVDVSHEDPEVKF NWYVDGVEVHNAKTKPREE QYNSTYRVVSVLTVLHQDW LNGKEYKCKVSNKALPAPIEK TISKAKGQPREPQVYTLPPCR EEMTKNQVSLWCLVKGFYPS DIAVEWESNGQPENNYKTT PPVLDSDGSFFLYSKLTVDKS RWQQGNVFSCSVMHEALH NHYTQKSLSLSPGK | DIQMTQSPSSLSASVGDR VTITCSASQDISNYLNWY QQKPGKAPKVLIYFTSSLH SGVPSRFSGSGSGTDFTLT ISSLQPEDFATYYCQQYST VPWTFGQGTKVEIKRTVA APSVFIFPPSDEQLKSGTA SVVCLLNNFYPREAKVQ WKVDNALQSGNSQESVT EQDSKDSTYSLSSTLTLSK ADYEKHKVYACEVTHQGL SSPVTKSFNRGEC | EVQLVESGGGLVQPGGSLRLSCAASGF NIKDTYIHWVRQAPGKGLEWVARIYPT NGYTRYADSVKGRFTISADTSKNTAYLQ MNSLRAEDTAVYYCSRWGGDGFYAM DYWGQGTLVTVSSASTKGPSVFPLAPC SRSTSESTAALGCLVKDYFPEPVTVSW NSGALTSGVHTFPAVLQSSGLYSLSSVV TVPSSSLGTKTYTCNVDHKPSNTKVDK RVGQPREPQVYTLPPSREEMTKNQVS LSCAVKGFYPSDIAVEWESNGQPENNY KTTPPVLDSDGSFFLVSKLTVDKSRWQ QGNVFSCSVMHEALHNHYTQKSLSLS PGKDKTHTCPPCPAPELLGGPSVFLFPP KPKDTLMISRTPEVTCVVVDVSHEDPE VKFNWYVDGVEVHNAKTKPREEQYN STYRVVSVLTVLHQDWLNGKEYKCKVS NKALPAPIEKTISKAKGQPREPQVCTLP PSREEMTKNQVSLSCAVKGFYPSDIAV EWESNGQPENNYKTTPPVLDSDGSFF LVSKLTVDKSRWQQGNVFSCSVMHEA LHNHYTQKSLSLSPGK | DIQMTQSPSSLSASVGDRVT ITCRASQDVNTAVAWYQQK PGKAPKLLIYSASFLYSGVPSR FSGSRSGTDFTLTISSLQPEDF ATYYCQQHYTTPPTFGQGTK VEIKRTVAAPSVFIFPPSDEQL KSGTASVVCLLNNFYPREAK VQWKVDNALQSGNSQESV TEQDSKDSTYSLSSTLTLSKA DYEKHKVYACEVTHQGLSSP VTKSFNRGECGQPREPQVYT LPPSREEMTKNQVSLWCLVK GFYPSDIAVEWESNGQPEN NYKTTPPVLDSDGSFFLYSKL TVDKSRWQQGNVFSCSVM HEALHNHYTQKSLSLSPGK |

**[Table 7**

| | | | | |
|---|---|---|---|---|
| [D] | EVQLVESGGGLVQPGGSLRL SCAASGYTFTNYGMNWVR QAPGKGLEWVGWINTYTGE PTYAADFKRRFTFSLDTSKST AYLQMNSLRAEDTAVYYCAK YPHYYGSSHWYFDVWGQG TLVTVSSASTKGPSVFPLAPS SKSTSGGTAALGCLVKDYFPE PVTVSWNSGALTSGVHTFPA VLQSSGLYSLSSVVTVPSSSL GTQTYICNVNHKPSNTKVDK KVEPKSCDKTHTCPPCPAPEL LGGPSVFLFPPKPKDTLMISR TPEVTCVVVDVSHEDPEVKF NWYVDGVEVHNAKTKPREE QYNSTYRVVSVLTVLHQDW LNGKEYKCKVSNKALPAPIEK TISKAKGQPREPQVYTLPPCR EEMTKNQVSLWCLVKGFYPS DIAVEWESNGQPENNYKTT PPVLDSDGSFFLYSKLTVDKS RWQQGNVFSCSVMHEALH NHYTQKSLSLSPGK | DIQMTQSPSSLSASVGDR VTITCSASQDISNYLNWY QQKPGKAPKVLIYFTSSLH SGVPSRFSGSGSGTDFTLT ISSLQPEDFATYYCQQYST VPWTFGQGTKVEIKRTVA APSVFIFPPSDEQLKSGTA SVVCLLNNFYPREAKVQ WKVDNALQSGNSQESVT EQDSKDSTYSLSSTLTLSK ADYEKHKVYACEVTHQGL SSPVTKSFNRGEC | EVQLVESGGGLVQPGGSLRLSCAASGF NIKDTYIHWVRQAPGKGLEWVARIYPT NGYTRYADSVKGRFTISADTSKNTAYLQ MNSLRAEDTAVYYCSRWGGDGFYAM DYWGQGTLVTVSSAPTKAPDVFPIISG CRHPKDNSPVVLACLITGYHPTSVTVT WYMGTQSQPQRTFPEIQRRDSYYMTS SQLSTPLQQWRQGEYKCVVQHTASKS KKEIFGQPREPQVYTLPPSREEMTKNQ VSLSCAVKGFYPSDIAVEWESNGQPEN NYKTTPPVLDSDGSFFLVSKLTVDKSR WQQGNVFSCSVMHEALHNHYTQKSL SLSPGKDKTHTCPPCPAPELLGGPSVFL FPPKPKDTLMISRTPEVTCVVVDVSHE DPEVKFNWYVDGVEVHNAKTKPREEQ YNSTYRVVSVLTVLHQDWLNGKEYKC KVSNKALPAPIEKTISKAKGQPREPQVC TLPPSREEMTKNQVSLSCAVKGFYPSDI AVEWESNGQPENNYKTTPPVLDSDGS FFLVSKLTVDKSRWQQGNVFSCSVMH EALHNHYTQKSLSLSPGK | DIQMTQSPSSLSASVGDRVT ITCRASQDVNTAVAWYQQK PGKAPKLLIYSASFLYSGVPSR FSGSRSGTDFTLTISSLQPEDF ATYYCQQHYTTPPTFGQGTK VEIKRTVAAPSVFIFPPSDEQL KSGTASVVCLLNNFYPREAK VQWKVDNALQSGNSQESV TEQDSKDSTYSLSSTLTLSKA DYEKHKVYACEVTHQGLSSP VTKSFNRGECGQPREPQVYT LPPSREEMTKNQVSLWCLVK GFYPSDIAVEWESNGQPEN NYKTTPPVLDSDGSFFLYSKL TVDKSRWQQGNVFSCSVM HEALHNHYTQKSLSLSPGK |

**[Table 8]**

| | | | | |
|---|---|---|---|---|
| [E] | EVQLVESGGGLVQPGGSLRL SCAASGYTFTNYGMNWVR QAPGKGLEWVGWINTYTGE PTYAADFKRRFTFSLDTSKST AYLQMNSLRAEDTAVYYCAK YPHYYGSSHWYFDVWGQG TLVTVSSASTKGPSVFPLAPS SKSTSGGTAALGCLVKDYFPE PVTVSWNSGALTSGVHTFPA VLQSSGLYSLSSVVTVPSSSL GTQTYICNVNHKPSNTKVDK KVEPKSCDKTHTCPPCPAPEL LGGPSVFLFPPKPKDTLMISR TPEVTCVVVDVSHEDPEVKF NWYVDGVEVHNAKTKPREE QYNSTYRVVSVLTVLHQDW LNGKEYKCKVSNKALPAPIEK TISKAKGQPREPQVYTLPPCR EEMTKNQVSLWCLVKGFYPS DIAVEWESNGQPENNYKTT PPVLDSDGSFFLYSKLTVDKS RWQQGNVFSCSVMHEALH NHYTQKSLSLSPGK | DIQMTQSPSSLSASVGDR VTITCSASQDISNYLNWY QQKPGKAPKVLIYFTSSLH SGVPSRFSGSGSGTDFTLT ISSLQPEDFATYYCQQYST VPWTFGQGTKVEIKRTVA APSVFIFPPSDEQLKSGTA SVVCLLNNFYPREAKVQ WKVDNALQSGNSQESVT EQDSKDSTYSLSSTLTLSK ADYEKHKVYACEVTHQGL SSPVTKSFNRGEC | EVQLVESGGGLVQPGGSLRLSCAASGF NIKDTYIHWVRQAPGKGLEWVARIYPT NGYTRYADSVKGRFTISADTSKNTAYLQ MNSLRAEDTAVYYCSRWGGDGFYAM DYWGQGTLVTVSSGSASAPTLFPLVSC ENSPSDTSSVAVGCLAQDFLPDSITFS WKYKNNSDISSTRGFPSVLRGGKYAAT SQVLLPSKDVMQGTDEHVVCKVQHP NGNKEKNVPLPVGQPREPQVYTLPPS REEMTKNQVSLSCAVKGFYPSDIAVEW ESNGQPENNYKTTPPVLDSDGSFFLVS KLTVDKSRWQQGNVFSCSVMHEALH NHYTQKSLSLSPGKDKTHTCPPCPAPE LLGGPSVFLFPPKPKDTLMISRTPEVTCV VVDVSHEDPEVKFNWYVDGVEVHNA KTKPREEQYNSTYRVVSVLTVLHQDWL NGKEYKCKVSNKALPAPIEKTISKAKGQ PREPQVCTLPPSREEMTKNQVSLSCAV KGFYPSDIAVEWESNGQPENNYKTTPP VLDSDGSFFLVSKLTVDKSRWQQGNV FSCSVMHEALHNHYTQKSLSLSPGK | DIQMTQSPSSLSASVGDRVT ITCRASQDVNTAVAWYQQK PGKAPKLLIYSASFLYSGVPSR FSGSRSGTDFTLTISSLQPEDF ATYYCQQHYTTPPTFGQGTK VEIKRTVAAPSVFIFPPSDEQL KSGTASVVCLLNNFYPREAK VQWKVDNALQSGNSQESV TEQDSKDSTYSLSSTLTLSKA DYEKHKVYACEVTHQGLSSP VTKSFNRGECGQPREPQVYT LPPSREEMTKNQVSLWCLVK GFYPSDIAVEWESNGQPEN NYKTTPPVLDSDGSFFLYSKL TVDKSRWQQGNVFSCSVM HEALHNHYTQKSLSLSPGK |

**[Table 9]**

| | | | | |
|---|---|---|---|---|
| [P] | EVQLVESGGGLVQPGGSLRL SCAASGYTFTNYGMNWVR QAPGKGLEWVGWINTYTGE PTYAADFKRRFTFSLDTSKST AYLQMNSLRAEDTAVYYCAK YPHYYGSSHWYFDVWGQG TLVTVSSASTKGPSVFPLAPS SKSTSGGTAALGCLVKDYFPE PVTVSWNSGALTSGVHTFPA VLQSSGLYSLSSVVTVPSSSL GTQTYICNVNHKPSNTKVDK KVEPKSCDKTHTCPPCPAPEL LGGPSVFLFPPKPKDTLMISR TPEVTCVVVDVSHEDPEVKF NWYVDGVEVHNAKTKPREE QYNSTYRVVSVLTVLHQDW LNGKEYKCKVSNKALPAPIEK TISKAKGQPREPQVYTLPPCR EEMTKNQVSLWCLVKGFYPS DIAVEWESNGQPENNYKTT PPVLDSDGSFFLYSKLTVDKS RWQQGNVFSCSVMHEALH NHYTQKSLSLSPGK | DIQMTQSPSSLSASVGDR VTITCSASQDISNYLNWY QQKPGKAPKVLIYFTSSLH SGVPSRFSGSGSGTDFTLT ISSLQPEDFATYYCQQYST VPWTFGQGTKVEIKRTVA APSVFIFPPSDEQLKSGTA SVVCLLNNFYPREAKVQ WKVDNALQSGNSQESVT EQDSKDSTYSLSSTLTLSK ADYEKHKVYACEVTHQGL SSPVTKSFNRGEC | EVQLVESGGGLVQPGGSLRLSCAASGF NIKDTYIHWVRQAPGKGLEWVARIYPT NGYTRYADSVKGRFTISADTSKNTAYLQ MNSLRAEDTAVYYCSRWGGDGFYAM DYWGQGTLVTVSSASTKGPSVFPLAPS SKSTSGGTAALGCLVKDYFPEPVTVSW NSGALTSGVHTFPAVLQSSGLYSLSSVV TVPSSSLGTQTYICNVNHKPSNTKVDK KVEPKSCGQPREPQVCTLPPSREEMTK NQVSLSCAVKGFYPSDIAVEWESNGQ PENNYKTTPPVLDSDGSFFLVSKLTVDK SRWQQGNVFSCSVMHEALHNHYTQ KSLSLSPGKDKTHTCPPCPAPELLGGPS VFLFPPKPKDTLMISRTPEVTCVVVDVS HEDPEVKFNWYVDGVEVHNAKTKPRE EQYNSTYRVVSVLTVLHQDWLNGKEY KCKVSNKALPAPIEKTISKAKGQPREPQ VCTLPPSREEMTKNQVSLSCAVKGFYP SDIAVEWESNGQPENNYKTTPPVLDS DGSFFLVSKLTVDKSRWQQGNVFSCS VMHEALHNHYTQKSLSLSPGK | DIQMTQSPSSLSASVGDRVT ITCRASQDVNTAVAWYQQK PGKAPKLLIYSASFLYSGVPSR FSGSRSGTDFTLTISSLQPEDF ATYYCQQHYTTPPTFGQGTK VEIKRTVAAPSVFIFPPSDEQL KSGTASVVCLLNNFYPREAK VQWKVDNALQSGNSQESV TEQDSKDSTYSLSSTLTLSKA DYEKHKVYACEVTHQGLSSP VTKSFNRGECGQPREPQVYT LPPCREEMTKNQVSLWCLVK GFYPSDIAVEWESNGQPEN NYKTTPPVLDSDGSFFLYSKL TVDKSRWQQGNVFSCSVM HEALHNHYTQKSLSLSPGK |

**[Table 10]**

| | | | | |
|---|---|---|---|---|
| [G] | EVQLVESGGGLVQPGGSLRL SCAASGYTFTNYGMNWVR QAPGKGLEWVGWINTYTGE PTYAADFKRRFTFSLDTSKST AYLQMNSLRAEDTAVYYCAK YPHYYGSSHWYFDVWGQG TLVTVSSASTKGPSVFPLAPS SKSTSGGTAALGCLVKDYFPE PVTVSWNSGALTSGVHTFPA VLQSSGLYSLSSVVTVPSSSL GTQTYICNVNHKPSNTKVDK KVEPKSCDKTHTCPPCPAPEL LGGPSVFLFPPKPKDTLMISR TPEVTCVVVDVSHEDPEVKF NWYVDGVEVHNAKTKPREE QYNSTYRVVSVLTVLHQDW LNGKEYKCKVSNKALPAPIEK TISKAKGQPREPQVYTLPPCR EEMTKNQVSLWCLVKGFYPS DIAVEWESNGQPENNYKTT PPVLDSDGSFFLYSKLTVDKS RWQQGNVFSCSVMHEALH NHYTQKSLSLSPGK | DIQMTQSPSSLSASVGDR VTITCSASQDISNYLNWY QQKPGKAPKVLIYFTSSLH SGVPSRFSGSGSGTDFTLT ISSLQPEDFATYYCQQYST VPWTFGQGTKVEIKRTVA APSVFIFPPSDEQLKSGTA SVVCLLNNFYPREAKVQ WKVDNALQSGNSQESVT EQDSKDSTYSLSSTLTLSK ADYEKHKVYACEVTHQGL SSPVTKSFNRGEC | EVQLVESGGGLVQPGGSLRLSCAASGF NIKDTYIHWVRQAPGKGLEWVARIYPT NGYTRYADSVKGRFTISADTSKNTAYLQ MNSLRAEDTAVYYCSRWGGDGFYAM DYWGQGTLVTVSSASTKGPSVFPLAPC SRSTSESTAALGCLVKDYFPEPVTVSW NSGALTSGVHTFPAVLQSSGLYSLSSVV TVPSSSLGTKTYTCNVDHKPSNTKVDK RVGQPREPQVCTLPPSREEMTKNQVS LSCAVKGFYPSDIAVEWESNGQPENNY KTTPPVLDSDGSFFLVSKLTVDKSRWQ QGNVFSCSVMHEALHNHYTQKSLSLS PGKDKTHTCPPCPAPELLGGPSVFLFPP KPKDTLMISRTPEVTCVVVDVSHEDPE VKFNWYVDGVEVHNAKTKPREEQYN STYRVVSVLTVLHQDWLNGKEYKCKVS NKALPAPIEKTISKAKGQPREPQVCTLP PSREEMTKNQVSLSCAVKGFYPSDIAV EWESNGQPENNYKTTPPVLDSDGSFF LVSKLTVDKSRWQQGNVFSCSVMHEA LHNHYTQKSLSLSPGK | DIQMTQSPSSLSASVGDRVT ITCRASQDVNTAVAWYQQK PGKAPKLLIYSASFLYSGVPSR FSGSRSGTDFTLTISSLQPEDF ATYYCQQHYTTPPTFGQGTK VEIKRTVAAPSVFIFPPSDEQL KSGTASVVCLLNNFYPREAK VQWKVDNALQSGNSQESV TEQDSKDSTYSLSSTLTLSKA DYEKHKVYACEVTHQGLSSP VTKSFNRGECGQPREPQVYT LPPCREEMTKNQVSLWCLVK GFYPSDIAVEWESNGQPEN NYKTTPPVLDSDGSFFLYSKL TVDKSRWQQGNVFSCSVM HEALHNHYTQKSLSLSPGK |

**[Table 11]**

| | | | | |
|---|---|---|---|---|
| [H] | EVQLVESGGGLVQPGGSLRL SCAASGYTFTNYGMNWVR QAPGKGLEWVGWINTYTGE PTYAADFKRRFTFSLDTSKST AYLQMNSLRAEDTAVYYCAK YPHYYGSSHWYFDVWGQG TLVTVSSASTKGPSVFPLAPS SKSTSGGTAALGCLVKDYFPE PVTVSWNSGALTSGVHTFPA VLQSSGLYSLSSVVTVPSSSL GTQTYICNVNHKPSNTKVDK KVEPKSCDKTHTCPPCPAPEL LGGPSVFLFPPKPKDTLMISR TPEVTCVVVDVSHEDPEVKF NWYVDGVEVHNAKTKPREE QYNSTYRVVSVLTVLHQDW LNGKEYKCKVSNKALPAPIEK TISKAKGQPREPQVYTLPPCR EEMTKNQVSLWCLVKGFYPS DIAVEWESNGQPENNYKTT PPVLDSDGSFFLYSKLTVDKS RWQQGNVFSCSVMHEALH NHYTQKSLSLSPGK | DIQMTQSPSSLSASVGDR VTITCSASQDISNYLNWY QQKPGKAPKVLIYFTSSLH SGVPSRFSGSGSGTDFTLT ISSLQPEDFATYYCQQYST VPWTFGQGTKVEIKRTVA APSVFIFPPSDEQLKSGTA SVVCLLNNFYPREAKVQ WKVDNALQSGNSQESVT EQDSKDSTYSLSSTLTLSK ADYEKHKVYACEVTHQGL SSPVTKSFNRGEC | EVQLVESGGGLVQPGGSLRLSCAASGF NIKDTYIHWVRQAPGKGLEWVARIYPT NGYTRYADSVKGRFTISADTSKNTAYLQ MNSLRAEDTAVYYCSRWGGDGFYAM DYWGQGTLVTVSSASPREPQVYTLPPS REEMTKNQVSLSCAVKGFYPSDIAVEW ESNGQPENNYKTTPPVLDSDGSFFLVS KLTVDKSRWQQGNVFSCSVMHEALH NHYTQKSLSLSPGKASTKGPSVFPLAPS SKSTSGGTAALGCLVKDYFPEPVTVSW NSGALTSGVHTFPAVLQSSGLYSLSSVV TVPSSSLGTQTYICNVNHKPSNTKVDK KVEPKSCDKTHTCPPCPAPELLGGPSVF LFPPKPKDTLMISRTPEVTCVVVDVSHE DPEVKFNWYVDGVEVHNAKTKPREEQ YNSTYRVVSVLTVLHQDWLNGKEYKC KVSNKALPAPIEKTISKAKGQPREPQVC TLPPSREEMTKNQVSLSCAVKGFYPSDI AVEWESNGQPENNYKTTPPVLDSDGS FFLVSKLTVDKSRWQQGNVFSCSVMH EALHNHYTQKSLSLSPGK** | DIQMTQSPSSLSASVGDRVT ITCRASQDVNTAVAWYQQK PGKAPKLLIYSASFLYSGVPSR FSGSRSGTDFTLTISSLQPEDF ATYYCQQHYTTPPTFGQGTK VEIKRTPREPQVYTLPPSREE MTKNQVSLWCLVKGFYPSDI AVEWESNGQPENNYKTTPP VLDSDGSFFLYSKLTVDKSR WQQGNVFSCSVMHEALHN HYTQKSLSLSPGKRTVAAPSV FIFPPSDEQLKSGTASVVCLL NNFYPREAKVQWKVDNAL QSGNSQESVTEQDSKDSTY SLSSTLTLSKADYEKHKVYAC EVTHQGLSSPVTKSFNRGEC ** |

**[Table 12]**

| | | | | |
|---|---|---|---|---|
| [I] | EVQLVESGGGLVQPGGSLRL SCAASGYTFTNYGMNWVR QAPGKGLEWVGWINTYTGE PTYAADFKRRFTFSLDTSKST AYLQMNSLRAEDTAVYYCAK YPHYYGSSHWYFDVWGQG TLVTVSSASTKGPSVFPLAPS SKSTSGGTAALGCLVKDYFPE PVTVSWNSGALTSGVHTFPA VLQSSGLYSLSSVVTVPSSSL GTQTYICNVNHKPSNTKVDK KVEPKSCDKTHTCPPCPAPEL LGGPSVFLFPPKPKDTLMISR TPEVTCVVVDVSHEDPEVKF NWYVDGVEVHNAKTKPREE QYNSTYRVVSVLTVLHQDW LNGKEYKCKVSNKALPAPIEK TISKAKGQPREPQVYTLPPCR EEMTKNQVSLWCLVKGFYPS DIAVEWESNGQPENNYKTT PPVLDSDGSFFLYSKLTVDKS RWQQGNVFSCSVMHEALH NHYTQKSLSLSPGK | DIQMTQSPSSLSASVGDR VTITCSASQDISNYLNWY QQKPGKAPKVLIYFTSSLH SGVPSRFSGSGSGTDFTLT ISSLQPEDFATYYCQQYST VPWTFGQGTKVEIKRTVA APSVFIFPPSDEQLKSGTA SVVCLLNNFYPREAKVQ WKVDNALQSGNSQESVT EQDSKDSTYSLSSTLTLSK ADYEKHKVYACEVTHQGL SSPVTKSFNRGEC | EVQLVESGGGLVQPGGSLRLSCAASGF NIKDTYIHWVRQAPGKGLEWVARIYPT NGYTRYADSVKGRFTISADTSKNTAYLQ MNSLRAEDTAVYYCSRWGGDGFYAM DYWGQGTLVTVSSASPREPQVYTLPPS REEMTKNQVSLSCAVKGFYPSDIAVEW ESNGQPENNYKTTPPVLDSDGSFFLVS KLTVDKSRWQQGNVFSCSVMHEALH NHYTQKSLSLSPGKASTKGPSVFPLAP CSRSTSESTAALGCLVKDYFPEPVTVSW NSGALTSGVHTFPAVLQSSGLYSLSSVV TVPSSSLGTKTYTCNVDHKPSNTKVDK RVDKTHTCPPCPAPELLGGPSVFLFPPK PKDTLMISRTPEVTCVVVDVSHEDPEV KFNWYVDGVEVHNAKTKPREEQYNST YRVVSVLTVLHQDWLNGKEYKCKVSN KALPAPIEKTISKAKGQPREPQVCTLPPS REEMTKNQVSLSCAVKGFYPSDIAVEW ESNGQPENNYKTTPPVLDSDGSFFLVS KLTVDKSRWQQGNVFSCSVMHEALH NHYTQKSLSLSPGK | DIQMTQSPSSLSASVGDRVT ITCRASQDVNTAVAWYQQK PGKAPKLLIYSASFLYSGVPSR FSGSRSGTDFTLTISSLQPEDF ATYYCQQHYTTPPTFGQGTK VEIKRTPREPQVYTLPPSREE MTKNQVSLWCLVKGFYPSDI AVEWESNGQPENNYKTTPP VLDSDGSFFLYSKLTVDKSR WQQGNVFSCSVMHEALHN HYTQKSLSLSPGKRTVAAPSV FIFPPSDEQLKSGTASVVCLL NNFYPREAKVQWKVDNAL QSGNSQESVTEQDSKDSTY SLSSTLTLSKADYEKHKVYAC EVTHQGLSSPVTKSFNRGEC |

**[Table 13]**

| | | | | |
|---|---|---|---|---|
| [J] | EVQLVESGGGLVQPGGSLRL SCAASGYTFTNYGMNWVR QAPGKGLEWVGWINTYTGE PTYAADFKRRFTFSLDTSKST AYLQMNSLRAEDTAVYYCAK YPHYYGSSHWYFDVWGQG TLVTVSSASTKGPSVFPLAPS SKSTSGGTAALGCLVKDYFPE PVTVSWNSGALTSGVHTFPA VLQSSGLYSLSSVVTVPSSSL GTQTYICNVNHKPSNTKVDK KVEPKSCDKTHTCPPCPAPEL LGGPSVFLFPPKPKDTLMISR TPEVTCVVVDVSHEDPEVKF NWYVDGVEVHNAKTKPREE QYNSTYRVVSVLTVLHQDW LNGKEYKCKVSNKALPAPIEK TISKAKGQPREPQVYTLPPCR EEMTKNQVSLWCLVKGFYPS DIAVEWESNGQPENNYKTT PPVLDSDGSFFLYSKLTVDKS RWQQGNVFSCSVMHEALH NHYTQKSLSLSPGK | DIQMTQSPSSLSASVGDR VTITCSASQDISNYLNWY QQKPGKAPKVLIYFTSSLH SGVPSRFSGSGSGTDFTLT ISSLQPEDFATYYCQQYST VPWTFGQGTKVEIKRTVA APSVFIFPPSDEQLKSGTA SVVCLLNNFYPREAKVQ WKVDNALQSGNSQESVT EQDSKDSTYSLSSTLTLSK ADYEKHKVYACEVTHQGL SSPVTKSFNRGEC | EVQLVESGGGLVQPGGSLRLSCAASGF NIKDTYIHWVRQAPGKGLEWVARIYPT NGYTRYADSVKGRFTISADTSKNTAYLQ MNSLRAEDTAVYYCSRWGGDGFYAM DYWGQGTLVTVSSASPREPQVYTLPPS REEMTKNQVSLSCAVKGFYPSDIAVEW ESNGQPENNYKTTPPVLDSDGSFFLVS KLTVDKSRWQQGNVFSCSVMHEALH NHYTQKSLSLSPGKAPTKAPDVFPIISG CRHPKDNSPVVLACLITGYHPTSVTVT WYMGTQSQPQRTFPEIQRRDSYYMTS SQLSTPLQQWRQGEYKCVVQHTASKS KKEIFDKTHTCPPCPAPELLGGPSVFLFP PKPKDTLMISRTPEVTCVVVDVSHEDP EVKFNWYVDGVEVHNAKTKPREEQY NSTYRVVSVLTVLHQDWLNGKEYKCK VSNKALPAPIEKTISKAKGQPREPQVCT LPPSREEMTKNQVSLSCAVKGFYPSDIA VEWESNGQPENNYKTTPPVLDSDGSF FLVSKLTVDKSRWQQGNVFSCSVMHE ALHNHYTQKSLSLSPGK | DIQMTQSPSSLSASVGDRVT ITCRASQDVNTAVAWYQQK PGKAPKLLIYSASFLYSGVPSR FSGSRSGTDFTLTISSLQPEDF ATYYCQQHYTTPPTFGQGTK VEIKRTPREPQVYTLPPSREE MTKNQVSLWCLVKGFYPSDI AVEWESNGQPENNYKTTPP VLDSDGSFFLYSKLTVDKSR WQQGNVFSCSVMHEALHN HYTQKSLSLSPGKRTVAAPSV FIFPPSDEQLKSGTASVVCLL NNFYPREAKVQWKVDNAL QSGNSQESVTEQDSKDSTY SLSSTLTLSKADYEKHKVYAC EVTHQGLSSPVTKSFNRGEC |

**[Table 14]**

| | | | | |
|---|---|---|---|---|
| [K] | EVQLVESGGGLVQPGGSLRL SCAASGYTFTNYGMNWVR QAPGKGLEWVGWINTYTGE PTYAADFKRRFTFSLDTSKST AYLQMNSLRAEDTAVYYCAK YPHYYGSSHWYFDVWGQG TLVTVSSASTKGPSVFPLAPS SKSTSGGTAALGCLVKDYFPE PVTVSWNSGALTSGVHTFPA VLQSSGLYSLSSVVTVPSSSL GTQTYICNVNHKPSNTKVDK KVEPKSCDKTHTCPPCPAPEL LGGPSVFLFPPKPKDTLMISR TPEVTCVVVDVSHEDPEVKF NWYVDGVEVHNAKTKPREE QYNSTYRVVSVLTVLHQDW LNGKEYKCKVSNKALPAPIEK TISKAKGQPREPQVYTLPPCR EEMTKNQVSLWCLVKGFYPS DIAVEWESNGQPENNYKTT PPVLDSDGSFFLYSKLTVDKS RWQQGNVFSCSVMHEALH NHYTQKSLSLSPGK | DIQMTQSPSSLSASVGDR VTITCSASQDISNYLNWY QQKPGKAPKVLIYFTSSLH SGVPSRFSGSGSGTDFTLT ISSLQPEDFATYYCQQYST VPWTFGQGTKVEIKRTVA APSVFIFPPSDEQLKSGTA SVVCLLNNFYPREAKVQ WKVDNALQSGNSQESVT EQDSKDSTYSLSSTLTLSK ADYEKHKVYACEVTHQGL SSPVTKSFNRGEC | EVQLVESGGGLVQPGGSLRLSCAASGF NIKDTYIHWVRQAPGKGLEWVARIYPT NGYTRYADSVKGRFTISADTSKNTAYLQ MNSLRAEDTAVYYCSRWGGDGFYAM DYWGQGTLVTVSSASPREPQVYTLPPS REEMTKNQVSLSCAVKGFYPSDIAVEW ESNGQPENNYKTTPPVLDSDGSFFLVS KLTVDKSRWQQGNVFSCSVMHEALH NHYTQKSLSLSPGKGSASAPTLFPLVSC ENSPSDTSSVAVGCLAQDFLPDSITFS WKYKNNSDISSTRGFPSVLRGGKYAAT SQVLLPSKDVMQGTDEHVVCKVQHP NGNKEKNVPLPVDKTHTCPPCPAPELL GGPSVFLFPPKPKDTLMISRTPEVTCVV VDVSHEDPEVKFNWYVDGVEVHNAK TKPREEQYNSTYRVVSVLTVLHQDWL NGKEYKCKVSNKALPAPIEKTISKAKGQ PREPQVCTLPPSREEMTKNQVSLSCAV KGFYPSDIAVEWESNGQPENNYKTTPP VLDSDGSFFLVSKLTVDKSRWQQGNV FSCSVMHEALHNHYTQKSLSLSPGK | DIQMTQSPSSLSASVGDRVT ITCRASQDVNTAVAWYQQK PGKAPKLLIYSASFLYSGVPSR FSGSRSGTDFTLTISSLQPEDF ATYYCQQHYTTPPTFGQGTK VEIKRTPREPQVYTLPPSREE MTKNQVSLWCLVKGFYPSDI AVEWESNGQPENNYKTTPP VLDSDGSFFLYSKLTVDKSR WQQGNVFSCSVMHEALHN HYTQKSLSLSPGKRTVAAPSV FIFPPSDEQLKSGTASVVCLL NNFYPREAKVQWKVDNAL QSGNSQESVTEQDSKDSTY SLSSTLTLSKADYEKHKVYAC EVTHQGLSSPVTKSFNRGEC |

**[Table 15]**

| | | | | |
|---|---|---|---|---|
| [L] | EVQLVESGGGLVQPGGSLRL SCAASGYTFTNYGMNWVR QAPGKGLEWVGWINTYTGE PTYAADFKRRFTFSLDTSKST AYLQMNSLRAEDTAVYYCAK YPHYYGSSHWYFDVWGQG TLVTVSSASTKGPSVFPLAPS SKSTSGGTAALGCLVKDYFPE PVTVSWNSGALTSGVHTFPA VLQSSGLYSLSSVVTVPSSSL GTQTYICNVNHKPSNTKVDK KVEPKSCDKTHTCPPCPAPEL LGGPSVFLFPPKPKDTLMISR TPEVTCVVVDVSHEDPEVKF NWYVDGVEVHNAKTKPREE QYNSTYRVVSVLTVLHQDW LNGKEYKCKVSNKALPAPIEK TISKAKGQPREPQVYTLPPCR EEMTKNQVSLWCLVKGFYPS DIAVEWESNGQPENNYKTT PPVLDSDGSFFLYSKLTVDKS RWQQGNVFSCSVMHEALH NHYTQKSLSLSPGK | DIQMTQSPSSLSASVGDR VTITCSASQDISNYLNWY QQKPGKAPKVLIYFTSSLH SGVPSRFSGSGSGTDFTLT ISSLQPEDFATYYCQQYST VPWTFGQGTKVEIKRTVA APSVFIFPPSDEQLKSGTA SVVCLLNNFYPREAKVQ WKVDNALQSGNSQESVT EQDSKDSTYSLSSTLTLSK ADYEKHKVYACEVTHQGL SSPVTKSFNRGEC | EVQLVESGGGLVQPGGSLRLSCAASGF NIKDTYIHWVRQAPGKGLEWVARIYPT NGYTRYADSVKGRFTISADTSKNTAYLQ MNSLRAEDTAVYYCSRWGGDGFYAM DYWGQGTLVTVSSASPREPQVCTLPPS REEMTKNQVSLSCAVKGFYPSDIAVEW ESNGQPENNYKTTPPVLDSDGSFFLVS KLTVDKSRWQQGNVFSCSVMHEALH NHYTQKSLSLSPGKASTKGPSVFPLAPS SKSTSGGTAALGCLVKDYFPEPVTVSW NSGALTSGVHTFPAVLQSSGLYSLSSVV TVPSSSLGTQTYICNVNHKPSNTKVDK KVEPKSCDKTHTCPPCPAPELLGGPSVF LFPPKPKDTLMISRTPEVTCVVVDVSHE DPEVKFNWYVDGVEVHNAKTKPREEQ YNSTYRVVSVLTVLHQDWLNGKEYKC KVSNKALPAPIEKTISKAKGQPREPQVC TLPPSREEMTKNQVSLSCAVKGFYPSDI AVEWESNGQPENNYKTTPPVLDSDGS FFLVSKLTVDKSRWQQGNVFSCSVMH EALHNHYTQKSLSLSPGK | DIQMTQSPSSLSASVGDRVT ITCRASQDVNTAVAWYQQK PGKAPKLLIYSASFLYSGVPSR FSGSRSGTDFTLTISSLQPEDF ATYYCQQHYTTPPTFGQGTK VEIKRTPREPQVYTLPPCREE MTKNQVSLWCLVKGFYPSDI AVEWESNGQPENNYKTTPP VLDSDGSFFLYSKLTVDKSR WQQGNVFSCSVMHEALHN HYTQKSLSLSPGKRTVAAPSV FIFPPSDEQLKSGTASVVCLL NNFYPREAKVQWKVDNAL QSGNSQESVTEQDSKDSTY SLSSTLTLSKADYEKHKVYAC EVTHQGLSSPVTKSFNRGEC |

**[Table 16]**

| | | | | |
|---|---|---|---|---|
| [M] | EVQLVESGGGLVQPGGSLRL SCAASGYTFTNYGMNWVR QAPGKGLEWVGWINTYTGE PTYAADFKRRFTFSLDTSKST AYLQMNSLRAEDTAVYYCAK YPHYYGSSHWYFDVWGQG TLVTVSSASTKGPSVFPLAPS SKSTSGGTAALGCLVKDYFPE PVTVSWNSGALTSGVHTFPA VLQSSGLYSLSSVVTVPSSSL GTQTYICNVNHKPSNTKVDK KVEPKSCDKTHTCPPCPAPEL LGGPSVFLFPPKPKDTLMISR TPEVTCVVVDVSHEDPEVKF NWYVDGVEVHNAKTKPREE QYNSTYRVVSVLTVLHQDW LNGKEYKCKVSNKALPAPIEK TISKAKGQPREPQVYTLPPCR EEMTKNQVSLWCLVKGFYPS DIAVEWESNGQPENNYKTT PPVLDSDGSFFLYSKLTVDKS RWQQGNVFSCSVMHEALH NHYTQKSLSLSPGK | DIQMTQSPSSLSASVGDR VTITCSASQDISNYLNWY QQKPGKAPKVLIYFTSSLH SGVPSRFSGSGSGTDFTLT ISSLQPEDFATYYCQQYST VPWTFGQGTKVEIKRTVA APSVFIFPPSDEQLKSGTA SVVCLLNNFYPREAKVQ WKVDNALQSGNSQESVT EQDSKDSTYSLSSTLTLSK ADYEKHKVYACEVTHQGL SSPVTKSFNRGEC | EVQLVESGGGLVQPGGSLRLSCAASGF NIKDTYIHWVRQAPGKGLEWVARIYPT NGYTRYADSVKGRFTISADTSKNTAYLQ MNSLRAEDTAVYYCSRWGGDGFYAM DYWGQGTLVTVSSASPREPQVCTLPPS REEMTKNQVSLSCAVKGFYPSDIAVEW ESNGQPENNYKTTPPVLDSDGSFFLVS KLTVDKSRWQQGNVFSCSVMHEALH NHYTQKSLSLSPGKASTKGPSVFPLAP CSRSTSESTAALGCLVKDYFPEPVTVSW NSGALTSGVHTFPAVLQSSGLYSLSSVV TVPSSSLGTKTYTCNVDHKPSNTKVDK RVDKTHTCPPCPAPELLGGPSVFLFPPK PKDTLMISRTPEVTCVVVDVSHEDPEV KFNWYVDGVEVHNAKTKPREEQYNST YRVVSVLTVLHQDWLNGKEYKCKVSN KALPAPIEKTISKAKGQPREPQVCTLPPS REEMTKNQVSLSCAVKGFYPSDIAVEW ESNGQPENNYKTTPPVLDSDGSFFLVS KLTVDKSRWQQGNVFSCSVMHEALH NHYTQKSLSLSPGK | DIQMTQSPSSLSASVGDRVT ITCRASQDVNTAVAWYQQK PGKAPKLLIYSASFLYSGVPSR FSGSRSGTDFTLTISSLQPEDF ATYYCQQHYTTPPTFGQGTK VEIKRTPREPQVYTLPPCREE MTKNQVSLWCLVKGFYPSDI AVEWESNGQPENNYKTTPP VLDSDGSFFLYSKLTVDKSR WQQGNVFSCSVMHEALHN HYTQKSLSLSPGKRTVAAPSV FIFPPSDEQLKSGTASVVCLL NNFYPREAKVQWKVDNAL QSGNSQESVTEQDSKDSTY SLSSTLTLSKADYEKHKVYAC EVTHQGLSSPVTKSFNRGEC |

**[Table 17]**

| | | | | |
|---|---|---|---|---|
| [N] | EVQLVESGGGLVQPGGSLRL SCAASGYTFTNYGMNWVR QAPGKGLEWVGWINTYTGE PTYAADFKRRFTFSLDTSKST AYLQMNSLRAEDTAVYYCAK YPHYYGSSHWYFDVWGQG TLVTVSSASTKGPSVFPLAPS SKSTSGGTAALGCLVKDYFPE PVTVSWNSGALTSGVHTFPA VLQSSGLYSLSSVVTVPSSSL GTQTYICNVNHKPSNTKVDK KVEPKSCDKTHTCPPCPAPEL LGGPSVFLFPPKPKDTLMISR TPEVTCVVVDVSHEDPEVKF NWYVDGVEVHNAKTKPREE QYNSTYRVVSVLTVLHQDW LNGKEYKCKVSNKALPAPIEK TISKAKGQPREPQVYTLPPCR EEMTKNQVSLWCLVKGFYPS DIAVEWESNGQPENNYKTT PPVLDSDGSFFLYSKLTVDKS RWQQGNVFSCSVMHEALH NHYTQKSLSLSPGK | DIQMTQSPSSLSASVGDR VTITCSASQDISNYLNWY QQKPGKAPKVLIYFTSSLH SGVPSRFSGSGSGTDFTLT ISSLQPEDFATYYCQQYST VPWTFGQGTKVEIKRTVA APSVFIFPPSDEQLKSGTA SVVCLLNNFYPREAKVQ WKVDNALQSGNSQESVT EQDSKDSTYSLSSTLTLSK ADYEKHKVYACEVTHQGL SSPVTKSFNRGEC | EVQLVESGGGLVQPGGSLRLSCAASGF NIKDTYIHWVRQAPGKGLEWVARIYPT NGYTRYADSVKGRFTISADTSKNTAYLQ MNSLRAEDTAVYYCSRWGGDGFYAM DYWGQGTLVTVSSASTAIRVFAIPPSFA SIFLTKSTKLTCLVTDLTTYDSVTISWTR QNGEAVKTHTNISESHPNATFSAVGEA SICEDDWNSGERFTCTVTHTDLPSPLK QTISRPKGDKTHTCPPCPAPELLGGPSV FLFPPKPKDTLMISRTPEVTCVVVDVSH EDPEVKFNWYVDGVEVHNAKTKPREE QYNSTYRVVSVLTVLHQDWLNGKEYK CKVSNKALPAPIEKTISKAKGQPREPQV CTLPPSREEMTKNQVSLSCAVKGFYPS DIAVEWESNGQPENNYKTTPPVLDSD GSFFLVSKLTVDKSRWQQGNVFSCSV MHEALHNHYTQKSLSLSPGK | DIQMTQSPSSLSASVGDRVT ITCRASQDVNTAVAWYQQK PGKAPKLLIYSASFLYSGVPSR FSGSRSGTDFTLTISSLQPEDF ATYYCQQHYTTPPTFGQGTK VEIKRTTAIRVFAIPPSFASIFLT KSTKLTCLVTDLTTYDSVTIS WTRQNGEAVKTHTNISESH PNATFSAVGEASICEDDWNS GERFTCTVTHTDLPSPLKQTI SRPKG |

**[Table 18]**

| | | | | |
|---|---|---|---|---|
| [O] | EVQLVESGGGLVQPGGSLRL SCAASGYTFTNYGMNWVR QAPGKGLEWVGWINTYTGE PTYAADFKRRFTFSLDTSKST AYLQMNSLRAEDTAVYYCAK YPHYYGSSHWYFDVWGQG TLVTVSSASTKGPSVFPLAPS SKSTSGGTAALGCLVKDYFPE PVTVSWNSGALTSGVHTFPA VLQSSGLYSLSSVVTVPSSSL GTQTYICNVNHKPSNTKVDK KVEPKSCDKTHTCPPCPAPEL LGGPSVFLFPPKPKDTLMISR TPEVTCVVVDVSHEDPEVKF NWYVDGVEVHNAKTKPREE QYNSTYRVVSVLTVLHQDW LNGKEYKCKVSNKALPAPIEK TISKAKGQPREPQVYTLPPSR EEMTKNQVSLWCLVKGFYPS DIAVEWESNGQPENNYKTT PPVLDSDGSFFLYSKLTVDKS RWQQGNVFSCSVMHEALH NHYTQKSLSLSPGK** | DIQMTQSPSSLSASVGDR VTITCSASQDISNYLNWY QQKPGKAPKVLIYFTSSLH SGVPSRFSGSGSGTDFTLT ISSLQPEDFATYYCQQYST VPWTFGQGTKVEIKRTVA APSVFIFPPSDEQLKSGTA SVVCLLNNFYPREAKVQ WKVDNALQSGNSQESVT EQDSKDSTYSLSSTLTLSK ADYEKHKVYACEVTHQGL SSPVTKSFNRGEC** | EVQLVESGGGLVQPGGSLRLSCAASGF NIKDTYIHWVRQAPGKGLEWVARIYPT NGYTRYADSVKGRFTISADTSKNTAYLQ MNSLRAEDTAVYYCSRWGGDGFYAM DYWGQGTLVTVSSASTKGPSVFPLAPS SKSTSGGTAALGCLVKDYFPEPVTVSW NSGALTSGVHTFPAVLQSSGLYSLSSVV TVPSSSLGTQTYICNVNHKPSNTKVDK KVEPKSC GQPREPQVCTLPPSREEMTKNQVSLSC AVKGFYPSDIAVEWESNGQPENNYKTT PPVLDSDGSFFLVSKLTVDKSRWQQG NVFSCSVMHEALHNHYTQKSLSLSPG K DKTHTCPPCPAPELLGGPSVFLFPPKPK DTLMISRTPEVTCVVVDVSHEDPEVKF NWYVDGVEVHNAKTKPREEQYNSTY RVVSVLTVLHQDWLNGKEYKCKVSNK ALPAPIEKTISKAK GQPREPQVYTLPPSREEMTKNQVSLSC AVKGFYPSDIAVEWESNGQPENNYKTT PPVLDSDGSFFLVSKLTVDKSRWQQG NVFSCSVMHEALHNHYTQKSLSLSPG K | DIQMTQSPSSLSASVGDRVT ITCRASQDVNTAVAWYQQK PGKAPKLLIYSASFLYSGVPSR FSGSRSGTDFTLTISSLQPEDF ATYYCQQHYTTPPTFGQGTK VEIKRTVAAPSVFIFPPSDEQL KSGTASVVCLLNNFYPREAK VQWKVDNALQSGNSQESV TEQDSKDSTYSLSSTLTLSKA DYEKHKVYACEVTHQGLSSP VTKSFNRGEC GQPREPQVYTLPPCREEMTK NQVSLWCLVKGFYPSDIAVE WESNGQPENNYKTTPPVLD SDGSFFLYSKLTVDKSRWQQ GNVFSCSVMHEALHNHYTQ KSLSLSPGK |

**[Table 19]**

| | | | | |
|---|---|---|---|---|
| [P] | EVQLVESGGGLVQPGGSLRL SCAASGYTFTNYGMNWVR QAPGKGLEWVGWINTYTGE PTYAADFKRRFTFSLDTSKST AYLQMNSLRAEDTAVYYCAK YPHYYGSSHWYFDVWGQG TLVTVSSASTKGPSVFPLAPS SKSTSGGTAALGCLVKDYFPE PVTVSWNSGALTSGVHTFPA VLQSSGLYSLSSVVTVPSSSL GTQTYICNVNHKPSNTKVDK KVEPKSCDKTHTCPPCPAPEL LGGPSVFLFPPKPKDTLMISR TPEVTCVVVDVSHEDPEVKF NWYVDGVEVHNAKTKPREE QYNSTYRVVSVLTVLHQDW LNGKEYKCKVSNKALPAPIEK TISKAKGQPREPQVYTLPPSR EEMTKNQVSLWCLVKGFYPS DIAVEWESNGQPENNYKTT PPVLDSDGSFFLYSKLTVDKS RWQQGNVFSCSVMHEALH NHYTQKSLSLSPGK** | DIQMTQSPSSLSASVGDR VTITCSASQDISNYLNWY QQKPGKAPKVLIYFTSSLH SGVPSRFSGSGSGTDFTLT ISSLQPEDFATYYCQQYST VPWTFGQGTKVEIKRTVA APSVFIFPPSDEQLKSGTA SVVCLLNNFYPREAKVQ WKVDNALQSGNSQESVT EQDSKDSTYSLSSTLTLSK ADYEKHKVYACEVTHQGL SSPVTKSFNRGEC** | EVQLVESGGGLVQPGGSLRLSCAASGF NIKDTYIHWVRQAPGKGLEWVARIYPT NGYTRYADSVKGRFTISADTSKNTAYLQ MNSLRAEDTAVYYCSRWGGDGFYAM DYWGQGTLVTVSSASTKGPSVFPLAPC SRSTSESTAALGCLVKDYFPEPVTVSW NSGALTSGVHTFPAVLQSSGLYSLSSVV TVPSSSLGTKTYTCNVDHKPSNTKVDK RVGQPREPQVCTLPPSREEMTKNQVS LSCAVKGFYPSDIAVEWESNGQPENNY KTTPPVLDSDGSFFLVSKLTVDKSRWQ QGNVFSCSVMHEALHNHYTQKSLSLS PGKDKTHTCPPCPAPELLGGPSVFLFPP KPKDTLMISRTPEVTCVVVDVSHEDPE VKFNWYVDGVEVHNAKTKPREEQYN STYRVVSVLTVLHQDWLNGKEYKCKVS NKALPAPIEKTISKAKGQPREPQVYTLP PSREEMTKNQVSLSCAVKGFYPSDIAV EWESNGQPENNYKTTPPVLDSDGSFF LVSKLTVDKSRWQQGNVFSCSVMHEA LHNHYTQKSLSLSPGK | DIQMTQSPSSLSASVGDRVT ITCRASQDVNTAVAWYQQK PGKAPKLLIYSASFLYSGVPSR FSGSRSGTDFTLTISSLQPEDF ATYYCQQHYTTPPTFGQGTK VEIKRTVAAPSVFIFPPSDEQL KSGTASVVCLLNNFYPREAK VQWKVDNALQSGNSQESV TEQDSKDSTYSLSSTLTLSKA DYEKHKVYACEVTHQGLSSP VTKSFNRGECGQPREPQVYT LPPCREEMTKNQVSLWCLVK GFYPSDIAVEWESNGQPEN NYKTTPPVLDSDGSFFLYSKL TVDKSRWQQGNVFSCSVM HEALHNHYTQKSLSLSPGK |

**[Table 20]**

| | | | | |
|---|---|---|---|---|
| [Q] | EVQLVESGGGLVQPGGSLRL SCAASGYTFTNYGMNWVR QAPGKGLEWVGWINTYTGE PTYAADFKRRFTFSLDTSKST AYLQMNSLRAEDTAVYYCAK YPHYYGSSHWYFDVWGQG TLVTVSSASTKGPSVFPLAPS SKSTSGGTAALGCLVKDYFPE PVTVSWNSGALTSGVHTFPA VLQSSGLYSLSSVVTVPSSSL GTQTYICNVNHKPSNTKVDK KVEPKSCDKTHTCPPCPAPEL LGGPSVFLFPPKPKDTLMISR TPEVTCVVVDVSHEDPEVKF NWYVDGVEVHNAKTKPREE QYNSTYRVVSVLTVLHQDW LNGKEYKCKVSNKALPAPIEK TISKAKGQPREPQVYTLPPSR EEMTKNQVSLWCLVKGFYPS DIAVEWESNGQPENNYKTT PPVLDSDGSFFLYSKLTVDKS RWQQGNVFSCSVMHEALH NHYTQKSLSLSPGK** | DIQMTQSPSSLSASVGDR VTITCSASQDISNYLNWY QQKPGKAPKVLIYFTSSLH SGVPSRFSGSGSGTDFTLT ISSLQPEDFATYYCQQYST VPWTFGQGTKVEIKRTVA APSVFIFPPSDEQLKSGTA SVVCLLNNFYPREAKVQ WKVDNALQSGNSQESVT EQDSKDSTYSLSSTLTLSK ADYEKHKVYACEVTHQGL SSPVTKSFNRGEC** | EVQLVESGGGLVQPGGSLRLSCAASGF NIKDTYIHWVRQAPGKGLEWVARIYPT NGYTRYADSVKGRFTISADTSKNTAYLQ MNSLRAEDTAVYYCSRWGGDGFYAM DYWGQGTLVTVSSASPREPQVCTLPPS REEMTKNQVSLSCAVKGFYPSDIAVEW ESNGQPENNYKTTPPVLDSDGSFFLVS KLTVDKSRWQQGNVFSCSVMHEALH NHYTQKSLSLSPGKASTKGPSVFPLAPS SKSTSGGTAALGCLVKDYFPEPVTVSW NSGALTSGVHTFPAVLQSSGLYSLSSVV TVPSSSLGTQTYICNVNHKPSNTKVDK KVEPKSCDKTHTCPPCPAPELLGGPSVF LFPPKPKDTLMISRTPEVTCVVVDVSHE DPEVKFNWYVDGVEVHNAKTKPREEQ YNSTYRVVSVLTVLHQDWLNGKEYKC KVSNKALPAPIEKTISKAKGQPREPQVY TLPPSREEMTKNQVSLSCAVKGFYPSDI AVEWESNGQPENNYKTTPPVLDSDGS FFLVSKLTVDKSRWQQGNVFSCSVMH EALHNHYTQKSLSLSPGK | DIQMTQSPSSLSASVGDRVT ITCRASQDVNTAVAWYQQK PGKAPKLLIYSASFLYSGVPSR FSGSRSGTDFTLTISSLQPEDF ATYYCQQHYTTPPTFGQGTK VEIKRTPREPQVYTLPPCREE MTKNQVSLWCLVKGFYPSDI AVEWESNGQPENNYKTTPP VLDSDGSFFLYSKLTVDKSR WQQGNVFSCSVMHEALHN HYTQKSLSLSPGKRTVAAPSV FIFPPSDEQLKSGTASVVCLL NNFYPREAKVQWKVDNAL QSGNSQESVTEQDSKDSTY SLSSTLTLSKADYEKHKVYAC EVTHQGLSSPVTKSFNRGEC |

**[Table 21]**

| | | | | |
|---|---|---|---|---|
| [R] | EVQLVESGGGLVQPGGSLRL SCAASGYTFTNYGMNWVR QAPGKGLEWVGWINTYTGE PTYAADFKRRFTFSLDTSKST AYLQMNSLRAEDTAVYYCAK YPHYYGSSHWYFDVWGQG TLVTVSSASTKGPSVFPLAPS SKSTSGGTAALGCLVKDYFPE PVTVSWNSGALTSGVHTFPA VLQSSGLYSLSSVVTVPSSSL GTQTYICNVNHKPSNTKVDK KVEPKSCDKTHTCPPCPAPEL LGGPSVFLFPPKPKDTLMISR TPEVTCVVVDVSHEDPEVKF NWYVDGVEVHNAKTKPREE QYNSTYRVVSVLTVLHQDW LNGKEYKCKVSNKALPAPIEK TISKAKGQPREPQVYTLPPSR EEMTKNQVSLWCLVKGFYPS DIAVEWESNGQPENNYKTT PPVLDSDGSFFLYSKLTVDKS RWQQGNVFSCSVMHEALH NHYTQKSLSLSPGK** | DIQMTQSPSSLSASVGDR VTITCSASQDISNYLNWY QQKPGKAPKVLIYFTSSLH SGVPSRFSGSGSGTDFTLT ISSLQPEDFATYYCQQYST VPWTFGQGTKVEIKRTVA APSVFIFPPSDEQLKSGTA SVVCLLNNFYPREAKVQ WKVDNALQSGNSQESVT EQDSKDSTYSLSSTLTLSK ADYEKHKVYACEVTHQGL SSPVTKSFNRGEC** | EVQLVESGGGLVQPGGSLRLSCAASGF NIKDTYIHWVRQAPGKGLEWVARIYPT NGYTRYADSVKGRFTISADTSKNTAYLQ MNSLRAEDTAVYYCSRWGGDGFYAM DYWGQGTLVTVSSASPREPQVCTLPPS REEMTKNQVSLSCAVKGFYPSDIAVEW ESNGQPENNYKTTPPVLDSDGSFFLVS KLTVDKSRWQQGNVFSCSVMHEALH NHYTQKSLSLSPGKASTKGPSVFPLAP CSRSTSESTAALGCLVKDYFPEPVTVSW NSGALTSGVHTFPAVLQSSGLYSLSSVV TVPSSSLGTKTYTCNVDHKPSNTKVDK RVDKTHTCPPCPAPELLGGPSVFLFPPK PKDTLMISRTPEVTCVVVDVSHEDPEV KFNWYVDGVEVHNAKTKPREEQYNST YRVVSVLTVLHQDWLNGKEYKCKVSN KALPAPIEKTISKAKGQPREPQVYTLPPS REEMTKNQVSLSCAVKGFYPSDIAVEW ESNGQPENNYKTTPPVLDSDGSFFLVS KLTVDKSRWQQGNVFSCSVMHEALH NHYTQKSLSLSPGK | DIQMTQSPSSLSASVGDRVT ITCRASQDVNTAVAWYQQK PGKAPKLLIYSASFLYSGVPSR FSGSRSGTDFTLTISSLQPEDF ATYYCQQHYTTPPTFGQGTK VEIKRTPREPQVYTLPPCREE MTKNQVSLWCLVKGFYPSDI AVEWESNGQPENNYKTTPP VLDSDGSFFLYSKLTVDKSR WQQGNVFSCSVMHEALHN HYTQKSLSLSPGKRTVAAPSV FIFPPSDEQLKSGTASVVCLL NNFYPREAKVQWKVDNAL QSGNSQESVTEQDSKDSTY SLSSTLTLSKADYEKHKVYAC EVTHQGLSSPVTKSFNRGEC |

**[Table 22]**

| | | | | |
|---|---|---|---|---|
| [S] | EVQLVESGGGLVQPGGSLRL SCAASGYTFTNYGMNWVR QAPGKGLEWVGWINTYTGE PTYAADFKRRFTFSLDTSKST AYLQMNSLRAEDTAVYYCAK YPHYYGSSHWYFDVWGQG TLVTVSSASTKGPSVFPLAPS SKSTSGGTAALGCLVKDYFPE PVTVSWNSGALTSGVHTFPA VLQSSGLYSLSSVVTVPSSSL GTQTYICNVNHKPSNTKVDK KVEPKSCDKTHTCPPCPAPEL LGGPSVFLFPPKPKDTLMISR TPEVTCVVVDVSHEDPEVKF NWYVDGVEVHNAKTKPREE QYNSTYRVVSVLTVLHQDW LNGKEYKCKVSNKALPAPIEK TISKAKGQPREPQVYTLPPCR EEMTKNQVSLWCLVKGFYPS DIAVEWESNGQPENNYKTT PPVLDSDGSFFLYSKLTVDKS RWQQGNVFSCSVMHEALH NHYTQKSLSLSPGK | DIQMTQSPSSLSASVGDR VTITCSASQDISNYLNWY QQKPGKAPKVLIYFTSSLH SGVPSRFSGSGSGTDFTLT ISSLQPEDFATYYCQQYST VPWTFGQGTKVEIKRTVA APSVFIFPPSDEQLKSGTA SVVCLLNNFYPREAKVQ WKVDNALQSGNSQESVT EQDSKDSTYSLSSTLTLSK ADYEKHKVYACEVTHQGL SSPVTKSFNRGEC | EVQLVESGGGLVQPGGSLRLSCAASGF NIKDTYIHWVRQAPGKGLEWVARIYPT NGYTRYADSVKGRFTISADTSKNTAYLQ MNSLRAEDTAVYYCSRWGGDGFYAM DYWGQGTLVTVSSASTKGPSVFPLAPS SKSTSGGTAALGCLVKDYFPEPVTVSW NSGALTSGVHTFPAVLQSSGLYSLSSVV TVPSSSLGTQTYICNVNHKPSNTKVDK KVEPKSCGGGGSGGGGSGGGGSGGG GSGQPREPQVYTLPPSREEMTKNQVS LSCAVKGFYPSDIAVEWESNGQPENNY KTTPPVLDSDGSFFLVSKLTVDKSRWQ QGNVFSCSVMHEALHNHYTQKSLSLS PGKDKTHTCPPCPAPELLGGPSVFLFPP KPKDTLMISRTPEVTCVVVDVSHEDPE VKFNWYVDGVEVHNAKTKPREEQYN STYRVVSVLTVLHQDWLNGKEYKCKVS NKALPAPIEKTISKAKGQPREPQVCTLP PSREEMTKNQVSLSCAVKGFYPSDIAV EWESNGQPENNYKTTPPVLDSDGSFF LVSKLTVDKSRWQQGNVFSCSVMHEA LHNHYTQKSLSLSPGK** | DIQMTQSPSSLSASVGDRVTIT CRASQDVNTAVAWYQQKPGK APKLLIYSASFLYSGVPSRFSGSR SGTDFTLTISSLQPEDFATYYCQ QHYTTPPTFGQGTKVEIKRTVA APSVFIFPPSDEQLKSGTASVVC LLNNFYPREAKVQWKVDNALQ SGNSQESVTEQDSKDSTYSLSS TLTLSKADYEKHKVYACEVTHQ GLSSPVTKSFNRGECGGGGSG GGGSGGGGSGGGGSGQPREP QVYTLPPSREEMTKNQVSLWC LVKGFYPSDIAVEWESNGQPEN NYKTTPPVLDSDGSFFLYSKLTV DKSRWQQGNVFSCSVMHEAL HNHYTQKSLSLSPGK** |

**[Table 23]**

| | | | | |
|---|---|---|---|---|
| [T] | EVQLVESGGGLVQPGGSLRL SCAASGYTFTNYGMNWVR QAPGKGLEWVGWINTYTGE PTYAADFKRRFTFSLDTSKST AYLQMNSLRAEDTAVYYCAK YPHYYGSSHWYFDVWGQG TLVTVSSASTKGPSVFPLAPS SKSTSGGTAALGCLVKDYFPE PVTVSWNSGALTSGVHTFPA VLQSSGLYSLSSVVTVPSSSL GTQTYICNVNHKPSNTKVDK KVEPKSCDKTHTCPPCPAPEL LGGPSVFLFPPKPKDTLMISR TPEVTCVVVDVSHEDPEVKF NWYVDGVEVHNAKTKPREE QYNSTYRVVSVLTVLHQDW LNGKEYKCKVSNKALPAPIEK TISKAKGQPREPQVYTLPPCR EEMTKNQVSLWCLVKGFYPS DIAVEWESNGQPENNYKTT PPVLDSDGSFFLYSKLTVDKS RWQQGNVFSCSVMHEALH NHYTQKSLSLSPGK | DIQMTQSPSSLSASVGDR VTITCSASQDISNYLNWY QQKPGKAPKVLIYFTSSLH SGVPSRFSGSGSGTDFTLT ISSLQPEDFATYYCQQYST VPWTFGQGTKVEIKRTVA APSVFIFPPSDEQLKSGTA SVVCLLNNFYPREAKVQ WKVDNALQSGNSQESVT EQDSKDSTYSLSSTLTLSK ADYEKHKVYACEVTHQGL SSPVTKSFNRGEC | EVQLVESGGGLVQPGGSLRLSCAASGF NIKDTYIHWVRQAPGKGLEWVARIYPT NGYTRYADSVKGRFTISADTSKNTAYLQ MNSLRAEDTAVYYCSRWGGDGFYAM DYWGQGTLVTVSSASTKGPSVFPLAPC SRSTSESTAALGCLVKDYFPEPVTVSW NSGALTSGVHTFPAVLQSSGLYSLSSVV TVPSSSLGTKTYTCNVDHKPSNTKVDK RVGGGGSGGGGSGGGGSGGGGSGQ PREPQVYTLPPSREEMTKNQVSLSCAV KGFYPSDIAVEWESNGQPENNYKTTPP VLDSDGSFFLVSKLTVDKSRWQQGNV FSCSVMHEALHNHYTQKSLSLSPGKD KTHTCPPCPAPELLGGPSVFLFPPKPKD TLMISRTPEVTCVVVDVSHEDPEVKFN WYVDGVEVHNAKTKPREEQYNSTYRV VSVLTVLHQDWLNGKEYKCKVSNKAL PAPIEKTISKAKGQPREPQVCTLPPSREE MTKNQVSLSCAVKGFYPSDIAVEWES NGQPENNYKTTPPVLDSDGSFFLVSKL TVDKSRWQQGNVFSCSVMHEALHN HYTQKSLSLSPGK | DIQMTQSPSSLSASVGDRVTIT CRASQDVNTAVAWYQQKPGK APKLLIYSASFLYSGVPSRFSGSR SGTDFTLTISSLQPEDFATYYCQ QHYTTPPTFGQGTKVEIKRTVA APSVFIFPPSDEQLKSGTASVVC LLNNFYPREAKVQWKVDNALQ SGNSQESVTEQDSKDSTYSLSS TLTLSKADYEKHKVYACEVTHQ GLSSPVTKSFNRGECGGGGSG GGGSGGGGSGGGGSGQPREP QVYTLPPSREEMTKNQVSLWC LVKGFYPSDIAVEWESNGQPEN NYKTTPPVLDSDGSFFLYSKLTV DKSRWQQGNVFSCSVMHEAL HNHYTQKSLSLSPGK |

**[Table 24]**

| | | | | |
|---|---|---|---|---|
| [U] | EVQLVESGGGLVQPGGSLRL SCAASGYTFTNYGMNWVR QAPGKGLEWVGWINTYTGE PTYAADFKRRFTFSLDTSKST AYLQMNSLRAEDTAVYYCAK YPHYYGSSHWYFDVWGQG TLVTVSSASTKGPSVFPLAPS SKSTSGGTAALGCLVKDYFPE PVTVSWNSGALTSGVHTFPA VLQSSGLYSLSSVVTVPSSSL GTQTYICNVNHKPSNTKVDK KVEPKSCDKTHTCPPCPAPEL LGGPSVFLFPPKPKDTLMISR TPEVTCVVVDVSHEDPEVKF NWYVDGVEVHNAKTKPREE QYNSTYRVVSVLTVLHQDW LNGKEYKCKVSNKALPAPIEK TISKAKGQPREPQVYTLPPCR EEMTKNQVSLWCLVKGFYPS DIAVEWESNGQPENNYKTT PPVLDSDGSFFLYSKLTVDKS RWQQGNVFSCSVMHEALH NHYTQKSLSLSPGK | DIQMTQSPSSLSASVGDR VTITCSASQDISNYLNWY QQKPGKAPKVLIYFTSSLH SGVPSRFSGSGSGTDFTLT ISSLQPEDFATYYCQQYST VPWTFGQGTKVEIKRTVA APSVFIFPPSDEQLKSGTA SVVCLLNNFYPREAKVQ WKVDNALQSGNSQESVT EQDSKDSTYSLSSTLTLSK ADYEKHKVYACEVTHQGL SSPVTKSFNRGEC | EVQLVESGGGLVQPGGSLRLSCAASGF NIKDTYIHWVRQAPGKGLEWVARIYPT NGYTRYADSVKGRFTISADTSKNTAYLQ MNSLRAEDTAVYYCSRWGGDGFYAM DYWGQGTLVTVSSASPREPQVYTLPPS REEMTKNQVSLSCAVKGFYPSDIAVEW ESNGQPENNYKTTPPVLDSDGSFFLVS KLTVDKSRWQQGNVFSCSVMHEALH NHYTQKSLSLSPGKGGGGSGGGGSGG GGSGGGGSASTKGPSVFPLAPSSKSTS GGTAALGCLVKDYFPEPVTVSWNSGAL TSGVHTFPAVLQSSGLYSLSSVVTVPSS SLGTQTYICNVNHKPSNTKVDKKVEPK SCDKTHTCPPCPAPELLGGPSVFLFPPK PKDTLMISRTPEVTCVVVDVSHEDPEV KFNWYVDGVEVHNAKTKPREEQYNST YRVVSVLTVLHQDWLNGKEYKCKVSN KALPAPIEKTISKAKGQPREPQVCTLPPS REEMTKNQVSLSCAVKGFYPSDIAVEW ESNGQPENNYKTTPPVLDSDGSFFLVS KLTVDKSRWQQGNVFSCSVMHEALH NHYTQKSLSLSPGK | DIQMTQSPSSLSASVGDRVTIT CRASQDVNTAVAWYQQKPGK APKLLIYSASFLYSGVPSRFSGSR SGTDFTLTISSLQPEDFATYYCQ QHYTTPPTFGQGTKVEIKRTPRE PQVYTLPPSREEMTKNQVSLW CLVKGFYPSDIAVEWESNGQPE NNYKTTPPVLDSDGSFFLYSKLT VDKSRWQQGNVFSCSVMHEA LHNHYTQKSLSLSPGKGGGGS GGGGSGGGGSGGGGSRTVAA PSVFIFPPSDEQLKSGTASVVCLL NNFYPREAKVQWKVDNALQS GNSQESVTEQDSKDSTYSLSST LTLSKADYEKHKVYACEVTHQG LSSPVTKSFNRGEC |

**[Table 25]**

| | | | | |
|---|---|---|---|---|
| [V] | EVQLVESGGGLVQPGGSLR LSCAASGYTFTNYGMNWV RQAPGKGLEWVGWINTYT GEPTYAADFKRRFTFSLDTS KSTAYLQMNSLRAEDTAVY YCAKYPHYYGSSHWYFDV WGQGTLVTVSSASTKGPSV FPLAPSSKSTSGGTAALGCL VKDYFPEPVTVSWNSGALT SGVHTFPAVLQSSGLYSLSS VVTVPSSSLGTQTYICNVN HKPSNTKVDKKVEPKSCDK THTCPPCPAPELLGGPSVFL FPPKPKDTLMISRTPEVTCV VVDVSHEDPEVKFNWYVD GVEVHNAKTKPREEQYNST YRVVSVLTVLHQDWLNGK EYKCKVSNKALPAPIEKTISK AKGQPREPQVYTLPPCREE MTKNQVSLWCLVKGFYPS DIAVEWESNGQPENNYKT TPPVLDSDGSFFLYSKLTVD KSRWQQGNVFSCSVMHE ALHNHYTQKSLSLSPGK | DIQMTQSPSSLSASVGDR VTITCSASQDISNYLNWY QQKPGKAPKVLIYFTSSLH SGVPSRFSGSGSGTDFTLT ISSLQPEDFATYYCQQYST VPWTFGQGTKVEIKRTVA APSVFIFPPSDEQLKSGTA SVVCLLNNFYPREAKVQ WKVDNALQSGNSQESVT EQDSKDSTYSLSSTLTLSK ADYEKHKVYACEVTHQGL SSPVTKSFNRGEC | EVQLVESGGGLVQPGGSLRLSCAASGF NIKDTYIHWVRQAPGKGLEWVARIYPT NGYTRYADSVKGRFTISADTSKNTAYLQ MNSLRAEDTAVYYCSRWGGDGFYAM DYWGQGTLVTVSSASPREPQVYTLPPS REEMTKNQVSLSCAVKGFYPSDIAVEW ESNGQPENNYKTTPPVLDSDGSFFLVS KLTVDKSRWQQGNVFSCSVMHEALH NHYTQKSLSLSPGKGGGGSGGGGSGG GGSGGGGSASTKGPSVFPLAPCSRSTS ESTAALGCLVKDYFPEPVTVSWNSGAL TSGVHTFPAVLQSSGLYSLSSVVTVPSS SLGTKTYTCNVDHKPSNTKVDKRVDKT HTCPPCPAPELLGGPSVFLFPPKPKDTL MISRTPEVTCVVVDVSHEDPEVKFNW YVDGVEVHNAKTKPREEQYNSTYRVV SVLTVLHQDWLNGKEYKCKVSNKALP APIEKTISKAKGQPREPQVCTLPPSREE MTKNQVSLSCAVKGFYPSDIAVEWES NGQPENNYKTTPPVLDSDGSFFLVSKL TVDKSRWQQGNVFSCSVMHEALHN HYTQKSLSLSPGK | DIQMTQSPSSLSASVGDRVTIT CRASQDVNTAVAWYQQKPGK APKLLIYSASFLYSGVPSRFSGSR SGTDFTLTISSLQPEDFATYYCQ QHYTTPPTFGQGTKVEIKRTPRE PQVYTLPPSREEMTKNQVSLW CLVKGFYPSDIAVEWESNGQPE NNYKTTPPVLDSDGSFFLYSKLT VDKSRWQQGNVFSCSVMHEA LHNHYTQKSLSLSPGKGGGGS GGGGSGGGGSGGGGSRTVAA PSVFIFPPSDEQLKSGTASVVCLL NNFYPREAKVQWKVDNALQS GNSQESVTEQDSKDSTYSLSST LTLSKADYEKHKVYACEVTHQG LSSPVTKSFNRGEC |

In another aspect, the present invention is directed to a bispecific antibody including a first arm binding to a first antigen and including VH1-CHa-Fc1 and VL1-CLb, and a second arm binding to a second antigen and including VH2-CH1-Fc2 and VL2-CL,
wherein the VH1 and the VH2 are each heavy-chain variable regions including the same or different antigen-binding regions,
the VL1 and VL2 are each light-chain variable regions including the same or different antigen-binding regions,
the CHa includes an IgG heavy-chain constant region CH3 and an IgG heavy-chain constant region CH1,
the CLb includes a CL1 including an IgG light-chain constant region λ or κ, and an IgG heavy-chain constant region CH3,
the CH1 is an IgG heavy-chain constant region CH1 and the CL is an IgG light-chain constant region CL, and
the Fc1 of the first arm is linked to the Fc2 of the second arm to form a heavy-chain constant region dimer.

The IgG heavy-chain constant region CH3 may include heavy-chain constant region CH3 derived from IgG1, IgG2, IgG3 or Ig4.

The IgG heavy-chain constant region CH1 may include heavy-chain constant region CH1 derived from IgG1, IgG2, IgG3 or IgG4. Specifically, the IgG heavy-chain constant region CH1 may include heavy-chain constant region CH1 derived from IgG1 or IgG4.

The CHa may include an IgG heavy-chain constant region CH1 and an IgG heavy-chain constant region CH3 in order from the N-terminus to the C-terminus of the first arm. The CHa may include an IgG heavy-chain constant region CH3 and an IgG heavy-chain constant region CH1 in the order from the N-terminus to the C-terminus of the first arm.

The CLb may include CL1 including an IgG light-chain constant region λ or κ, and heavy-chain constant region CH3 derived from IgG in order from the N-terminus to the C-terminus of the first arm. For example, CLb may include heavy-chain constant region CH3 derived from IgG and CL1 including light-chain constant region λ or κ derived from IgG in the order from N-terminus to C-terminus in the first arm.

In one embodiment, the CH1 of CHa and CL1 of CLb may be linked via a disulfide bond or without a disulfide bond. Specifically, CH1 of CHa and CL1 of CLb may be linked without a disulfide bond.

The CHa and CLb each include CH3 and the CH3 of the CHa and the CH3 of the CLb are linked to form a dimer. The CHa and CLb each include CH3 and CH3 may form a dimer through a disulfide bond.

Specifically, the dimer formed through CH3 included in each of CHa and CLb of the first arm, and the CH3 dimer in the Fab region including CH1 and CL1 included in each of CHa and CLb are linked through a disulfide bond, and CH1 and CL1 are linked without a disulfide bond.

One of the dimers includes at least one selected from the group consisting of T366W, S354C, and Y349C, and the other includes at least one selected from the group consisting of S354C, Y349C, T366S, L368A, and Y407V, to form a knob-in-hole structure. Specifically, one of the dimers formed by bonding CH3 of CHa to CH3 of CLb includes S354C, T366S, L368A, and Y407V, and the other includes Y349C and T366W, to form a knob-in-hole structure.

Specifically, the CH1 of CHa and the CL1 of CLb are linked without a disulfide bond, i) any one of CH3 of CHa and CH3 of CLb includes at least one selected from the group consisting of Y349C, T366S, L368A and Y407V, and the other includes S354C and/or T366W, or ii) any one of CH3 of CHa and CH3 of CLb includes at least one selected from the group consisting of S354C, T366S, L368A and Y407V, and the other includes Y349C and/or T366W.

The CH1 of CHa and CL1 of CLb are linked without a disulfide bond, i) any one of CH3 of CHa and CH3 of CLb includes Y349C, T366S, L368A and Y407V, and the other includes S354C and T366W, or ii) any one of CH3 of CHa and CH3 of CLb includes S354C, T366S, L368A, and Y407V, and the other includes Y349C and T366W.

CH3 of CHa or CH3 of CLb may include the sequence of SEQ ID NOs: 8 to 13. CH3 of CHa or CH3 of CLb may include a mutation of knob (T366W) (SEQ ID NO: 8) or hole (T366S/L368A/Y407V) (SEQ ID NO: 9) of the IgG1 CH3 domain. The IgG1 CH3 domain may include a mutation, namely, a knob (S354C/T366W) (SEQ ID NO: 10), a hole (Y349C/T366S/L368A/Y407V) (SEQ ID NO: 11), knob (Y349C/T366W) (SEQ ID NO: 12), or hole (S354C/T366S/L368A/Y407V) (SEQ ID NO: 13) to generate a cysteine for a disulfide bond.

In some cases, CH3 and CH1 of CHa, and CH3 and CL1 of CLb may be linked through a linker. The linker may be a peptide linker and may include about 5-25 aa residues, or specifically about 5-10 aa residues. For example, the linker may include hydrophilic amino acids such as glycine and/or serine, but are not limited thereto.

Specifically, the linker, for example, includes a glycine linker (G, Gly)p (p is 1 to 10), or a GS linker (GₙS)ₘ (n and m are each 1 to 10) in order to impart structural flexibility. Specifically, the linker may include GGGGS or (GGGGS)2, or (G, Gly)p 5-10 aa glycine wherein p is 5 to 10.

Among dimers formed by Fc₁ of the first arm and Fc₂ of the second arm, the CH3 dimer may include monomers that may be linked with or without a disulfide bond. In one embodiment, one of the CH3 dimers of the Fc includes at least one selected from the group consisting of Y349C, S354C, T366S, T366W, L368A and Y407V, and includes at least one selected from the group consisting of S354C, Y349C, T366W, T366S, L368A and Y407V, to form a knob-in-hole structure among dimers.

The first arm and the second arm may be linked through a hinge. The first arm and the second arm may be linked through a hinge including one or more sequences selected from the group consisting of:
DKTHTCPPCP;
EPKSSDKTHTCPPCP; and
ESKYGPPCPPCP.

The configuration of the bispecific antibody format that simultaneously binds to the first antigen and the second antigen is as follows. The bispecific antibody format is formed by combination of a total of four polypeptides, two heavy (H) chains and two light chains. The configuration of the heavy chain and the light chain of the first arm that binds to the first antigen is as follows. The heavy chain includes VH-CH3a-CH1a-Hinge-CH2-CH3b or VH-CH1a-CH3a-Hinge-CH2-CH3b. Here, CH3a may include a sequence of an IgG1 CH3 domain, and CH1a may include a sequence of an IgG1 CH1 domain (SEQ ID NO: 5) or an IgG4 CH1 domain (SEQ ID NOS: 18 and 19) or an IgD CH1 domain (SEQ ID NO: 20). CH3a or CH3b may include a mutation of a knob (T366W) (SEQ ID NO: 8) or a hole (T366S/L368A/Y407V) (SEQ ID NO: 9) of the IgG1 CH3 domain. The IgG1 CH3 domain may include a mutation, namely a knob (S354C/T366W) (SEQ ID NO: 10), hole (Y349C/T366S/L368A/Y407V) (SEQ ID NO: 11), knob (Y349C/T366W) (SEQ ID NO: 12), or hole (S354C/T366S/L368A /Y407V) (SEQ ID NO: 13) to generate a cysteine for a disulfide bond. The light chain of the first arm that binds to the first antigen is composed of VL-CH3c-CLb or VL-CLb-CH3c. Here, CH3c represents the IgG1 CH3 domain and includes a mutation of a knob (T366W) (SEQ ID NO: 8) or a hole (T366S/L368A/Y407V) (SEQ ID NO: 9). The IgG1 CH3 domain may include a mutation, namely a knob (S354C/T366W) (SEQ ID NO: 10) or a hole (Y349C/T366S/L368A/Y407V) (SEQ ID NO: 11) or a knob (Y349C/T366W) (SEQ ID NO: 12) or a hole (S354C/T366S /L368A/Y407V) (SEQ ID NO: 13) to generate a cysteine for a disulfide bond. CLb may be a kappa type (SEQ ID NOs: 14 and 15) or lambda type (SEQ ID NOs: 16 and 17).

The configuration of the heavy chain and the light chain of the second arm that binds to the second antigen is as follows. The configuration includes VH-CH1-Hinge-CH2-CH3d or VH-CH1-Hinge-CH2-CH3d. The IgG1 CH3d domain may include a mutation of a knob (T366W) (SEQ ID NO: 8) or a hole (T366S/L368A/Y407V) (SEQ ID NO: 9). The IgG1 CH3d domain may also include a mutation to generate cysteines for disulfide bonds, namely a knob (S354C/T366W) (SEQ ID NO: 10) or a hole (Y349C/T366S/L368A/Y407V) (SEQ ID NO: 11). The configuration of the light chain may include VL-CLb. CLb may be a kappa type (SEQ ID NO: 14) or a lambda type (SEQ ID NO: 16).

In the Q-SBL1 (SEQ ID NO: 31, 32, 62, 63) bispecific antibody format, the configuration of heavy- and light-chains of the first arm binding to the first antigen is as follows. The configuration of the heavy chain of the first arm includes VH-CH3a-Linker-CH1a-Hinge-CH2-CH3b. Here, the amino acid sequence of the linker was determined as GGGGSGGGGS (SEQ ID NO: 28). The CH1a domain may be an IgG1 CH1 domain (SEQ ID NO: 5). In order to remove the disulfide bond between the CH1a domain and CLb, the hinge region may have an amino acid sequence of DKTHTCPPCP (SEQ ID NO: 22). The CH3a domain includes a hole mutation and a mutation for forming a disulfide bond with CH3c of the light chain, and the mutation of the CH3a domain may be a hole (Y349C/T366S/L368A/Y407V) (SEQ ID NO: 11). The CH3b domain may include a hole mutation (T366S/L368A/Y407V) (SEQ ID NO: 9). An elbow sequence AS may be added between the VH region and the CH3a region (SEQ ID NO: 25).

The configuration of the first arm light chain may include VL-CH3c-Linker-CLb. The CH3c domain includes a knob mutation and a mutation for a disulfide bond with CH3a of the heavy chain, and the mutation of the CH3c domain may be a knob (S354C/T366W) (SEQ ID NO: 10). The amino acid sequence of the linker may be GGGGSGGGGS (SEQ ID NO: 28). The CLb domain may be a Kappa type and may include a C216S (Eu numbering) mutation to remove a disulfide bond with the CH1 domain of the first arm heavy chain (SEQ ID NO: 17). When the CLb domain is a lambda type, it may include a C214S (Eu numbering) mutation in order to remove the disulfide bond with the CH1 domain of the first arm heavy chain. This means that the disulfide bond to form the CH1a/CLb dimer is completely removed. An elbow sequence RT was added between the VL region and the CH3a region (SEQ ID NO: 26).

The configuration of the heavy chain and the light chain of the second arm that binds to the second antigen is as follows. The configuration of the heavy chain of the second arm may include VH-CH1-CH2-CH3d and the CH3d domain may include a knob (T366W) (SEQ ID NO: 8) mutation. The configuration of the light chain of the second arm is VL-CLb. CLb may be a kappa type (SEQ ID NO: 14) or a lambda type (SEQ ID NO: 16).

| **Q-SBL1** | | | | | |
|---|---|---|---|---|---|
| | **1st arm heavy-chain sequence** | **2nd arm heavy-chain sequence** | | **1st arm light-chain sequence** | **2nd arm light-chain sequenc e** |
| **VH** | VH | VH | **VL** | VL | VL |
| **Elbow sequence** | AS | - | **Elbow sequence** | RT | - |
| **CH3a** | IgG1 CH3 (Y349C/T366S/L368A/Y407 V) | - | **CH3c** | IgG1 CH3 (S354C/T366 W) | - |
| **linker** | GGGGSGGGGS | - | **linker** | GGGGSGGGGS | - |
| **CH1** | IgG1 CH1 | IgG1 CH1 | **CLb** | kappa CL | kappa CL |
| **Hinge** | DKTHTCPPCP | EPKSCDKTHTCPP CP | | | |
| **CH2** | IgG1 CH2 | IgG1 CH2 | | | |
| **CH3b** | IgG1 CH3 (T366S/L368A/Y407V) | IgG1 CH3 (T366W) | | | |

The R-SBL1 (SEQ ID NOS: 31, 32, 62, and 63) bispecific antibody format is different from Q-SBL1 in that it has a configuration in which only the CH1a domain portion in the heavy-chain region of the first arm that binds to the first antigen and may include a C131S mutation to remove the disulfide bond with CLb in the light-chain region of the first arm (SEQ ID NO: 19).

| **R-SBL1** | | | | | |
|---|---|---|---|---|---|
| | **1st arm heavy-chain sequence** | **2nd arm heavy-chain sequence** | | **1st arm light-chain sequence** | **2nd arm light-chain sequenc** e |
| **VH** | VH | VH | **VL** | VL | VL |
| **Elbow sequence** | AS | - | **Elbow sequence** | RT | - |
| **CH3** | IgG1 CH3 (Y349C/T366S/L368A/Y407 V) | - | **CH3** | IgG1 CH3 (S354C/T366 W) | - |

| | | | | | |
|---|---|---|---|---|---|
| **linker** | GGGGSGGGGS | - | **linker** | GGGGSGGGGS | - |
| **CH1** | IgG4 CH1 | IgG1 CH1 | **CL** | kappa CL | kappa CL |
| **Hinge** | DKTHTCPPCP | EPKSCDKTHTCPP CP | | | |
| **CH2** | IgG1 CH2 | IgG1 CH2 | | | |
| **CH3** | IgG1 CH3 (T366S/L368A/Y407V) | IgG1 CH3 (T366W) | | | |

In another aspect, the present invention is directed to a multispecific antibody including the bispecific antibody described above.

As used herein, the term "multispecific" refers to an ability of a binding protein to specifically bind three different types of targets to control the activity of the targets. For example, multi specificity can be obtained by conjugation of a monoclonal antibody or fragment thereof that specifically binds to each target, possesses three or more distinct antigen-binding arms and has monovalence for each bound antigen.

For example, one or more antibody fragments that bind to additional antigens may be further included at the N-terminus of the first arm or the second arm or at the Fc₁ terminus of the first arm or the Fc₂ terminus of the second arm. An antigen-binding fragment that binds to an additional antigen may be further included at the N-terminus of the first arm or the second arm, or an antigen-binding fragment that binds to an additional antigen may be further included at the Fc₁ or Fc₂ terminus of the first arm. As a result, it is possible to construct multispecific antibodies targeting three or more antigens.

The antibody fragment includes a portion of an intact antibody, or an antigen-binding or variable region of an intact antibody. For example, the antibody fragment may include a Fab, Fab', F(ab')2, Fv, scFv, or diabody.

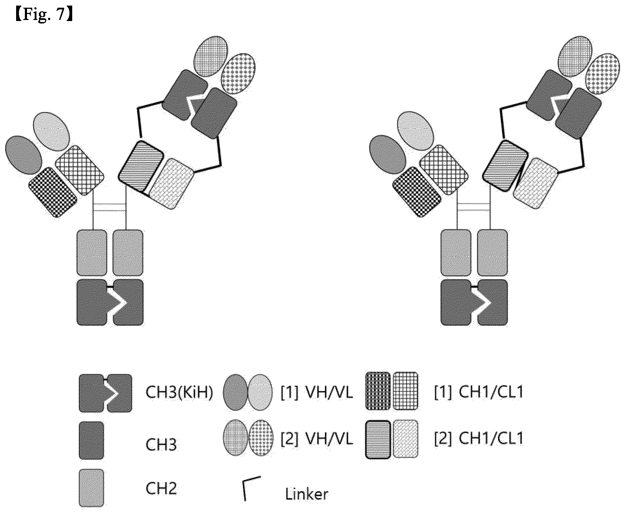

In one embodiment, an antibody fragment including VH3-CHa and VL3-CLb binding to a third antigen may be included at the N-terminus of the first arm. In some cases, an antibody fragment in the form of scFv that binds to a third antigen may be included at the Fc terminus (FIG. 7).

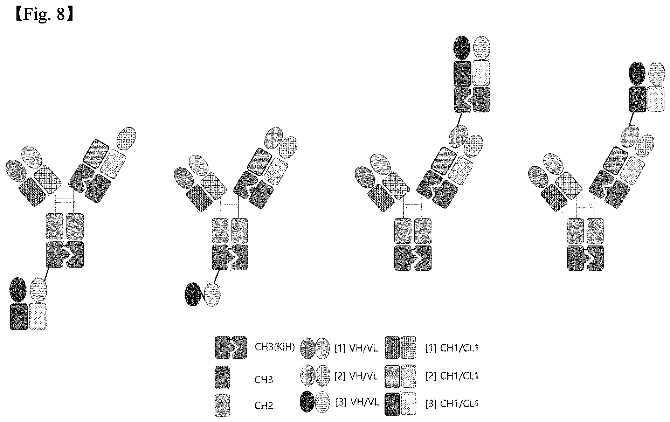

In another embodiment, an antibody fragment including VH3-CHa and VL3-CLb that bind to a third antigen is included at the N-terminus of the first arm and an scFv form antibody fragment that binds to a fourth antigen may be included at the Fc terminus. In some cases, an antibody fragment in the form of an scFv that binds to a third antigen and a fourth antigen may be included at the Fc terminus (FIG. 8).

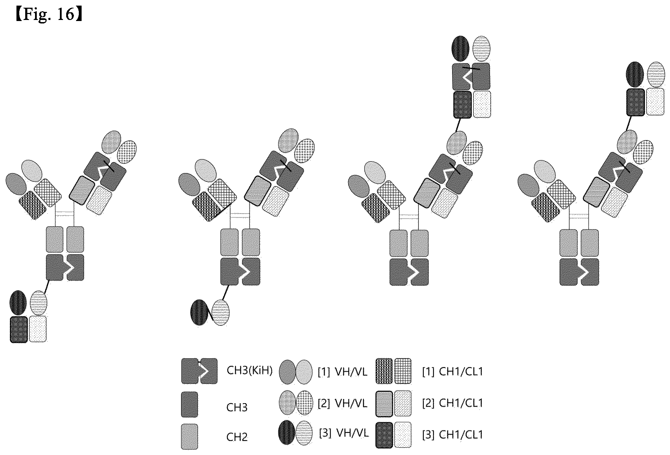

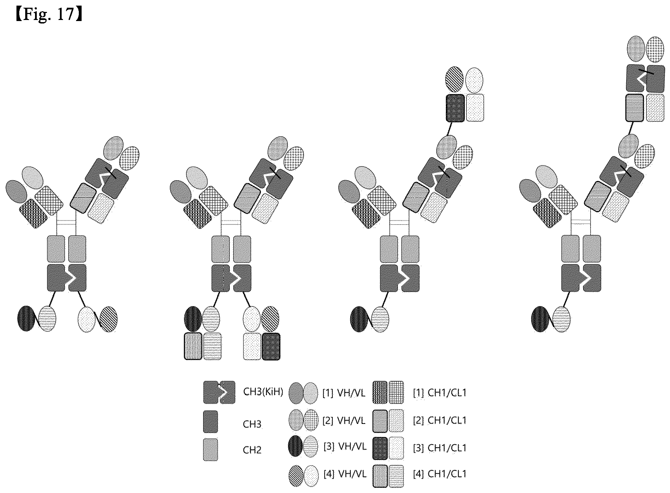

The bispecific target form may be expanded to a tri-valent multispecific antibody and a tetra-valent multispecific antibody. In the form of a representative bispecific target antibody, the tri-valent multispecific antibody may be constructed by linking the scFv or Fab form targeting a third antigen to the N-terminus or C-terminus of the heavy-chain region of the first arm or the heavy-chain region of the second arm through a linker (FIG. 16). In addition, the tetra-valent multispecific antibody may be constructed by further linking a scFv or Fab form targeting a fourth antigen thereto through a linker (FIG. 17).

As used herein, the term "mutation (variation)" may mean a mutation (variation), for example, substitution, addition and/or deletion, of an amino acid sequence constituting a heavy-chain variable region and/or light-chain variable region and may include any mutation that does not impair antigen binding and efficacy, without limitation. Introduction of mutations into the binding protein according to the present invention may be applied, for example, to an external variable region or an internal variable region, or both an external variable region and an internal variable region.

When taking into consideration mutations having biologically equivalent activity, the polypeptides, binding proteins or nucleic acid molecule encoding the same according to the present invention is interpreted to include a sequence having substantial identity with the sequence set forth in the sequence number. The term "substantial identity" means that a sequence has a homology of at least 61%, preferably a homology of at least 70%, more preferably at least 80%, and most preferably at least 90%, 91%,92%, 93%, 94%, 95%, 96%, 97%, 98%, and 99% when aligning the sequence of the present invention and any other sequence so as to correspond to each other as much as possible and analyzing the aligned sequence using algorithms commonly used in the art. Alignment methods for sequence comparison are well-known in the art. The NCBI Basic Local Alignment Search Tool (BLAST) is accessible through NCBI or the like, and can be used in conjunction with sequence analysis programs such as BLASTP, BLASTM, BLASTX, TBLASTN and TBLASTX over the Internet. BLAST is available at www.ncbi.nlm.nih.gov/BLAST/. A method of comparing sequence homology using this program can be found at www.ncbi.nlm.nih.gov/BLAST/blast_help.html.

Based on this, the sequence according to the present invention may have a homology of 60%, 70%, 75%, 80%, 85%, 90%, 91%, 92%, 93%, 94%, 95%, 96%, 97%, 98%, 99%, or more compared to the sequence disclosed herein or the entirety thereof. Homology can be determined through sequence comparison and/or alignment by methods known in the art. For example, the percentage sequence homology of the nucleic acid or protein according to the present invention can be determined using a sequence comparison algorithm (i.e., BLAST or BLAST 2.0), manual alignment, or visual inspection.

In another aspect, the present invention is directed to a nucleic acid encoding the bispecific antibody.

The first arm and/or the second arm of the bispecific antibody may be produced in a recombinant manner. The nucleic acid is isolated and inserted into a replicable vector, followed by further cloning (amplification of DNA) or further expression. Based on this, in another aspect, the present invention is directed to a vector including the nucleic acid.

The term "nucleic acid" is intended to encompass both DNA (gDNA and cDNA) and RNA molecules, and a nucleotide, which is the basic constituent unit of nucleic acids, includes naturally derived nucleotides as well as analogues thereof, in which sugar or base moieties are modified. The sequence of the nucleic acid encoding heavy- and light-chain variable regions of the present invention can vary. Such variation includes addition, deletion, or non-conservative or conservative substitution of nucleotides.

DNA encoding the first arm and/or second arm of the binding protein may be easily separated or synthesized using conventional procedures (for example, using an oligonucleotide probe capable of specifically binding to DNA). A variety of vectors are obtainable. Vector components generally include, but are not limited to, one or more of the following components: signal sequences, replication origins, one or more marker genes, enhancer elements, promoters, and transcription termination sequences.

As used herein, the term "vector" refers to a means for expressing target genes in host cells, and includes plasmid vectors, cosmid vectors, and viral vectors such as bacteriophage vectors, adenovirus vectors, retroviral vectors, and adeno-associated viral vectors. The nucleic acid encoding the antibody in the vector is operably linked to a promoter.

The term "operably linked" means functional linkage between a nucleic acid expression regulation sequence (e.g., an array of the binding site of the promoter, signal sequence, or transcription regulator) and another nucleic acid sequence, and enables the regulation sequence to regulate transcription and/or translation of the other nucleic acid sequence.

When a prokaryotic cell is used as a host, it generally includes a potent promoter capable of conducting transcription (such as a tac promoter, lac promoter, lacUV5 promoter, lpp promoter, pLλ promoter, pRλ promoter, rac5 promoter, amp promoter, recA promoter, SP6 promoter, trp promoter, or T7 promoter), a ribosome-binding site for initiation of translation, and a transcription/translation termination sequence. In addition, for example, when a eukaryotic cell is used as a host, it includes a promoter derived from the genome of a mammalian cell (e.g., a metallothionein promoter, a β-actin promoter, a human hemoglobin promoter or a human muscle creatine promoter), or a promoter derived from a mammalian virus (e.g., an adenovirus late promoter, vaccinia virus 7.5K promoter, SV40 promoter, cytomegalovirus (CMV) promoter, HSV tk promoter, mouse mammary tumor virus (MMTV) promoter, HIV LTR promoter, Moloney virus promoter, Epstein-Barr virus (EBV) promoter, or Rous sarcoma virus (RSV) promoter), and generally has a polyadenylation sequence as a transcription termination sequence.

Optionally, the vector may be fused with another sequence in order to facilitate purification of the antibody expressed thereby. The sequence to be fused therewith may include, for example, glutathione S-transferase (Pharmacia, USA), maltose-binding protein (NEB, USA), FLAG (IBI, USA), 6x His (hexahistidine; Qiagen, USA) and the like.

The vector includes antibiotic resistance genes commonly used in the art as selectable markers, and examples thereof include genes conferring resistance to ampicillin, gentamycin, carbenicillin, chloramphenicol, streptomycin, kanamycin, geneticin, neomycin, and tetracycline.

In another aspect, the present invention is directed to a cell transformed with the above-mentioned vector. The cell used to produce the bispecific antibody of the present invention may be a prokaryote, yeast, or higher eukaryotic cell, but is not limited thereto.

Prokaryotic host cells such as *Escherichia coli,* strains of the genus *Bacillus,* such as *Bacillus subtilis* and *Bacillus thuringiensis, Streptomyces* spp., *Pseudomonas* spp. (for example, *Pseudomonas putida), Proteus mirabilis* and *Staphylococcus* spp. (for example, *Staphylococcus carnosus*) may be used.

Interest in animal cells is the greatest, and examples of useful host cell lines include, but are not limited to, COS-7, BHK, CHO, CHOK1, DXB-11, DG-44, CHO/-DHFR, CV1, COS-7, HEK293, BHK, TM4, VERO, HELA, MDCK, BRL 3A, W138, Hep G2, SK-Hep, MMT, TRI, MRC 5, FS4, 3T3, RIN, A549, PC12, K562, PER.C6, SP2/0, NS-0, U20S, and HT1080.

In another aspect, the present invention is directed to a method of producing a bispecific antibody including (a) culturing the cells, and (b) recovering a bispecific antibody from the cultured cells.

The cells may be cultured in various media. Any commercially available medium can be used as a culture medium without limitation. All other essential supplements well-known to those skilled in the art may be included in appropriate concentrations. Culture conditions such as temperature and pH are those that are conventionally used with the host cells selected for expression, which will be apparent to those skilled in the art.

The recovery of the bispecific antibody may be carried out, for example, by centrifugation or ultrafiltration to remove impurities and further purification of the resulting product using, for example, affinity chromatography. Other additional purification techniques such as anion or cation exchange chromatography, hydrophobic interaction chromatography and hydroxyapatite (HA) chromatography may be used.

Hereinafter, the present invention will be described in more detail with reference to examples. However, it will be obvious to those skilled in the art that these examples are provided only for illustration of the present invention and should not be construed as limiting the scope of the present invention. Accordingly, the substantial scope of the present invention will be defined by the appended claims and equivalents thereto.

### Example 1. Anti-VEGF x anti-HER2 bispecific antibody design

The first antigen was determined as a HER2 protein, and the heavy-chain variable region amino acid sequence (SEQ ID NO: 3) and the light-chain variable region amino acid sequence (SEQ ID NO: 4) of trastuzumab that bind to HER2 were used for the VH or VL in a bispecific antibody format. The second antigen was determined as a VEGF-A target and the heavy-chain variable region amino acid sequence (SEQ ID NO: 1) and the light-chain variable region amino acid sequence (SEQ ID NO: 2) of bevacizumab were used for the VH or VL in the bispecific antibody format. Amino acid sequence information was obtained through https://go.drugbank.com/.

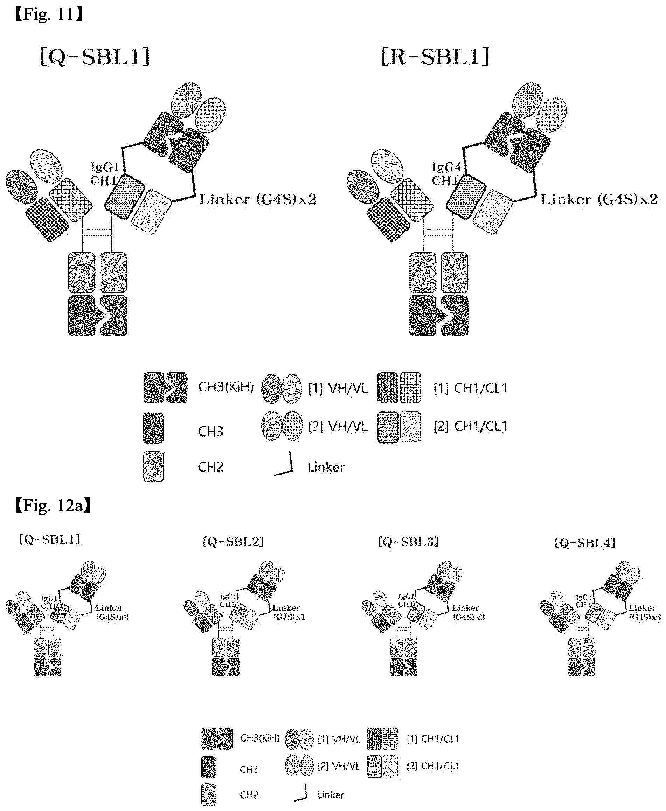

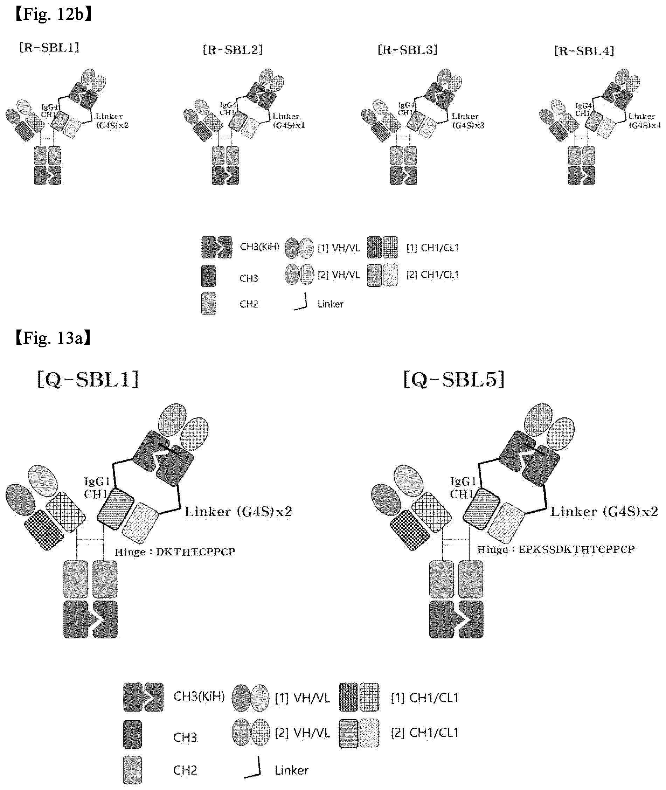

Various candidates were established to select the optimal bispecific antibody formats. A total of three engineering attempts were made using Q-SBL1 and R-SBL1 as basic format antibodies and diversity was given to the length of the linker between the CH3 dimer and CH1/CL in the Fab region of the first arm (FIGS. 12A, and 12B). i) GGGGS (SEQ ID NO: 27), ii) GGGGSGGGGS (SEQ ID NO: 28), iii) GGGGSGGGGSGGGGS (SEQ ID NO: 29), and iv) GGGGSGGGGSGGGGSGGGGS (SEQ ID NO: 30) were applied to the linker of Q-SBL1 (SEQ ID NOS: 31,32,62,63) and R-SBL1 (SEQ ID NOS: 49, 50, 62, and 63). The candidates, to which the linker of GGGGS was applied, were Q-SBL2 (SEQ ID NOS: 33, 34, 62, and 63) and R-SBL2 (SEQ ID NOS: 51, 52, 62, and 63), the candidates to which the linker of GGGGSGGGGS was applied were Q-SBL1 (SEQ ID NOS: 33, 34, 62, and 63), and R-SBL1 (SEQ ID NOS: 49, 50, 62, and 63), and the candidates, to which the linker of GGGGSGGGGSGGGGS was applied, were Q-SBL3 (SEQ ID NOS: 35,36,62, and 63), and R-SBL3 (SEQ ID NOS: 53,54,62, and 63), and the candidates, to which the linker of GGGGSGGGGSGGGGSGGGGS was applied, were Q-SBL4 (SEQ ID NOS: 37, 38, 62, and 63) and R-SBL4 (SEQ ID NOS: 55, 56, 62, and 63).

| **Q-SBL1** | | | | | |
|---|---|---|---|---|---|
| | **1st arm heavy-chain sequence** | **2nd arm heavy-chain sequence** | | **1st arm light-chain sequence** | **2nd arm light-chain sequen ce** |
| **VH** | VH | VH | **VL** | VL | VL |
| **Elbow sequen ce** | AS | - | **Elbow sequen ce** | RT | - |
| **CH3** | IgG1 CH3 (Y349C/T366S/L368A/Y 407V) | - | **CH3** | IgG1 CH3 (S354C/T366W) | - |
| **linker** | GGGGSGGGGS | - | **linker** | GGGGSGGGGS | - |
| **CH1** | IgG1 CH1 | IgG1 CH1 | **CL** | kappa CL | kappa CL |
| **Hinge** | DKTHTCPPCP | EPKSCDKTHTCP PCP | | | |
| **CH2** | IgG1 CH2 | IgG1 CH2 | | | |
| **CH3** | IgG1 CH3 (T366S/L368A/Y407V) | IgG1 CH3 (T366W) | | | |

| Q-SBL2 | | | | | |
|---|---|---|---|---|---|
| | **1st arm heavy-chain sequence** | **2nd arm heavy-chain sequence** | | **1st arm light-chain sequence** | **2nd arm light-chain sequen ce** |
| **VH** | VH | VH | **VL** | VL | VL |
| **Elbow sequen ce** | AS | - | **Elbow sequen ce** | RT | - |
| **CH3** | IgG1 CH3 (Y349C/T366S/L368A/Y 407V) | - | **CH3** | IgG1 CH3 (S354C/T366W) | - |
| **linker** | GGGGS | - | **linker** | GGGGS | - |
| **CH1** | IgG1 CH1 | IgG1 CH1 | **CL** | kappa CL | kappa CL |
| **Hinge** | DKTHTCPPCP | EPKSCDKTHTCP PCP | | | |
| **CH2** | IgG1 CH2 | IgG1 CH2 | | | |
| **CH3** | IgG1 CH3 (T366S/L368A/Y407V) | IgG1 CH3 (T366W) | | | |

| **Q-SBL3** | | | | | |
|---|---|---|---|---|---|
| | **1st arm heavy-chain sequence** | **2nd arm heavy-chain sequence** | | **1st arm light-chain sequence** | **2nd arm light-chain sequen ce** |
| **VH** | VH | VH | **VL** | VL | VL |
| **Elbow sequence** | AS | - | **Elbow sequen ce** | RT | - |
| **CH3** | IgG1 CH3 (Y349C/T366S/L368A/Y 407V) | - | **CH3** | IgG1 CH3 (S354C/T366W) | - |
| **linker** | GGGGSGGGGSGGGGS | - | **linker** | GGGGSGGGGSGGGGS | - |
| **CH1** | IgG1 CH1 | IgG1 CH1 | **CL** | kappa CL | kappa CL |
| **Hinge** | DKTHTCPPCP | EPKSCDKTHTCP PCP | | | |
| **CH2** | IgG1 CH2 | IgG1 CH2 | | | |
| **CH3** | IgG1 CH3 (T366S/L368A/Y407V) | IgG1 CH3 (T366W) | | | |

| **Q-SBL4** | | | | | |
|---|---|---|---|---|---|
| | **1st arm heavy-chain sequence** | **2nd arm heavy-chain sequence** | | **1st arm light-chain sequence** | **2nd arm light-chain sequen ce** |
| **VH** | VH | VH | **VL** | VL | VL |
| **Elbow sequen ce** | AS | - | **Elbow sequen ce** | RT | - |
| **CH3** | IgG1 CH3 (Y349C/T366S/L368A/Y 407V) | - | **CH3** | IgG1 CH3 (S354C/T366W) | - |
| **linker** | GGGGSGGGGSGGGGSG GGGS | - | **linker** | GGGGSGGGGSGGGGSG GGGS | - |
| **CH1** | IgG1 CH1 | IgG1 CH1 | **CL** | kappa CL | kappa CL |
| **Hinge** | DKTHTCPPCP | EPKSCDKTHTCP PCP | | | |
| **CH2** | IgG1 CH2 | IgG1 CH2 | | | |
| **CH3** | IgG1 CH3 (T366S/L368A/Y407V) | IgG1 CH3 (T366W) | | | |

| **R-SBL1** | | | | | |
|---|---|---|---|---|---|
| | **1st arm heavy-chain sequence** | **2nd arm heavy-chain sequence** | | **1st arm light-chain sequence** | **2nd arm light-chain sequen ce** |
| **VH** | VH | VH | **VL** | VL | VL |
| **Elbow sequen ce** | AS | - | **Elbow sequen ce** | RT | - |
| **CH3** | IgG1 CH3 (Y349C/T366S/L368A/Y 407V) | - | **CH3** | IgG1 CH3 (S354C/T366W) | - |
| **linker** | GGGGSGGGGS | - | **linker** | GGGGSGGGGS | - |
| **CH1** | IgG4 CH1 | IgG1 CH1 | **CL** | kappa CL | kappa CL |
| **Hinge** | DKTHTCPPCP | EPKSCDKTHTCP PCP | | | |
| **CH2** | IgG1 CH2 | IgG1 CH2 | | | |
| **CH3** | IgG1 CH3 (T366S/L368A/Y407V) | IgG1 CH3 (T366W) | | | |

| **R-SBL2** | | | | | |
|---|---|---|---|---|---|
| | **1st arm heavy-chain sequence** | **2nd arm heavy-chain sequence** | | **1st arm light-chain sequence** | **2nd arm light-chain sequen ce** |
| **VH** | VH | VH | **VL** | VL | VL |
| **Elbow sequen ce** | AS | - | **Elbow sequen ce** | RT | - |
| **CH3** | IgG1 CH3 (Y349C/T366S/L368A/Y 407V) | - | **CH3** | IgG1 CH3 (S354C/T366W) | - |
| **linker** | GGGGS | - | **linker** | GGGGS | - |
| **CH1** | IgG4 CH1 | IgG1 CH1 | **CL** | kappa CL | kappa CL |
| **Hinge** | DKTHTCPPCP | EPKSCDKTHTCP PCP | | | |
| **CH2** | IgG1 CH2 | IgG1 CH2 | | | |
| **CH3** | IgG1 CH3 (T366S/L368A/Y407V) | IgG1 CH3 (T366W) | | | |

| **R-SBL3** | | | | | |
|---|---|---|---|---|---|
| | **1st arm heavy-chain sequence** | **2nd arm heavy-chain sequence** | | **1st arm light-chain sequence** | **2nd arm light-chain sequen ce** |
| **VH** | VH | VH | **VL** | VL | VL |
| **Elbow sequen ce** | AS | - | **Elbow sequen ce** | RT | - |
| **CH3** | IgG1 CH3 (Y349C/T366S/L368A/Y 407V) | - | **CH3** | IgG1 CH3 (S354C/T366W) | - |
| **linker** | GGGGSGGGGSGGGGS | - | **linker** | GGGGSGGGGSGGGGS | - |
| **CH1** | IgG4 CH1 | IgG1 CH1 | **CL** | kappa CL | kappa CL |
| **Hinge** | DKTHTCPPCP | EPKSCDKTHTCP PCP | | | |
| **CH2** | IgG1 CH2 | IgG1 CH2 | | | |
| **CH3** | IgG1 CH3 (T366S/L368A/Y407V) | IgG1 CH3 (T366W) | | | |

| **R-SBL4** | | | | | |
|---|---|---|---|---|---|
| | **1st arm heavy-chain sequence** | **2nd arm heavy-chain sequence** | | **1st arm light-chain sequence** | **2nd arm light-chain sequen ce** |
| **VH** | VH | VH | **VL** | VL | VL |
| **Elbow sequen ce** | AS | - | **Elbow sequen ce** | RT | - |
| **CH3** | IgG1 CH3 (Y349C/T366S/L368A/Y 407V) | - | **CH3** | IgG1 CH3 (S354C/T366W) | - |
| **linker** | GGGGSGGGGSGGGGSG GGGS | - | **linker** | GGGGSGGGGSGGGGSG GGGS | - |
| **CH1** | IgG4 CH1 | IgG1 CH1 | **CL** | kappa CL | kappa CL |
| **Hinge** | DKTHTCPPCP | EPKSCDKTHTCP PCP | | | |
| **CH2** | IgG1 CH2 | IgG1 CH2 | | | |
| **CH3** | IgG1 CH3 (T366S/L368A/Y407V) | IgG1 CH3 (T366W) | | | |

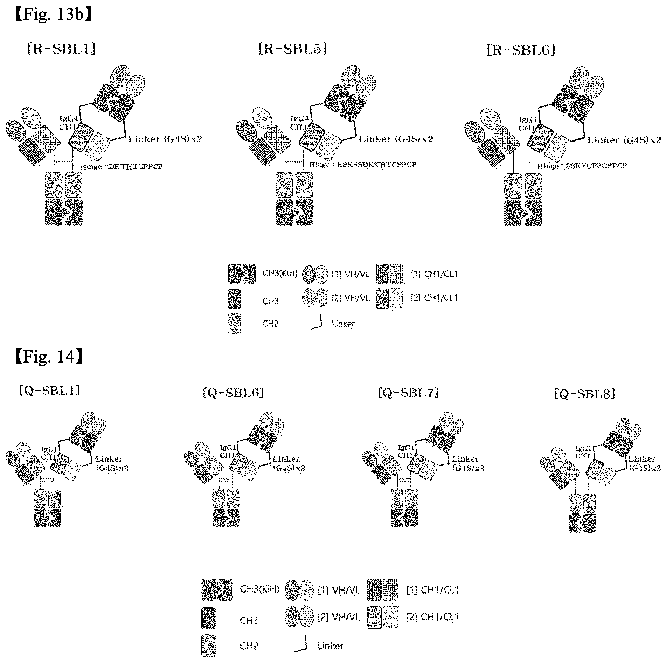

Second, all candidate groups were derived under the same conditions by introducing an IgG1 hinge (EPKSSDKTHTCPPCP) (SEQ ID NO: 23) or an IgG4 hinge (ESKYGPPCPPCP) (SEQ ID NO: 24) into the hinge region amino acid sequence of the first arm in Q-SBL1 and R-SBL1, and the amino acid sequence of the hinge region of the second arm was EPKSCDKTHTCPPCP (SEQ ID NO: 21) (FIGS. 13A and 13B). When an IgG1 hinge (EPKSSDKTHTCPPCP) (SEQ ID NO: 23) was used as the hinge region of the heavy-chain region of the first arm in Q-SBL1, Q-SBL5 (SEQ ID NOS: 39, 40, 62, and 63) was obtained. When the IgG1 hinge (EPKSSDKTHTCPPCP) (SEQ ID NO: 23) was used as the hinge region of the heavy-chain region of the first arm in R-SBL1, R-SBL5 (SEQ ID NOS: 57,58,62,63) was obtained, and when an IgG4 hinge (ESKYGPPCPPCP) (SEQ ID NO: 24) was used as the hinge region of the heavy-chain region of one arm, R-SBL6 (SEQ ID NOS: 59, 60, 62, and 63) was obtained. Here, when the IgG1 hinge of the first arm was used, the C220S (Eu numbering) mutation was present (SEQ ID NO: 23). The mutation was formed in order to remove the disulfide bond with CLb.

| **Q-SBL1** | | | | | |
|---|---|---|---|---|---|
| | **1st arm heavy-chain sequence** | **2nd arm heavy-chain sequence** | | **1st arm light-chain sequence** | **2nd arm light-chain sequenc e** |
| **VH** | VH | VH | **VL** | VL | VL |
| **Elbow sequenc e** | AS | - | **Elbow sequenc e** | RT | - |
| **CH3** | IgG1 CH3 (Y349C/T366S/L368A/Y407 V) | - | **CH3** | IgG1 CH3 (S354C/T366 W) | - |
| **linker** | GGGGSGGGGS | - | **linker** | GGGGSGGGGS | - |
| **CH1** | IgG1 CH1 | IgG1 CH1 | **CL** | kappa CL | kappa CL |
| **Hinge** | DKTHTCPPCP | EPKSCDKTHTCPP CP | | | |
| **CH2** | IgG1 CH2 | IgG1 CH2 | | | |
| **CH3** | IgG1 CH3 (T366S/L368A/Y407V) | IgG1 CH3 (T366W) | | | |

| **Q-SBLS** | | | | | |
|---|---|---|---|---|---|
| | **1st arm heavy-chain sequence** | **2nd arm heavy-chain sequence** | | **1st arm light-chain sequence** | **2nd arm light-chain sequenc e** |
| **VH** | VH | VH | **VL** | VL | VL |
| **Elbow sequenc e** | AS | - | **Elbow sequenc e** | RT | - |
| **CH3** | IgG1 CH3 (Y349C/T366S/L368A/Y407 V) | - | **CH3** | IgG1 CH3 (S354C/T366 W) | - |
| **linker** | GGGGSGGGGS | - | **linker** | GGGGSGGGGS | - |
| **CH1** | IgG1 CH1 | IgG1 CH1 | **CL** | kappa CL | kappa CL |
| **Hinge** | EPKSSDKTHTCPPCP | EPKSCDKTHTCPP CP | | | |
| **CH2** | IgG1 CH2 | IgG1 CH2 | | | |
| **CH3** | IgG1 CH3 (T366S/L368A/Y407V) | IgG1 CH3 (T366W) | | | |

| **R-SBL1** | | | | | |
|---|---|---|---|---|---|
| | **1st arm heavy-chain sequence** | **2nd arm heavy-chain sequence** | | **1st arm light-chain sequence** | **2nd arm light-chain sequenc e** |
| **VH** | VH | VH | **VL** | VL | VL |
| **Elbow sequenc e** | AS | null | **Elbow sequenc** e | RT | null |
| **CH3** | IgG1 CH3 (Y349C/T366S/L368A/Y407 V) | null | **CH3** | IgG1 CH3 (S354C/T366 W) | null |
| **linker** | GGGGSGGGGS | null | **linker** | GGGGSGGGGS | null |
| **CH1** | IgG4 CH1 | IgG1 CH1 | **CL** | kappa CL | kappa CL |
| **Hinge** | DKTHTCPPCP | EPKSCDKTHTCPP CP | | | |
| **CH2** | IgG1 CH2 | IgG1 CH2 | | | |
| **CH3** | IgG1 CH3 (T366S/L368A/Y407V) | IgG1 CH3 (T366W) | | | |

| **R-SBLS** | | | | | |
|---|---|---|---|---|---|
| | **1st arm heavy-chain sequence** | **2nd arm heavy-chain sequence** | | **1st arm light-chain sequence** | **2nd arm light-chain sequenc e** |
| **VH** | VH | VH | **VL** | VL | VL |
| **Elbow sequenc e** | AS | null | **Elbow sequenc e** | RT | null |
| **CH3** | IgG1 CH3 (Y349C/T366S/L368A/Y407 V) | null | **CH3** | IgG1 CH3 (S354C/T366 W) | null |
| **linker** | GGGGSGGGGS | null | **linker** | GGGGSGGGGS | null |
| **CH1** | IgG4 CH1 | IgG1 CH1 | **CL** | kappa CL | kappa CL |
| **Hinge** | EPKSSDKTHTCPPCP | EPKSCDKTHTCPP CP | | | |
| **CH2** | IgG1 CH2 | IgG1 CH2 | | | |
| **CH3** | IgG1 CH3 (T366S/L368A/Y407V) | IgG1 CH3 (T366W) | | | |

| **R-SBL6** | | | | | |
|---|---|---|---|---|---|
| | **1st arm heavy-chain sequence** | **2nd arm heavy-chain sequence** | | **1st arm light-chain sequence** | **2nd arm light-chain sequenc e** |
| **VH** | VH | VH | **VL** | VL | VL |
| **Elbow sequenc e** | AS | null | **Elbow sequenc e** | RT | null |
| **CH3** | IgG1 CH3 (Y349C/T366S/L368A/Y407 V) | null | **CH3** | IgG1 CH3 (S354C/T366 W) | null |
| **linker** | GGGGSGGGGS | null | **linker** | GGGGSGGGGS | null |
| **CH1** | IgG4 CH1 | IgG1 CH1 | **CL** | kappa CL | kappa CL |
| **Hinge** | ESKYGPPCPPCP | EPKSCDKTHTCPP CP | | | |
| **CH2** | IgG1 CH2 | IgG1 CH2 | | | |
| **CH3** | IgG1 CH3 (T366S/L368A/Y407V) | IgG1 CH3 (T366W) | | | |

Third, a candidate group was derived by exchanging the positions of the CH3 domain mutated to the knob or hole included in the CH3 domain (FIG. 14). More specifically, in Q-SBL1, the CH3 domain of the Fab region in the heavy-chain region of the first arm includes a hole (Y349C/T366S/L368A/Y407V) (SEQ ID NO: 11), and the CH3 domain of the Fc region includes a hole (T366S/L368A/Y407V) (SEQ ID NO: 9). The CH3 domain of the Fab region in the light-chain region of the first arm includes a knob (S354C/T366W) (SEQ ID NO: 10). In addition, the CH3 domain of the Fc region in the heavy-chain region of the second arm includes a knob (T366W) (SEQ ID NO: 8).

In Q-SBL6 (SEQ ID NOS: 41, 42, 62, and 63), the CH3 domain of the Fab region in the heavy-chain region of the first arm includes a knob (S354C/T366W) (SEQ ID NO: 10), and the CH3 domain of the Fc region includes a hole (T366S/L368A/Y407V) (SEQ ID NO: 9). The CH3 domain of the Fab region in the light-chain region of the first arm includes a hole (Y349C/T366S/L368A/Y407V) (SEQ ID NO: 11). In addition, the CH3 domain of the Fc region in the heavy-chain region of the second arm includes a knob (T366W) (SEQ ID NO: 8).

In Q-SBL7 (SEQ ID NOS: 43, 44, 62, and 64), the CH3 domain of the Fab region in the heavy-chain region of the first arm includes a hole (Y349C/T366S/L368A/Y407V) (SEQ ID NO: 11) and the CH3 domain of the Fc region includes a knob (T366W) (SEQ ID NO: 8). The CH3 domain of the Fab region in the light-chain region of the first arm includes a knob (S354C/T366W) (SEQ ID NO: 10). In addition, the CH3 domain of the Fc region in the heavy-chain region of the second arm includes a hole (Y349C/T366S/L368A/Y407V) (SEQ ID NO: 11). In Q-SBL8 (SEQ ID NOS: 45, 46, 62, 64), the CH3 domain of the Fab region in the heavy-chain region of the first arm includes a knob (S354C/T366W) (SEQ ID NO: 10), and the CH3 domain of the Fc region includes a knob (T366W) (SEQ ID NO: 8). The CH3 domain of the Fab region in the light-chain region of the first arm includes a hole (Y349C/T366S/L368A/Y407V) (SEQ ID NO: 11). In addition, the CH3 domain of the Fc region in the heavy-chain region of the second arm includes a hole (T366S/L368A/Y407V) (SEQ ID NO: 9).

| **Q-SBL1** | | | | | |
|---|---|---|---|---|---|
| | **1st arm heavy-chain sequence** | **2nd arm heavy-chain sequence** | | **1st arm light-chain sequence** | **2nd arm light-chain sequen ce** ce |
| **VH** | VH | VH | **VL** | VL | VL |
| **Elbow sequen ce** | AS | null | **Elbow sequen ce** | RT | null |
| **CH3** | IgG1 CH3 (Y349C/T366S/L368A/ Y407V) | null | **CH3** | IgG1 CH3 (S354C/T366W) | null |
| **linker** | GGGGSGGGGS | null | **linker** | GGGGSGGGGS | null |
| **CH1** | IgG1 CH1 | IgG1 CH1 | **CL** | kappa CL | kappa CL |
| **Hinge** | DKTHTCPPCP | EPKSCDKTHTCPP CP | | | |
| **CH2** | IgG1 CH2 | IgG1 CH2 | | | |
| **CH3** | IgG1 CH3 (T366S/L368A/Y407V) | IgG1 CH3 (T366W) | | | |

| **Q-SBL6** | | | | | |
|---|---|---|---|---|---|
| | **1st arm heavy-chain sequence** | **2nd arm heavy-chain sequence** | | **1st arm light-chain sequence** | **2nd arm light-chain sequen ce** |
| **VH** | VH | VH | **VL** | VL | VL |
| **Elbow sequen ce** | AS | null | **Elbow sequen ce** | RT | null |
| **CH3** | IgG1 CH3 (S354C/T366W) | null | **CH3** | IgG1 CH3 (Y349C/T366S/L368A/ Y407V) | null |
| **linker** | GGGGSGGGGS | null | **linker** | GGGGSGGGGS | null |
| **CH1** | IgG1 CH1 | IgG1 CH1 | **CL** | kappa CL | kappa CL |
| **Hinge** | DKTHTCPPCP | EPKSCDKTHTCPP CP | | | |
| **CH2** | IgG1 CH2 | IgG1 CH2 | | | |
| **CH3** | IgG1 CH3 (T366S/L368A/Y407V) | IgG1 CH3 (T366W) | | | |

| **Q-SBL7** | | | | | |
|---|---|---|---|---|---|
| | **1st arm heavy-chain sequence** | **2nd arm heavy-chain sequence** | | **1st arm light-chain sequence** | **2nd arm light-chain sequen ce** |
| **VH** | VH | VH | **VL** | VL | VL |
| **Elbow sequen ce** | AS | null | **Elbow sequence** | RT | null |
| **CH3** | IgG1 CH3 (Y349C/T366S/L368A/ Y407V) | null | **CH3** | IgG1 CH3 (S354C/T366W) | null |
| **linker** | GGGGSGGGGS | null | **linker** | GGGGSGGGGS | null |
| **CH1** | IgG1 CH1 | IgG1 CH1 | **CL** | kappa CL | kappa CL |
| **Hinge** | DKTHTCPPCP | EPKSCDKTHTCPP CP | | | |
| **CH2** | IgG1 CH2 | IgG1 CH2 | | | |
| **CH3** | IgG1 CH3 (T366W) | IgG1 CH3 (T366S/L368A/Y4 07V) | | | |

| **Q-SBL8** | | | | | |
|---|---|---|---|---|---|
| | **1st arm heavy-chain sequence** | **2nd arm heavy-chain sequence** | | **1st arm light-chain sequence** | **2nd arm light-chain sequen ce** |
| **VH** | VH | VH | **VL** | VL | VL |
| **Elbow sequen ce** | AS | null | **Elbow sequen ce** | RT | null |
| **CH3** | IgG1 CH3 (S354C/T366W) | null | **CH3** | IgG1 CH3 (Y349C/T366S/L368A/ Y407V) | null |
| **linker** | GGGGSGGGGS | null | **linker** | GGGGSGGGGS | null |
| **CH1** | IgG1 CH1 | IgG1 CH1 | **CL** | kappa CL | kappa CL |
| **Hinge** | DKTHTCPPCP | EPKSCDKTHTCPP CP | | | |
| **CH2** | IgG1 CH2 | IgG1 CH2 | | | |
| **CH3** | IgG1 CH3 (T366W) | IgG1 CH3 (T366S/L368A/Y4 07V) | | | |

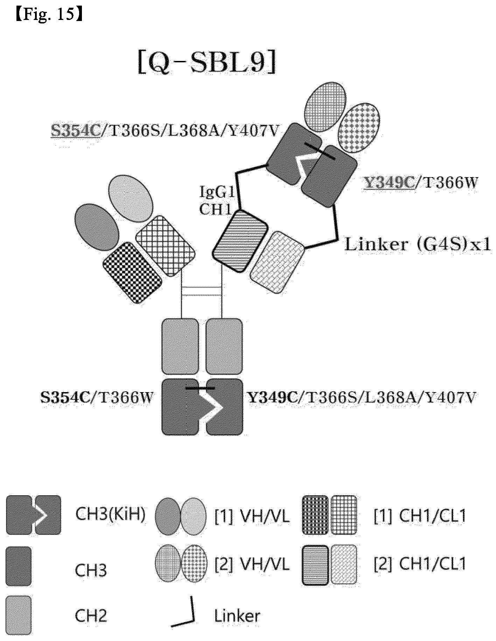

Fourth, Q-SBL9 (SEQ ID NOS: 47, 48, 61, and 62) has disulfide bonds formed through point mutations in other amino acids of the knob and hole parts in the CH3 domain of the heavy-chain region of the first arm, and this is most prominent feature (FIG. 15). More specifically, the Q-SBL9 bispecific antibody format has the following structure of the heavy chain and the light chain of the first arm that binds to the first antigen. The heavy chain of the first arm has the structure of VH-CH3a-Linker-CH1-Hinge-CH2-CH3b. Here, the amino acid sequence of the linker was determined as GGGGS (SEQ ID NO: 27). The CH1 region used herein was the IgG1 CH1 domain. The hinge region amino acid sequence to remove the disulfide bond with the CH1 domain was EPKSSDKTHTCPPCP (SEQ ID NO: 23). The CH3a domain includes a hole mutation and a mutation for a disulfide bond with CH3c of the light chain, and the mutation portion of the CH3a domain is the hole (S354C/T366S/L368A/Y407V) (SEQ ID NO: 13). The CH3b domain includes a mutation in the hole (Y349C/T366S/L368A/Y407V) (SEQ ID NO: 11). The CH3b domain of the first arm forms a disulfide bond with the CH3d domain of the second arm. An elbow sequence was added between the VH region and the CH3a region (SEQ ID NO: 25).

The light chain of the first arm has the configuration of VL-CH3c-Linker-CLb. The CH3c domain of the light chain includes a knob mutation and a mutation for a disulfide bond with CH3a of the heavy chain, and the mutated portion of the CH3c domain is the knob (Y349C/T366W) (SEQ ID NO: 12). The amino acid sequence of the linker is GGGGS (SEQ ID NO: 27). The CLb domain is a kappa type (SEQ ID NO: 15), and the CLb domain includes a C216S (Eu numbering) mutation to remove a disulfide bond with the CH1 domain of the first arm heavy chain. An elbow sequence was added between the VL region and the CH3a region (SEQ ID NO: 26).

The heavy chain and the light chain of the second arm that binds to the second antigen have the following configuration. The heavy chain of the second arm has VH-CH1-CH2-CH3d, and the CH3d domain includes a knob (S354C/T366W) (SEQ ID NO: 10) mutation. This part forms a disulfide bond with the CH3b domain of the heavy-chain region of the first arm. The light chain of the second arm has the structure of VL-CLb. CLb is a kappa type (SEQ ID NO: 14) or lambda type (SEQ ID NO: 16).

| **Q-SBL9** | | | | | |
|---|---|---|---|---|---|
| | **1st arm heavy-chain sequence** | **2nd arm heavy-chain sequence** | | **1st arm light-chain sequence** | **2nd arm light-chain sequenc e** |
| **VH** | VH | VH | **VL** | VL | VL |
| **Elbow sequence** | AS | null | **Elbow sequence** | RT | null |
| **CH3** | IgG1 CH3 (S354C/T366S/L368A/Y407 V) | null | **CH3** | IgG1 CH3 (Y349C/T366 W) | null |
| **linker** | GGGGS | null | **linker** | GGGGS | null |
| **CH1** | IgG1 CH1 | IgG1 CH1 | **CL** | kappa CL | kappa CL |
| **Hinge** | EPKSCDKTHTCPPCP | EPKSCDKTHTCPP CP | | | |
| **CH2** | IgG1 CH2 | IgG1 CH2 | | | |
| **CH3** | IgG1 CH3 (Y349C/T366S/L368A/Y407 V) | IgG1 CH3 (S354C/T366W) | | | |

The specific configuration of each candidate is as follows.

| Configu ration | Sequence | No. |
|---|---|---|
| Q-SBL1 HC (First arm heavy-chain region) | EVQLVESGGGLVQPGGSLRLSCAASGFNIKDTYIHWVRQAPGKGLEWVARI YPTNGYTRYADSVKGRFTISADTSKNTAYLQMNSLRAEDTAVYYCSRWGGD GFYAMDYWGQGTLVTVSSASPREPQVCTLPPSREEMTKNQVSLSCAVKGFY PSDIAVEWESNGQPENNYKTTPPVLDSDGSFFLVSKLTVDKSRWQQGNVFS CSVMHEALHNHYTQKSLSLSPGKGGGGSGGGGSASTKGPSVFPLAPSSKST SGGTAALGCLVKDYFPEPVTVSWNSGALTSGVHTFPAVLQSSGLYSLSSVV TVPSSSLGTQTYICNVNHKPSNTKVDKKVDKTHTCPPCPAPELLGGPSVFL FPPKPKDTLMISRTPEVTCVVVDVSHEDPEVKFNWYVDGVEVHNAKTKPRE EQYNSTYRVVSVLTVLHQDWLNGKEYKCKVSNKALPAPIEKTISKAKGQPR EPQVYTLPPSREEMTKNQVSLSCAVKGFYPSDIAVEWESNGQPENNYKTTP PVLDSDGSFFLVSKLTVDKSRWQQGNVFSCSVMHEALHNHYTQKSLSLSPG K | 31 |
| Q-SBL1 LC (First arm light-chain region) | DIQMTQSPSSLSASVGDRVTITCRASQDVNTAVAWYQQKPGKAPKLLIYSA SFLYSGVPSRFSGSRSGTDFTLTISSLQPEDFATYYCQQHYTTPPTFGQGT KVEIKRTPREPQVYTLPPCREEMTKNQVSLWCLVKGFYPSDIAVEWESNGQ PENNYKTTPPVLDSDGSFFLYSKLTVDKSRWQQGNVFSCSVMHEALHNHYT QKSLSLSPGKGGGGSGGGGSRTVAAPSVFIFPPSDEQLKSGTASVVCLLNN FYPREAKVQWKVDNALQSGNSQESVTEQDSKDSTYSLSSTLTLSKADYEKH KVYACEVTHQGLSSPVTKSFNRGES | 32 |
| Q-SBL2 HC (First arm heavy-chain region) | EVQLVESGGGLVQPGGSLRLSCAASGFNIKDTYIHWVRQAPGKGLEWVARI YPTNGYTRYADSVKGRFTISADTSKNTAYLQMNSLRAEDTAVYYCSRWGGD GFYAMDYWGQGTLVTVSSASPREPQVCTLPPSREEMTKNQVSLSCAVKGFY PSDIAVEWESNGQPENNYKTTPPVLDSDGSFFLVSKLTVDKSRWQQGNVFS CSVMHEALHNHYTQKSLSLSPGKGGGGSASTKGPSVFPLAPSSKSTSGGTA ALGCLVKDYFPEPVTVSWNSGALTSGVHTFPAVLQSSGLYSLSSVVTVPSS SLGTQTYICNVNHKPSNTKVDKKVDKTHTCPPCPAPELLGGPSVFLFPPKP KDTLMISRTPEVTCVVVDVSHEDPEVKFNWYVDGVEVHNAKTKPREEQYNS TYRVVSVLTVLHQDWLNGKEYKCKVSNKALPAPIEKTISKAKGQPREPQVY TLPPSREEMTKNQVSLSCAVKGFYPSDIAVEWESNGQPENNYKTTPPVLDS DGSFFLVSKLTVDKSRWQQGNVFSCSVMHEALHNHYTQKSLSLSPGK | 33 |
| Q-SBL2 LC (First arm light-chain region) | DIQMTQSPSSLSASVGDRVTITCRASQDVNTAVAWYQQKPGKAPKLLIYSA SFLYSGVPSRFSGSRSGTDFTLTISSLQPEDFATYYCQQHYTTPPTFGQGT KVEIKRTPREPQVYTLPPCREEMTKNQVSLWCLVKGFYPSDIAVEWESNGQ PENNYKTTPPVLDSDGSFFLYSKLTVDKSRWQQGNVFSCSVMHEALHNHYT QKSLSLSPGKGGGGSRTVAAPSVFIFPPSDEQLKSGTASVVCLLNNFYPRE AKVQWKVDNALQSGNSQESVTEQDSKDSTYSLSSTLTLSKADYEKHKVYAC EVTHQGLSSPVTKSFNRGES | 34 |
| Q-SBL3 HC (First arm heavy-chain region) | EVQLVESGGGLVQPGGSLRLSCAASGFNIKDTYIHWVRQAPGKGLEWVARI YPTNGYTRYADSVKGRFTISADTSKNTAYLQMNSLRAEDTAVYYCSRWGGD GFYAMDYWGQGTLVTVSSASPREPQVCTLPPSREEMTKNQVSLSCAVKGFY PSDIAVEWESNGQPENNYKTTPPVLDSDGSFFLVSKLTVDKSRWQQGNVFS CSVMHEALHNHYTQKSLSLSPGKGGGGSGGGGSGGGGSASTKGPSVFPLAP SSKSTSGGTAALGCLVKDYFPEPVTVSWNSGALTSGVHTFPAVLQSSGLYS LSSVVTVPSSSLGTQTYICNVNHKPSNTKVDKKVDKTHTCPPCPAPELLGG PSVFLFPPKPKDTLMISRTPEVTCVVVDVSHEDPEVKFNWYVDGVEVHNAK TKPREEQYNSTYRVVSVLTVLHQDWLNGKEYKCKVSNKALPAPIEKTISKA KGQPREPQVYTLPPSREEMTKNQVSLSCAVKGFYPSDIAVEWESNGQPENN YKTTPPVLDSDGSFFLVSKLTVDKSRWQQGNVFSCSVMHEALHNHYTQKSL SLSPGK | 35 |
| Q-SBL3 LC (First arm light-chain region) | DIQMTQSPSSLSASVGDRVTITCRASQDVNTAVAWYQQKPGKAPKLLIYSA SFLYSGVPSRFSGSRSGTDFTLTISSLQPEDFATYYCQQHYTTPPTFGQGT KVEIKRTPREPQVYTLPPCREEMTKNQVSLWCLVKGFYPSDIAVEWESNGQ PENNYKTTPPVLDSDGSFFLYSKLTVDKSRWQQGNVFSCSVMHEALHNHYT QKSLSLSPGKGGGGSGGGGSGGGGSRTVAAPSVFIFPPSDEQLKSGTASVV CLLNNFYPREAKVQWKVDNALQSGNSQESVTEQDSKDSTYSLSSTLTLSKA DYEKHKVYACEVTHQGLSSPVTKSFNRGES | 36 |
| Q-SBL4 HC (First arm heavy-chain region) | EVQLVESGGGLVQPGGSLRLSCAASGFNIKDTYIHWVRQAPGKGLEWVARI YPTNGYTRYADSVKGRFTISADTSKNTAYLQMNSLRAEDTAVYYCSRWGGD GFYAMDYWGQGTLVTVSSASPREPQVCTLPPSREEMTKNQVSLSCAVKGFY PSDIAVEWESNGQPENNYKTTPPVLDSDGSFFLVSKLTVDKSRWQQGNVFS CSVMHEALHNHYTQKSLSLSPGKGGGGSGGGGSGGGGSGGGGSASTKGPSV FPLAPSSKSTSGGTAALGCLVKDYFPEPVTVSWNSGALTSGVHTFPAVLQS SGLYSLSSVVTVPSSSLGTQTYICNVNHKPSNTKVDKKVDKTHTCPPCPAP ELLGGPSVFLFPPKPKDTLMISRTPEVTCVVVDVSHEDPEVKFNWYVDGVE VHNAKTKPREEQYNSTYRVVSVLTVLHQDWLNGKEYKCKVSNKALPAPIEK TISKAKGQPREPQVYTLPPSREEMTKNQVSLSCAVKGFYPSDIAVEWESNG QPENNYKTTPPVLDSDGSFFLVSKLTVDKSRWQQGNVFSCSVMHEALHNHY TQKSLSLSPGK | 37 |
| Q-SBL4 LC (First arm light-chain region) | DIQMTQSPSSLSASVGDRVTITCRASQDVNTAVAWYQQKPGKAPKLLIYSA SFLYSGVPSRFSGSRSGTDFTLTISSLQPEDFATYYCQQHYTTPPTFGQGT KVEIKRTPREPQVYTLPPCREEMTKNQVSLWCLVKGFYPSDIAVEWESNGQ PENNYKTTPPVLDSDGSFFLYSKLTVDKSRWQQGNVFSCSVMHEALHNHYT QKSLSLSPGKGGGGSGGGGSGGGGSGGGGSRTVAAPSVFIFPPSDEQLKSG TASVVCLLNNFYPREAKVQWKVDNALQSGNSQESVTEQDSKDSTYSLSSTL TLSKADYEKHKVYACEVTHQGLSSPVTKSFNRGES | 38 |
| Q-SBL5 HC (First arm heavy-chain region) | EVQLVESGGGLVQPGGSLRLSCAASGFNIKDTYIHWVRQAPGKGLEWVARI YPTNGYTRYADSVKGRFTISADTSKNTAYLQMNSLRAEDTAVYYCSRWGGD GFYAMDYWGQGTLVTVSSASPREPQVCTLPPSREEMTKNQVSLSCAVKGFY PSDIAVEWESNGQPENNYKTTPPVLDSDGSFFLVSKLTVDKSRWQQGNVFS CSVMHEALHNHYTQKSLSLSPGKGGGGSGGGGSASTKGPSVFPLAPSSKST SGGTAALGCLVKDYFPEPVTVSWNSGALTSGVHTFPAVLQSSGLYSLSSVV TVPSSSLGTQTYICNVNHKPSNTKVDKKVEPKSSDKTHTCPPCPAPELLGG PSVFLFPPKPKDTLMISRTPEVTCVVVDVSHEDPEVKFNWYVDGVEVHNAK TKPREEQYNSTYRVVSVLTVLHQDWLNGKEYKCKVSNKALPAPIEKTISKA KGQPREPQVYTLPPSREEMTKNQVSLSCAVKGFYPSDIAVEWESNGQPENN YKTTPPVLDSDGSFFLVSKLTVDKSRWQQGNVFSCSVMHEALHNHYTQKSL SLSPGK | 39 |
| Q-SBL5 LC (First arm light-chain region) | DIQMTQSPSSLSASVGDRVTITCRASQDVNTAVAWYQQKPGKAPKLLIYSA SFLYSGVPSRFSGSRSGTDFTLTISSLQPEDFATYYCQQHYTTPPTFGQGT KVEIKRTPREPQVYTLPPCREEMTKNQVSLWCLVKGFYPSDIAVEWESNGQ PENNYKTTPPVLDSDGSFFLYSKLTVDKSRWQQGNVFSCSVMHEALHNHYT QKSLSLSPGKGGGGSGGGGSRTVAAPSVFIFPPSDEQLKSGTASVVCLLNN FYPREAKVQWKVDNALQSGNSQESVTEQDSKDSTYSLSSTLTLSKADYEKH KVYACEVTHQGLSSPVTKSFNRGES | 40 |
| Q-SBL6 HC (First arm heavy-chain region) | EVQLVESGGGLVQPGGSLRLSCAASGFNIKDTYIHWVRQAPGKGLEWVARI YPTNGYTRYADSVKGRFTISADTSKNTAYLQMNSLRAEDTAVYYCSRWGGD GFYAMDYWGQGTLVTVSSASPREPQVYTLPPCREEMTKNQVSLWCLVKGFY PSDIAVEWESNGQPENNYKTTPPVLDSDGSFFLYSKLTVDKSRWQQGNVFS CSVMHEALHNHYTQKSLSLSPGKGGGGSGGGGSASTKGPSVFPLAPSSKST SGGTAALGCLVKDYFPEPVTVSWNSGALTSGVHTFPAVLQSSGLYSLSSVV TVPSSSLGTQTYICNVNHKPSNTKVDKKVDKTHTCPPCPAPELLGGPSVFL FPPKPKDTLMISRTPEVTCVVVDVSHEDPEVKFNWYVDGVEVHNAKTKPRE EQYNSTYRVVSVLTVLHQDWLNGKEYKCKVSNKALPAPIEKTISKAKGQPR EPQVYTLPPSREEMTKNQVSLSCAVKGFYPSDIAVEWESNGQPENNYKTTP PVLDSDGSFFLVSKLTVDKSRWQQGNVFSCSVMHEALHNHYTQKSLSLSPG K | 41 |
| Q-SBL6 LC (First arm light-chain region) | DIQMTQSPSSLSASVGDRVTITCRASQDVNTAVAWYQQKPGKAPKLLIYSA SFLYSGVPSRFSGSRSGTDFTLTISSLQPEDFATYYCQQHYTTPPTFGQGT KVEIKRTPREPQVCTLPPSREEMTKNQVSLSCAVKGFYPSDIAVEWESNGQ PENNYKTTPPVLDSDGSFFLVSKLTVDKSRWQQGNVFSCSVMHEALHNHYT QKSLSLSPGKGGGGSGGGGSRTVAAPSVFIFPPSDEQLKSGTASVVCLLNN FYPREAKVQWKVDNALQSGNSQESVTEQDSKDSTYSLSSTLTLSKADYEKH KVYACEVTHQGLSSPVTKSFNRGES | 42 |
| Q-SBL7 HC (First arm heavy-chain region) | EVQLVESGGGLVQPGGSLRLSCAASGFNIKDTYIHWVRQAPGKGLEWVARI YPTNGYTRYADSVKGRFTISADTSKNTAYLQMNSLRAEDTAVYYCSRWGGD GFYAMDYWGQGTLVTVSSASPREPQVCTLPPSREEMTKNQVSLSCAVKGFY PSDIAVEWESNGQPENNYKTTPPVLDSDGSFFLVSKLTVDKSRWQQGNVFS CSVMHEALHNHYTQKSLSLSPGKGGGGSGGGGSASTKGPSVFPLAPSSKST SGGTAALGCLVKDYFPEPVTVSWNSGALTSGVHTFPAVLQSSGLYSLSSVV TVPSSSLGTQTYICNVNHKPSNTKVDKKVDKTHTCPPCPAPELLGGPSVFL FPPKPKDTLMISRTPEVTCVVVDVSHEDPEVKFNWYVDGVEVHNAKTKPRE EQYNSTYRVVSVLTVLHQDWLNGKEYKCKVSNKALPAPIEKTISKAKGQPR EPQVYTLPPSREEMTKNQVSLWCLVKGFYPSDIAVEWESNGQPENNYKTTP PVLDSDGSFFLYSKLTVDKSRWQQGNVFSCSVMHEALHNHYTQKSLSLSPG K | 43 |
| Q-SBL7 LC (First arm light-chain region) | DIQMTQSPSSLSASVGDRVTITCRASQDVNTAVAWYQQKPGKAPKLLIYSA SFLYSGVPSRFSGSRSGTDFTLTISSLQPEDFATYYCQQHYTTPPTFGQGT KVEIKRTPREPQVYTLPPCREEMTKNQVSLWCLVKGFYPSDIAVEWESNGQ PENNYKTTPPVLDSDGSFFLYSKLTVDKSRWQQGNVFSCSVMHEALHNHYT QKSLSLSPGKGGGGSGGGGSRTVAAPSVFIFPPSDEQLKSGTASVVCLLNN FYPREAKVQWKVDNALQSGNSQESVTEQDSKDSTYSLSSTLTLSKADYEKH KVYACEVTHQGLSSPVTKSFNRGES | 44 |
| Q-SBL8 HC (First arm heavy-chain region) | EVQLVESGGGLVQPGGSLRLSCAASGFNIKDTYIHWVRQAPGKGLEWVARI YPTNGYTRYADSVKGRFTISADTSKNTAYLQMNSLRAEDTAVYYCSRWGGD GFYAMDYWGQGTLVTVSSASPREPQVYTLPPCREEMTKNQVSLWCLVKGFY PSDIAVEWESNGQPENNYKTTPPVLDSDGSFFLYSKLTVDKSRWQQGNVFS CSVMHEALHNHYTQKSLSLSPGKGGGGSGGGGSASTKGPSVFPLAPSSKST SGGTAALGCLVKDYFPEPVTVSWNSGALTSGVHTFPAVLQSSGLYSLSSVV TVPSSSLGTQTYICNVNHKPSNTKVDKKVDKTHTCPPCPAPELLGGPSVFL FPPKPKDTLMISRTPEVTCVVVDVSHEDPEVKFNWYVDGVEVHNAKTKPRE EQYNSTYRVVSVLTVLHQDWLNGKEYKCKVSNKALPAPIEKTISKAKGQPR EPQVYTLPPSREEMTKNQVSLWCLVKGFYPSDIAVEWESNGQPENNYKTTP PVLDSDGSFFLYSKLTVDKSRWQQGNVFSCSVMHEALHNHYTQKSLSLSPG K | 45 |
| Q-SBL8 LC (First arm light-chain region) | DIQMTQSPSSLSASVGDRVTITCRASQDVNTAVAWYQQKPGKAPKLLIYSA SFLYSGVPSRFSGSRSGTDFTLTISSLQPEDFATYYCQQHYTTPPTFGQGT KVEIKRTPREPQVCTLPPSREEMTKNQVSLSCAVKGFYPSDIAVEWESNGQ PENNYKTTPPVLDSDGSFFLVSKLTVDKSRWQQGNVFSCSVMHEALHNHYT QKSLSLSPGKGGGGSGGGGSRTVAAPSVFIFPPSDEQLKSGTASVVCLLNN FYPREAKVQWKVDNALQSGNSQESVTEQDSKDSTYSLSSTLTLSKADYEKH KVYACEVTHQGLSSPVTKSFNRGES | 46 |
| Q-SBL9 HC (First arm heavy-chain region) | EVQLVESGGGLVQPGGSLRLSCAASGFNIKDTYIHWVRQAPGKGLEWVARI YPTNGYTRYADSVKGRFTISADTSKNTAYLQMNSLRAEDTAVYYCSRWGGD GFYAMDYWGQGTLVTVSSASPREPQVYTLPPCREEMTKNQVSLSCAVKGFY PSDIAVEWESNGQPENNYKTTPPVLDSDGSFFLVSKLTVDKSRWQQGNVFS CSVMHEALHNHYTQKSLSLSPGKGGGGSASTKGPSVFPLAPSSKSTSGGTA ALGCLVKDYFPEPVTVSWNSGALTSGVHTFPAVLQSSGLYSLSSVVTVPSS SLGTQTYICNVNHKPSNTKVDKKVEPKSSDKTHTCPPCPAPELLGGPSVFL FPPKPKDTLMISRTPEVTCVVVDVSHEDPEVKFNWYVDGVEVHNAKTKPRE EQYNSTYRVVSVLTVLHQDWLNGKEYKCKVSNKALPAPIEKTISKAKGQPR EPQVCTLPPSREEMTKNQVSLSCAVKGFYPSDIAVEWESNGQPENNYKTTP PVLDSDGSFFLVSKLTVDKSRWQQGNVFSCSVMHEALHNHYTQKSLSLSPG K | 47 |
| Q-SBL9 LC (First arm light-chain region) | DIQMTQSPSSLSASVGDRVTITCRASQDVNTAVAWYQQKPGKAPKLLIYSA SFLYSGVPSRFSGSRSGTDFTLTISSLQPEDFATYYCQQHYTTPPTFGQGT KVEIKRTPREPQVCTLPPSREEMTKNQVSLWCLVKGFYPSDIAVEWESNGQ PENNYKTTPPVLDSDGSFFLYSKLTVDKSRWQQGNVFSCSVMHEALHNHYT QKSLSLSPGKGGGGSRTVAAPSVFIFPPSDEQLKSGTASVVCLLNNFYPRE AKVQWKVDNALQSGNSQESVTEQDSKDSTYSLSSTLTLSKADYEKHKVYAC EVTHQGLSSPVTKSFNRGES | 48 |
| R-SBL1 HC (First arm heavy-chain region) | EVQLVESGGGLVQPGGSLRLSCAASGFNIKDTYIHWVRQAPGKGLEWVARI YPTNGYTRYADSVKGRFTISADTSKNTAYLQMNSLRAEDTAVYYCSRWGGD GFYAMDYWGQGTLVTVSSASPREPQVCTLPPSREEMTKNQVSLSCAVKGFY PSDIAVEWESNGQPENNYKTTPPVLDSDGSFFLVSKLTVDKSRWQQGNVFS CSVMHEALHNHYTQKSLSLSPGKGGGGSGGGGSASTKGPSVFPLAPSSRST SESTAALGCLVKDYFPEPVTVSWNSGALTSGVHTFPAVLQSSGLYSLSSVV TVPSSSLGTKTYTCNVDHKPSNTKVDKRVDKTHTCPPCPAPELLGGPSVFL FPPKPKDTLMISRTPEVTCVVVDVSHEDPEVKFNWYVDGVEVHNAKTKPRE EQYNSTYRVVSVLTVLHQDWLNGKEYKCKVSNKALPAPIEKTISKAKGQPR EPQVYTLPPSREEMTKNQVSLSCAVKGFYPSDIAVEWESNGQPENNYKTTP PVLDSDGSFFLVSKLTVDKSRWQQGNVFSCSVMHEALHNHYTQKSLSLSPG K | 49 |
| R-SBL1 LC (First arm light-chain region) | DIQMTQSPSSLSASVGDRVTITCRASQDVNTAVAWYQQKPGKAPKLLIYSA SFLYSGVPSRFSGSRSGTDFTLTISSLQPEDFATYYCQQHYTTPPTFGQGT KVEIKRTPREPQVYTLPPCREEMTKNQVSLWCLVKGFYPSDIAVEWESNGQ PENNYKTTPPVLDSDGSFFLYSKLTVDKSRWQQGNVFSCSVMHEALHNHYT QKSLSLSPGKGGGGSGGGGSRTVAAPSVFIFPPSDEQLKSGTASVVCLLNN FYPREAKVQWKVDNALQSGNSQESVTEQDSKDSTYSLSSTLTLSKADYEKH KVYACEVTHQGLSSPVTKSFNRGES | 50 |
| R-SBL2 HC (First arm heavy-chain region) | EVQLVESGGGLVQPGGSLRLSCAASGFNIKDTYIHWVRQAPGKGLEWVARI YPTNGYTRYADSVKGRFTISADTSKNTAYLQMNSLRAEDTAVYYCSRWGGD GFYAMDYWGQGTLVTVSSASPREPQVCTLPPSREEMTKNQVSLSCAVKGFY PSDIAVEWESNGQPENNYKTTPPVLDSDGSFFLVSKLTVDKSRWQQGNVFS CSVMHEALHNHYTQKSLSLSPGKGGGGSASTKGPSVFPLAPSSRSTSESTA ALGCLVKDYFPEPVTVSWNSGALTSGVHTFPAVLQSSGLYSLSSVVTVPSS SLGTKTYTCNVDHKPSNTKVDKRVDKTHTCPPCPAPELLGGPSVFLFPPKP KDTLMISRTPEVTCVVVDVSHEDPEVKFNWYVDGVEVHNAKTKPREEQYNS TYRVVSVLTVLHQDWLNGKEYKCKVSNKALPAPIEKTISKAKGQPREPQVY TLPPSREEMTKNQVSLSCAVKGFYPSDIAVEWESNGQPENNYKTTPPVLDS DGSFFLVSKLTVDKSRWQQGNVFSCSVMHEALHNHYTQKSLSLSPGK* | 51 |
| R-SBL2 LC (First arm light-chain region) | DIQMTQSPSSLSASVGDRVTITCRASQDVNTAVAWYQQKPGKAPKLLIYSA SFLYSGVPSRFSGSRSGTDFTLTISSLQPEDFATYYCQQHYTTPPTFGQGT KVEIKRTPREPQVYTLPPCREEMTKNQVSLWCLVKGFYPSDIAVEWESNGQ PENNYKTTPPVLDSDGSFFLYSKLTVDKSRWQQGNVFSCSVMHEALHNHYT QKSLSLSPGKGGGGSRTVAAPSVFIFPPSDEQLKSGTASVVCLLNNFYPRE AKVQWKVDNALQSGNSQESVTEQDSKDSTYSLSSTLTLSKADYEKHKVYAC EVTHQGLSSPVTKSFNRGES | 52 |
| R-SBL3 HC (First arm heavy-chain region) | EVQLVESGGGLVQPGGSLRLSCAASGFNIKDTYIHWVRQAPGKGLEWVARI YPTNGYTRYADSVKGRFTISADTSKNTAYLQMNSLRAEDTAVYYCSRWGGD GFYAMDYWGQGTLVTVSSASPREPQVCTLPPSREEMTKNQVSLSCAVKGFY PSDIAVEWESNGQPENNYKTTPPVLDSDGSFFLVSKLTVDKSRWQQGNVFS CSVMHEALHNHYTQKSLSLSPGKGGGGSGGGGSGGGGSASTKGPSVFPLAP SSRSTSESTAALGCLVKDYFPEPVTVSWNSGALTSGVHTFPAVLQSSGLYS LSSVVTVPSSSLGTKTYTCNVDHKPSNTKVDKRVDKTHTCPPCPAPELLGG PSVFLFPPKPKDTLMISRTPEVTCVVVDVSHEDPEVKFNWYVDGVEVHNAK TKPREEQYNSTYRVVSVLTVLHQDWLNGKEYKCKVSNKALPAPIEKTISKA KGQPREPQVYTLPPSREEMTKNQVSLSCAVKGFYPSDIAVEWESNGQPENN YKTTPPVLDSDGSFFLVSKLTVDKSRWQQGNVFSCSVMHEALHNHYTQKSL SLSPGK | 53 |
| R-SBL3 LC (First arm light-chain region) | DIQMTQSPSSLSASVGDRVTITCRASQDVNTAVAWYQQKPGKAPKLLIYSA SFLYSGVPSRFSGSRSGTDFTLTISSLQPEDFATYYCQQHYTTPPTFGQGT KVEIKRTPREPQVYTLPPCREEMTKNQVSLWCLVKGFYPSDIAVEWESNGQ PENNYKTTPPVLDSDGSFFLYSKLTVDKSRWQQGNVFSCSVMHEALHNHYT QKSLSLSPGKGGGGSGGGGSGGGGSRTVAAPSVFIFPPSDEQLKSGTASVV CLLNNFYPREAKVQWKVDNALQSGNSQESVTEQDSKDSTYSLSSTLTLSKA DYEKHKVYACEVTHQGLSSPVTKSFNRGES | 54 |
| R-SBL4 HC (First arm heavy-chain region) | EVQLVESGGGLVQPGGSLRLSCAASGFNIKDTYIHWVRQAPGKGLEWVARI YPTNGYTRYADSVKGRFTISADTSKNTAYLQMNSLRAEDTAVYYCSRWGGD GFYAMDYWGQGTLVTVSSASPREPQVCTLPPSREEMTKNQVSLSCAVKGFY PSDIAVEWESNGQPENNYKTTPPVLDSDGSFFLVSKLTVDKSRWQQGNVFS CSVMHEALHNHYTQKSLSLSPGKGGGGSGGGGSGGGGSGGGGSASTKGPSV FPLAPSSRSTSESTAALGCLVKDYFPEPVTVSWNSGALTSGVHTFPAVLQS SGLYSLSSVVTVPSSSLGTKTYTCNVDHKPSNTKVDKRVDKTHTCPPCPAP ELLGGPSVFLFPPKPKDTLMISRTPEVTCVVVDVSHEDPEVKFNWYVDGVE VHNAKTKPREEQYNSTYRVVSVLTVLHQDWLNGKEYKCKVSNKALPAPIEK TISKAKGQPREPQVYTLPPSREEMTKNQVSLSCAVKGFYPSDIAVEWESNG QPENNYKTTPPVLDSDGSFFLVSKLTVDKSRWQQGNVFSCSVMHEALHNHY TQKSLSLSPGK | 55 |
| R-SBL4 LC (First arm light-chain region) | DIQMTQSPSSLSASVGDRVTITCRASQDVNTAVAWYQQKPGKAPKLLIYSA SFLYSGVPSRFSGSRSGTDFTLTISSLQPEDFATYYCQQHYTTPPTFGQGT KVEIKRTPREPQVYTLPPCREEMTKNQVSLWCLVKGFYPSDIAVEWESNGQ PENNYKTTPPVLDSDGSFFLYSKLTVDKSRWQQGNVFSCSVMHEALHNHYT QKSLSLSPGKGGGGSGGGGSGGGGSGGGGSRTVAAPSVFIFPPSDEQLKSG TASVVCLLNNFYPREAKVQWKVDNALQSGNSQESVTEQDSKDSTYSLSSTL TLSKADYEKHKVYACEVTHQGLSSPVTKSFNRGES | 56 |
| R-SBL5 HC (First arm heavy-chain region) | EVQLVESGGGLVQPGGSLRLSCAASGFNIKDTYIHWVRQAPGKGLEWVARI YPTNGYTRYADSVKGRFTISADTSKNTAYLQMNSLRAEDTAVYYCSRWGGD GFYAMDYWGQGTLVTVSSASPREPQVCTLPPSREEMTKNQVSLSCAVKGFY PSDIAVEWESNGQPENNYKTTPPVLDSDGSFFLVSKLTVDKSRWQQGNVFS CSVMHEALHNHYTQKSLSLSPGKGGGGSGGGGSASTKGPSVFPLAPSSRST SESTAALGCLVKDYFPEPVTVSWNSGALTSGVHTFPAVLQSSGLYSLSSVV TVPSSSLGTKTYTCNVDHKPSNTKVDKRVEPKSSDKTHTCPPCPAPELLGG PSVFLFPPKPKDTLMISRTPEVTCVVVDVSHEDPEVKFNWYVDGVEVHNAK TKPREEQYNSTYRVVSVLTVLHQDWLNGKEYKCKVSNKALPAPIEKTISKA KGQPREPQVYTLPPSREEMTKNQVSLSCAVKGFYPSDIAVEWESNGQPENN YKTTPPVLDSDGSFFLVSKLTVDKSRWQQGNVFSCSVMHEALHNHYTQKSL SLSPGK | 57 |
| R-SBL5 LC (First arm light-chain region) | DIQMTQSPSSLSASVGDRVTITCRASQDVNTAVAWYQQKPGKAPKLLIYSA SFLYSGVPSRFSGSRSGTDFTLTISSLQPEDFATYYCQQHYTTPPTFGQGT KVEIKRTPREPQVYTLPPCREEMTKNQVSLWCLVKGFYPSDIAVEWESNGQ PENNYKTTPPVLDSDGSFFLYSKLTVDKSRWQQGNVFSCSVMHEALHNHYT QKSLSLSPGKGGGGSGGGGSRTVAAPSVFIFPPSDEQLKSGTASVVCLLNN FYPREAKVQWKVDNALQSGNSQESVTEQDSKDSTYSLSSTLTLSKADYEKH KVYACEVTHQGLSSPVTKSFNRGES | 58 |
| R-SBL6 HC (First arm heavy-chain region) | EVQLVESGGGLVQPGGSLRLSCAASGFNIKDTYIHWVRQAPGKGLEWVARI YPTNGYTRYADSVKGRFTISADTSKNTAYLQMNSLRAEDTAVYYCSRWGGD GFYAMDYWGQGTLVTVSSASPREPQVCTLPPSREEMTKNQVSLSCAVKGFY PSDIAVEWESNGQPENNYKTTPPVLDSDGSFFLVSKLTVDKSRWQQGNVFS CSVMHEALHNHYTQKSLSLSPGKGGGGSGGGGSASTKGPSVFPLAPSSRST SESTAALGCLVKDYFPEPVTVSWNSGALTSGVHTFPAVLQSSGLYSLSSVV TVPSSSLGTKTYTCNVDHKPSNTKVDKRVESKYGPPCPPCPAPELLGGPSV FLFPPKPKDTLMISRTPEVTCVVVDVSHEDPEVKFNWYVDGVEVHNAKTKP REEQYNSTYRVVSVLTVLHQDWLNGKEYKCKVSNKALPAPIEKTISKAKGQ PREPQVYTLPPSREEMTKNQVSLSCAVKGFYPSDIAVEWESNGQPENNYKT TPPVLDSDGSFFLVSKLTVDKSRWQQGNVFSCSVMHEALHNHYTQKSLSLS PGK | 59 |
| R-SBL6 LC (First arm light-chain region) | DIQMTQSPSSLSASVGDRVTITCRASQDVNTAVAWYQQKPGKAPKLLIYSA SFLYSGVPSRFSGSRSGTDFTLTISSLQPEDFATYYCQQHYTTPPTFGQGT KVEIKRTPREPQVYTLPPCREEMTKNQVSLWCLVKGFYPSDIAVEWESNGQ PENNYKTTPPVLDSDGSFFLYSKLTVDKSRWQQGNVFSCSVMHEALHNHYT QKSLSLSPGKGGGGSGGGGSRTVAAPSVFIFPPSDEQLKSGTASVVCLLNN FYPREAKVQWKVDNALQSGNSQESVTEQDSKDSTYSLSSTLTLSKADYEKH KVYACEVTHQGLSSPVTKSFNRGES | 60 |
| LC-1 HC ( second arm heavy-chain region) | EVQLVESGGGLVQPGGSLRLSCAASGYTFTNYGMNWVRQAPGKGLEWVGWI NTYTGEPTYAADFKRRFTFSLDTSKSTAYLQMNSLRAEDTAVYYCAKYPHY YGSSHWYFDVWGQGTLVTVSSASTKGPSVFPLAPSSKSTSGGTAALGCLVK DYFPEPVTVSWNSGALTSGVHTFPAVLQSSGLYSLSSVVTVPSSSLGTQTY ICNVNHKPSNTKVDKKVEPKSCDKTHTCPPCPAPELLGGPSVFLFPPKPKD TLMISRTPEVTCVVVDVSHEDPEVKFNWYVDGVEVHNAKTKPREEQYNSTY RVVSVLTVLHQDWLNGKEYKCKVSNKALPAPIEKTISKAKGQPREPQVYTL PPCREEMTKNQVSLWCLVKGFYPSDIAVEWESNGQPENNYKTTPPVLDSDG SFFLYSKLTVDKSRWQQGNVFSCSVMHEALHNHYTQKSLSLSPGK | 61 |
| LC-1 LC ( second arm light-chain region) | DIQMTQSPSSLSASVGDRVTITCSASQDISNYLNWYQQKPGKAPKVLIYFT SSLHSGVPSRFSGSGSGTDFTLTISSLQPEDFATYYCQQYSTVPWTFGQGT KVEIKRTVAAPSVFIFPPSDEQLKSGTASVVCLLNNFYPREAKVQWKVDNA LQSGNSQESVTEQDSKDSTYSLSSTLTLSKADYEKHKVYACEVTHQGLSSP VTKSFNRGEC | 62 |
| LC-3 HC ( second arm heavy-chain region) | EVQLVESGGGLVQPGGSLRLSCAASGYTFTNYGMNWVRQAPGKGLEWVGWI NTYTGEPTYAADFKRRFTFSLDTSKSTAYLQMNSLRAEDTAVYYCAKYPHY YGSSHWYFDVWGQGTLVTVSSASTKGPSVFPLAPSSKSTSGGTAALGCLVK DYFPEPVTVSWNSGALTSGVHTFPAVLQSSGLYSLSSVVTVPSSSLGTQTY ICNVNHKPSNTKVDKKVEPKSCDKTHTCPPCPAPELLGGPSVFLFPPKPKD TLMISRTPEVTCVVVDVSHEDPEVKFNWYVDGVEVHNAKTKPREEQYNSTY RVVSVLTVLHQDWLNGKEYKCKVSNKALPAPIEKTISKAKGQPREPQVYTL PPSREEMTKNQVSLWCLVKGFYPSDIAVEWESNGQPENNYKTTPPVLDSDG SFFLYSKLTVDKSRWQQGNVFSCSVMHEALHNHYTQKSLSLSPGK | 63 |
| LC-3-H-K HC (second arm heavy-chain region) | EVQLVESGGGLVQPGGSLRLSCAASGYTFTNYGMNWVRQAPGKGLEWVGWI NTYTGEPTYAADFKRRFTFSLDTSKSTAYLQMNSLRAEDTAVYYCAKYPHY YGSSHWYFDVWGQGTLVTVSSASTKGPSVFPLAPSSKSTSGGTAALGCLVK DYFPEPVTVSWNSGALTSGVHTFPAVLQSSGLYSLSSVVTVPSSSLGTQTY ICNVNHKPSNTKVDKKVEPKSCDKTHTCPPCPAPELLGGPSVFLFPPKPKD TLMISRTPEVTCVVVDVSHEDPEVKFNWYVDGVEVHNAKTKPREEQYNSTY RVVSVLTVLHQDWLNGKEYKCKVSNKALPAPIEKTISKAKGQPREPQVYTL PPSREEMTKNQVSLSCAVKGFYPSDIAVEWESNGQPENNYKTTPPVLDSDG SFFLVSKLTVDKSRWQQGNVFSCSVMHEALHNHYTQKSLSLSPGK | 64 |

### Example 2. Bispecific antibody production

Vector plasmids containing coding genes for the heavy and light-chain regions of the first arm and vector plasmids containing coding genes for the heavy and light-chain regions of the second arm were produced. Genes encoding two heavy and light chains were inserted into one vector plasmid. CMV was used as the promoter and WPRE (woodchuck hepatitis virus post-transcriptional regulatory element) was inserted after the coding gene to increase the expression level during transient expression.

ExpiCHO-S animal cells (Thermo Fisher A29127) were co-transfected with vector plasmids containing the coding genes of the heavy- and light-chain regions of the first arm and vector plasmids containing the coding genes of the heavy- and light-chain regions of the second arm. The co-transfection was performed using ExpiFectamine CHO Transfection Kit (Thermo Fisher A29130) according to the corresponding manual. Two weeks after co-transfection, the supernatant was obtained by centrifugation (10000 xg, 15 mins) to obtain the bispecific antibody, and the supernatant was filtered (0.22 um) to remove residual cell debris. FIG. 18 illustrates the expression pattern of the supernatant through SDS-PAGE under non-reducing conditions.

### Example 3. Bispecific antibody expression

Octet quantitative assay based on a Protein A biosensor (Fortebio 18-5010) was used to assay bispecific antibodies. A calibration curve was obtained using a known IgG1 type sample as a standard material and the expression level of the sample was calculated through the calibration curve.

**[Table 26] Concentration of antibody in culture supernatant quantified by octet assay**

| **Sample ID** | **Concentration (ug/ml)** |
|---|---|
| Q-SBL1 | 471.5 |
| Q-SBL2 | 415.2 |
| Q-SBL3 | 354.9 |
| Q-SBL4 | 343.8 |
| R-SBL1 | 337.8 |
| R-SBL2 | 338.0 |
| R-SBL3 | 211.8 |
| R-SBL4 | 223.4 |
| Q-SBL5 | 493.1 |
| R-SBL5 | 407.1 |
| R-SBL6 | 351.4 |
| Q-SBL6 | 272.7 |
| Q-SBL7 | 438.8 |
| Q-SBL8 | 284.6 |
| Q-SBL9 | 322.0 |

### Example 4. Bispecific antibody purification

A purified bispecific antibody having a purity of greater than 95% was isolated using AKTA avant 25/150 (Cytiva) as a protein purification system by protein A affinity chromatography, ion exchange chromatography (IEX), and hydrophobic interaction chromatography (HIC) or the like to perform various research and analysis of the bispecific antibody of the present invention. The bispecific antibody material obtained in each purification step was identified by SDS-PAGE using an 8% bis-Tris gel and MES buffer. The purity of the bispecific antibody was also measured using size exclusion high-performance liquid chromatography (SEC-HPLC) using a TSKgel G3000SWxl (Tosoh Bioscience) column.

The culture solution obtained by expressing the bispecific antibody candidates of the present invention was filtered using a 0.22 um filter paper and then primarily purified using a MabSelect SuRe (Cytiva) column, which is a type of protein A affinity chromatography.

The primary purification was specifically performed as follows. The MabSelect Sure column was equilibrated with a 50 mM Tris (pH 7.0) buffer solution and then the filtered culture solution was loaded onto the column. Proteins not bound to the column were washed with the equilibration buffer solution for 5 cv. Then, impurities non-specifically bound to the MabSelect Sure column were removed with a 50 mM Tris (pH 7.0) buffer solution and a 20 mM Bis-Tris (pH 5.5) buffer solution containing 0.5 M sodium chloride. Then, the bispecific antibody specifically binding to the MabSelect Sure column was eluted using a 0.2 M glycine (pH 3.2) buffer solution for 4 cv. The eluted bispecific antibody sample was neutralized to pH 5.0 with a 1.0 M Tris buffer solution and then filtered through a 0.22 um filter paper.

The next purification was performed using a Capto SP (Cytiva) column based on cation exchange chromatography in ion exchange chromatography, and the details are as follows. The Capto SP column was stabilized with a 50 mM sodium acetate (pH 5.0) buffer solution, a bispecific antibody sample neutralized to pH 5.0 was applied thereto, and impurities not bound to the column were removed with the same buffer solution. The bispecific antibody bound to the column was eluted with 0.1 M to 1.0 M sodium chloride.

In the final purification process, hydrophobic interaction chromatography was performed to remove high molecular weight (HMW) and low molecular weight (LMW) impurities from the secondarily purified bispecific antibody sample. In this process, a butyl-based Sepharose column was used and samples were prepared by substituting the bispecific antibody purified product obtained after ion exchange chromatography with a high-concentration salt buffer solution such that the salt concentration in the bispecific antibody was 1.0 M to 1.5 M. The column was equilibrated with a 50 mM sodium acetate (pH 5.0) buffer solution having the same salt concentration as the applied sample and then the prepared sample was loaded thereon. The bound bispecific antibody was eluted for 20 cv with a gradient method of 50 mM sodium acetate (pH 5.0) having no salt. The final purified bispecific antibody eluted with a purity of 95% or higher was concentrated to a concentration of 1 to 2 mg/mL using a 10 kDa molecular-weight cut-off ultrafiltration tube and then used in an appropriate buffer solution depending on analysis conditions.

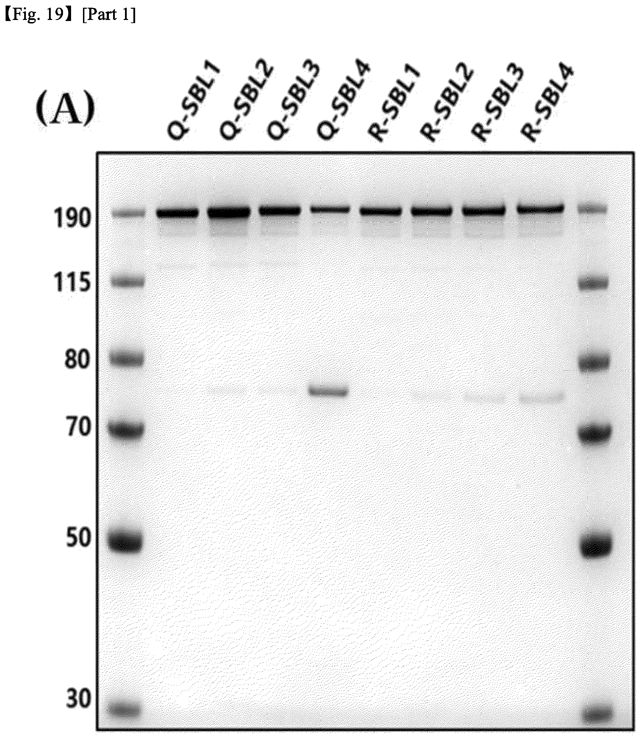

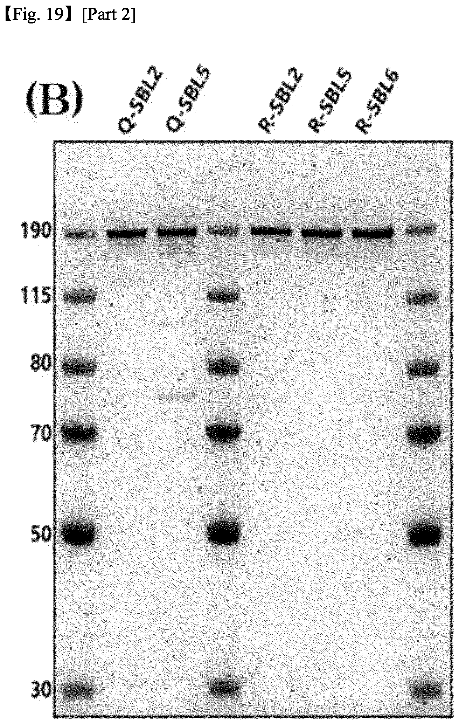

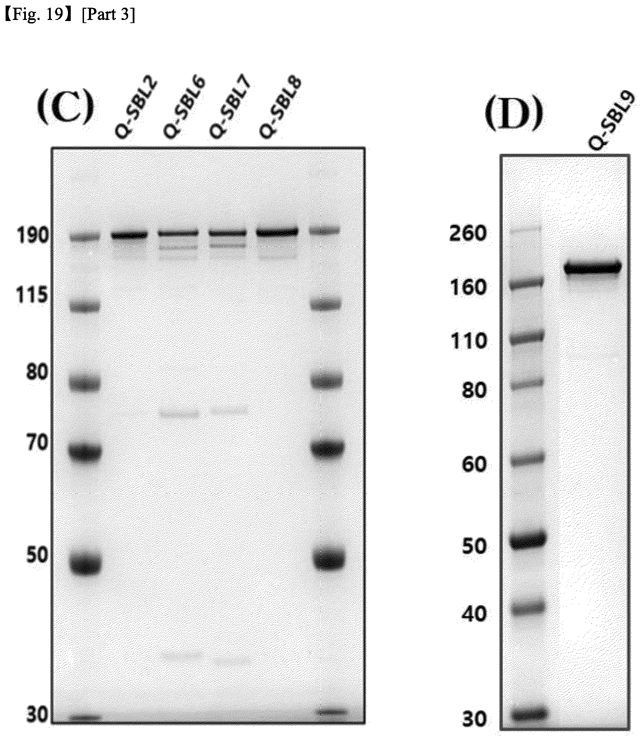

FIG. 19 is a schematic diagram of an SDS-PAGE gel showing the protein content obtained by tertiary purification of the bispecific antibody candidates of the present invention using hydrophobic interaction chromatography.

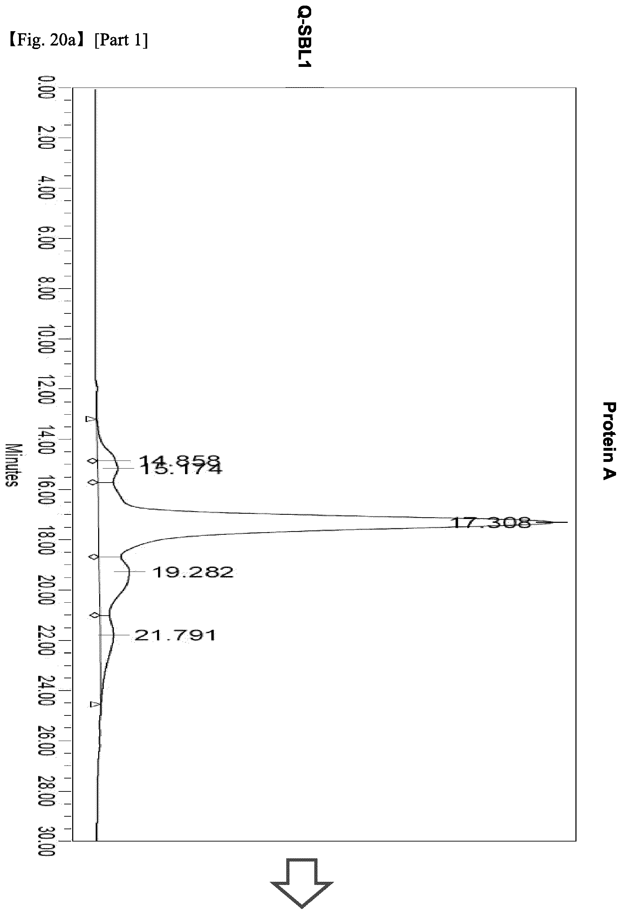

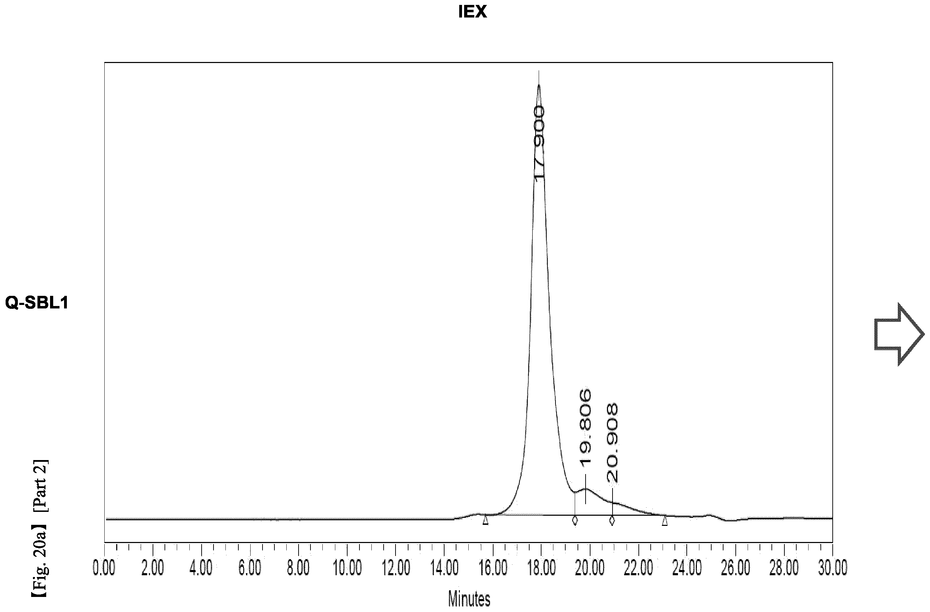

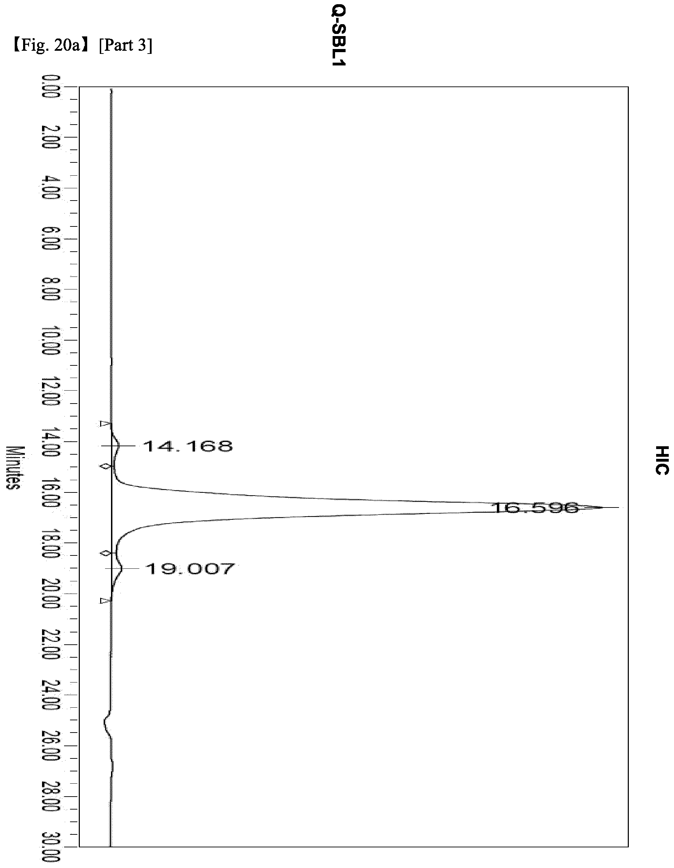

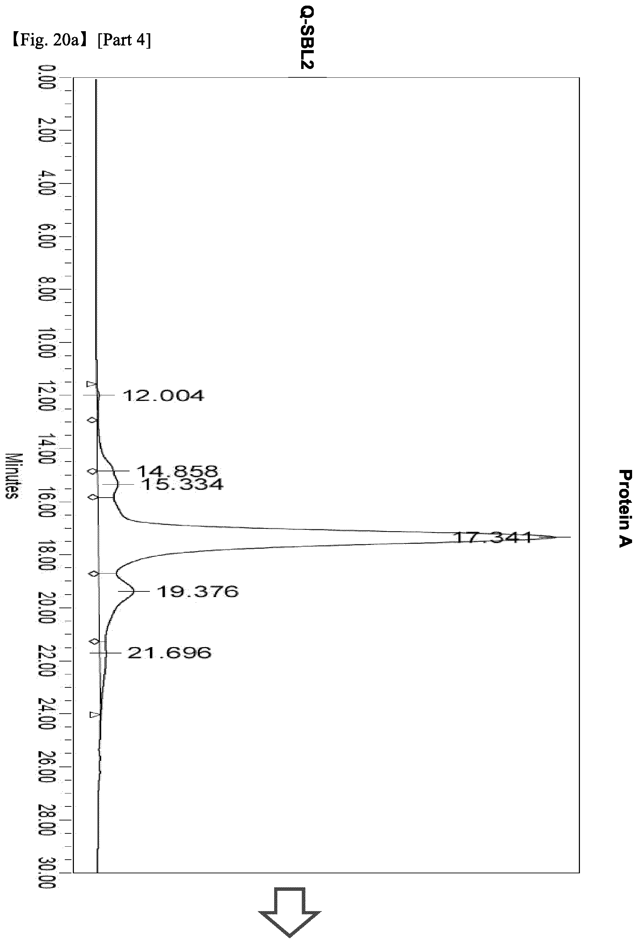

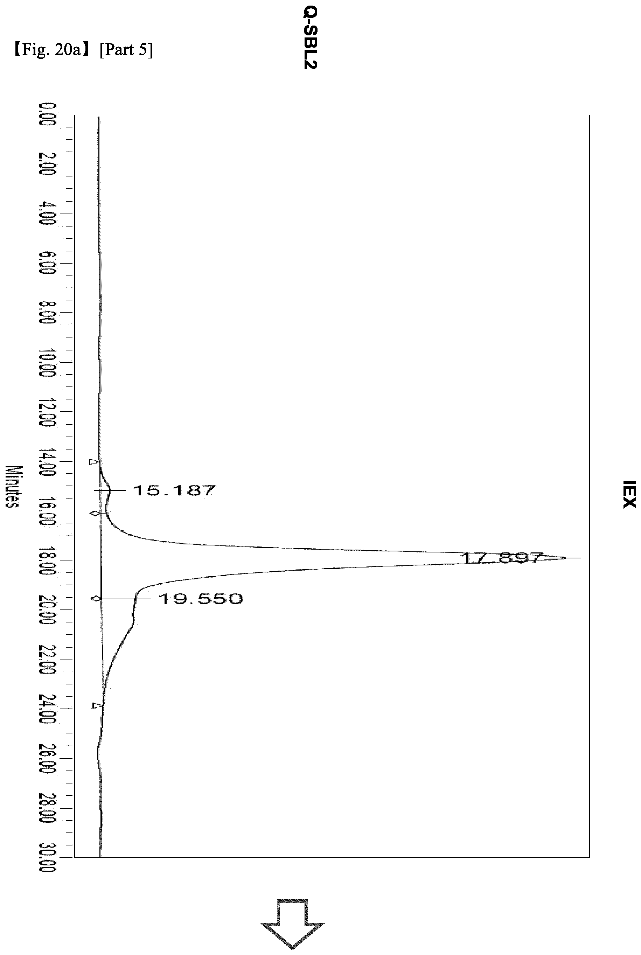

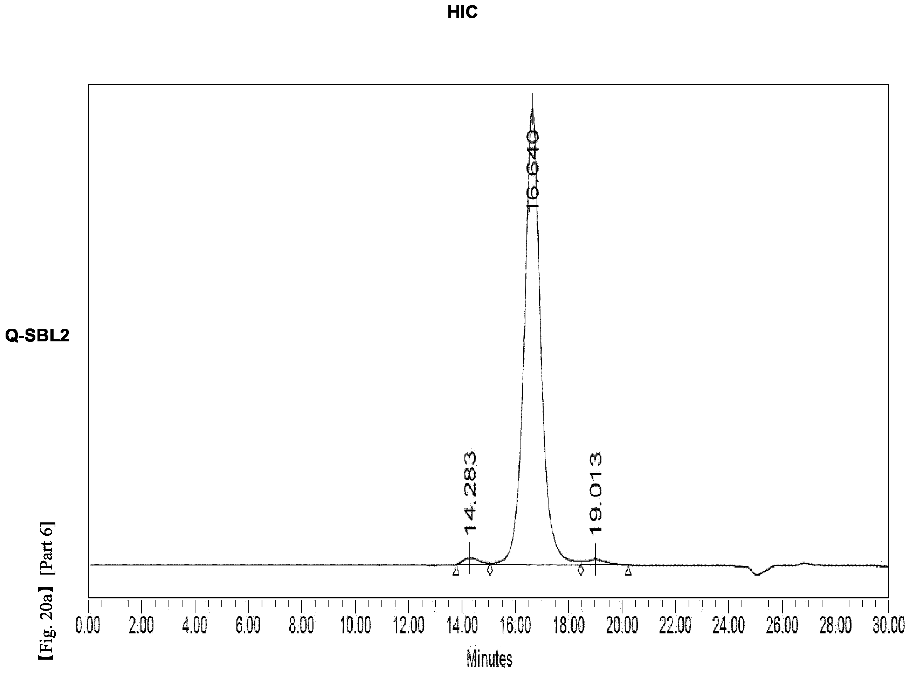

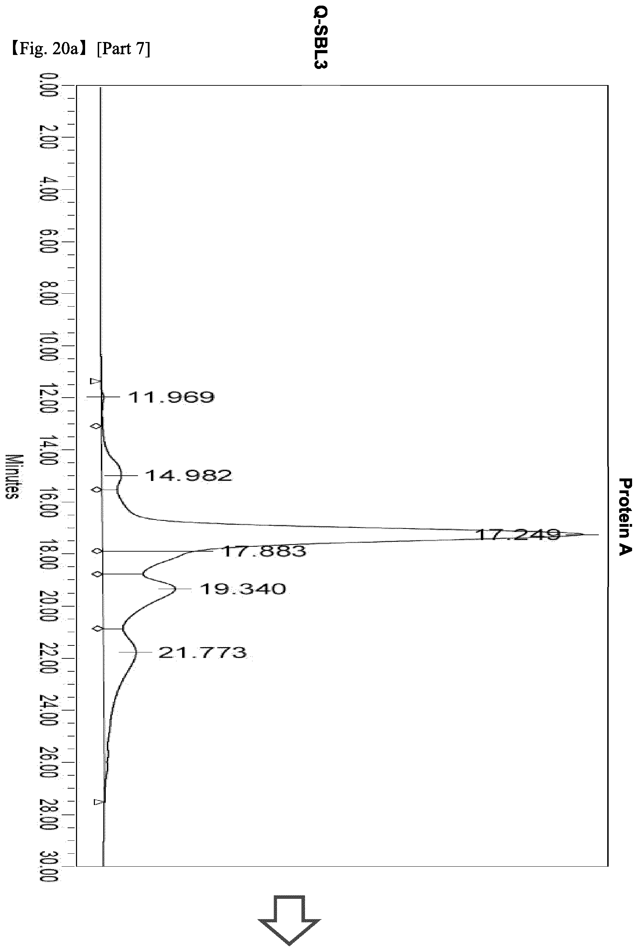

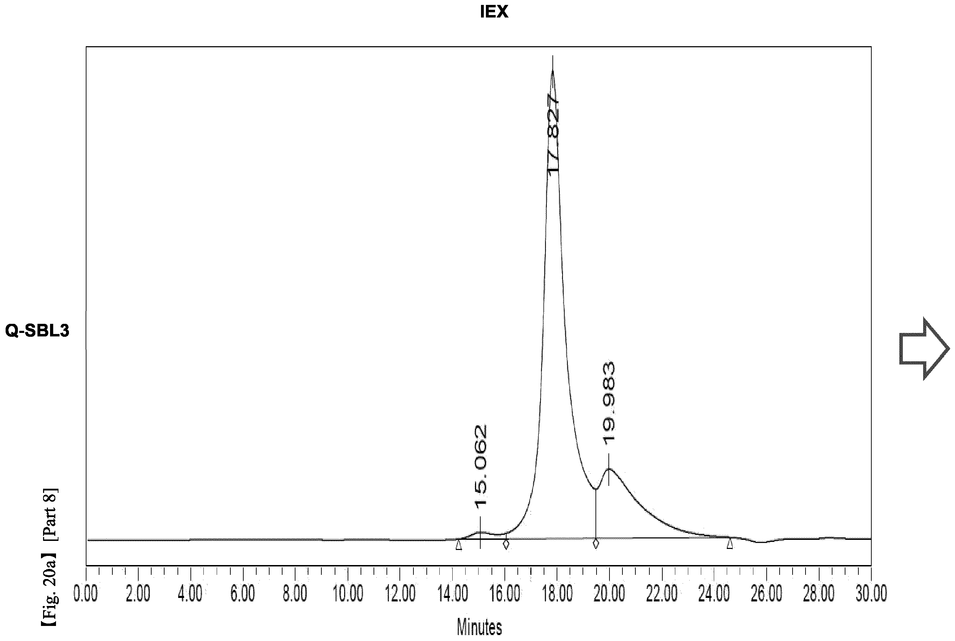

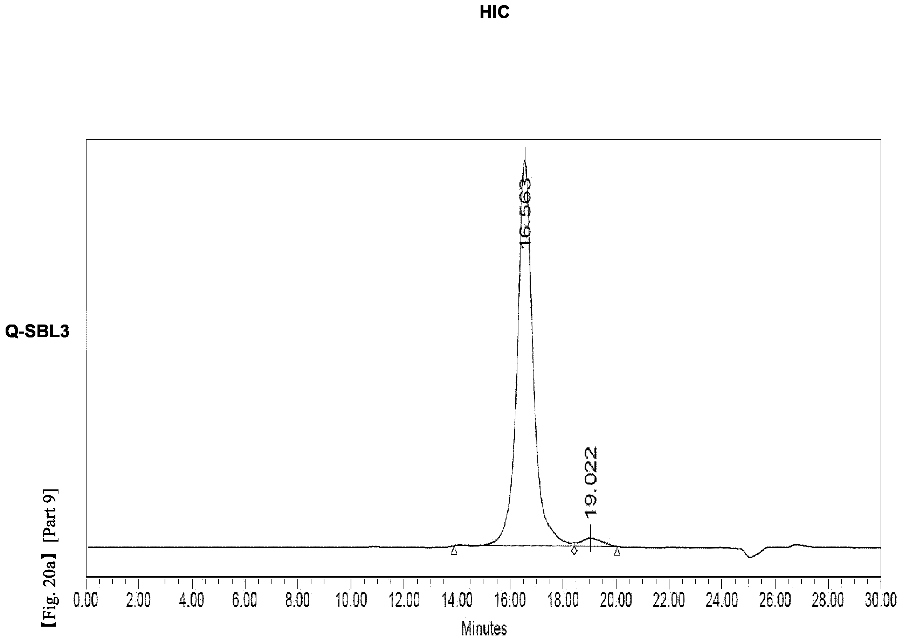

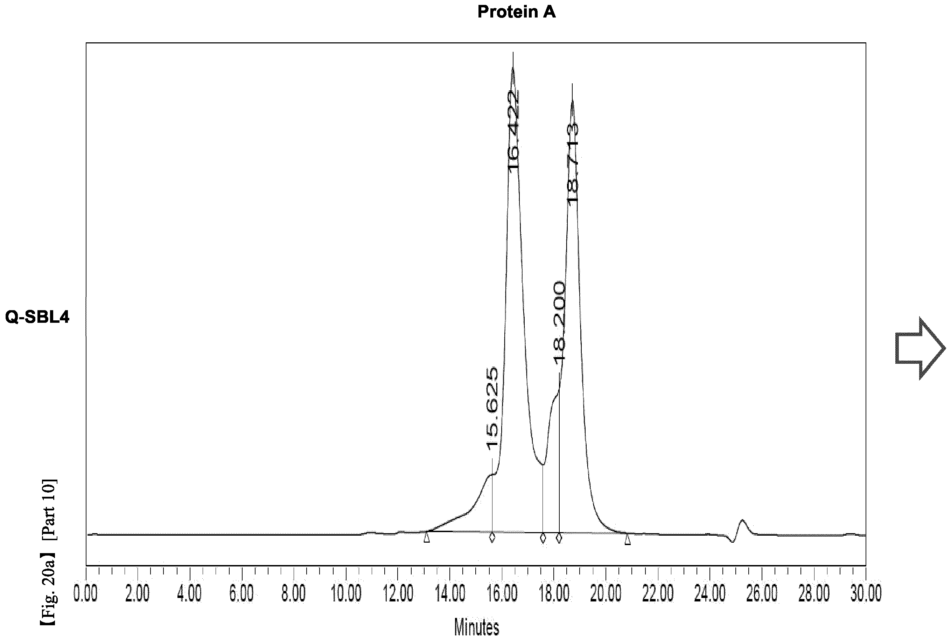

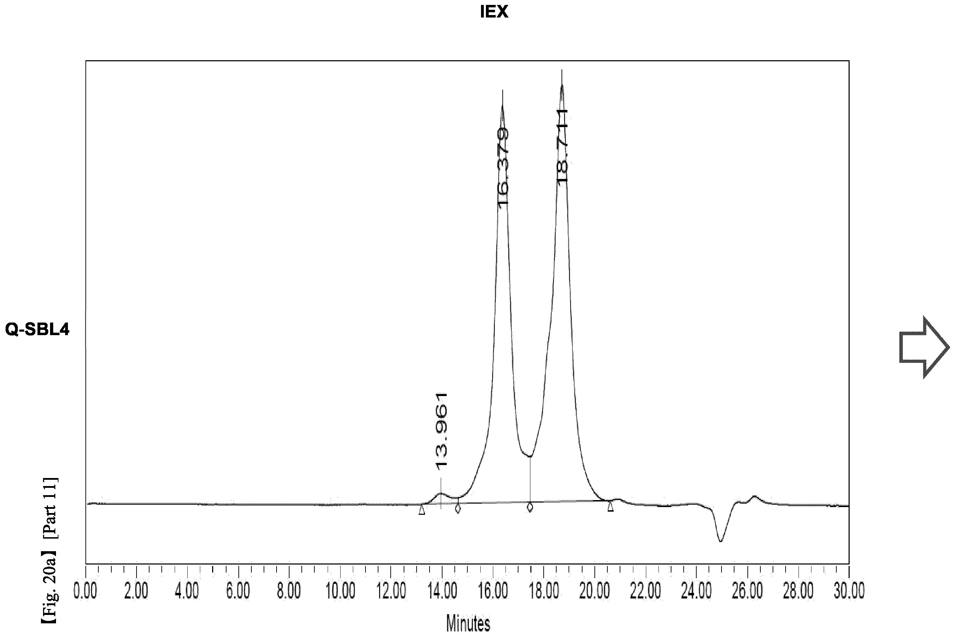

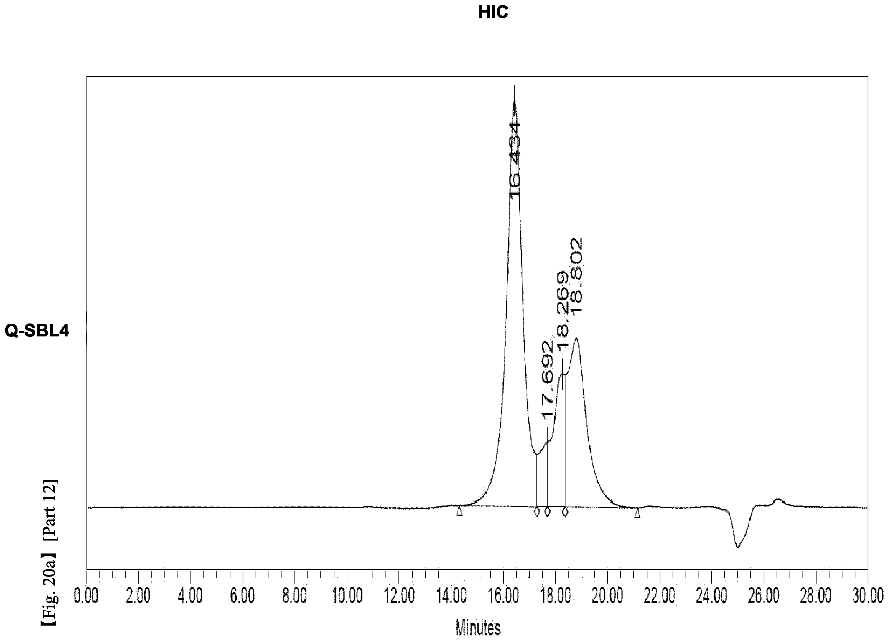

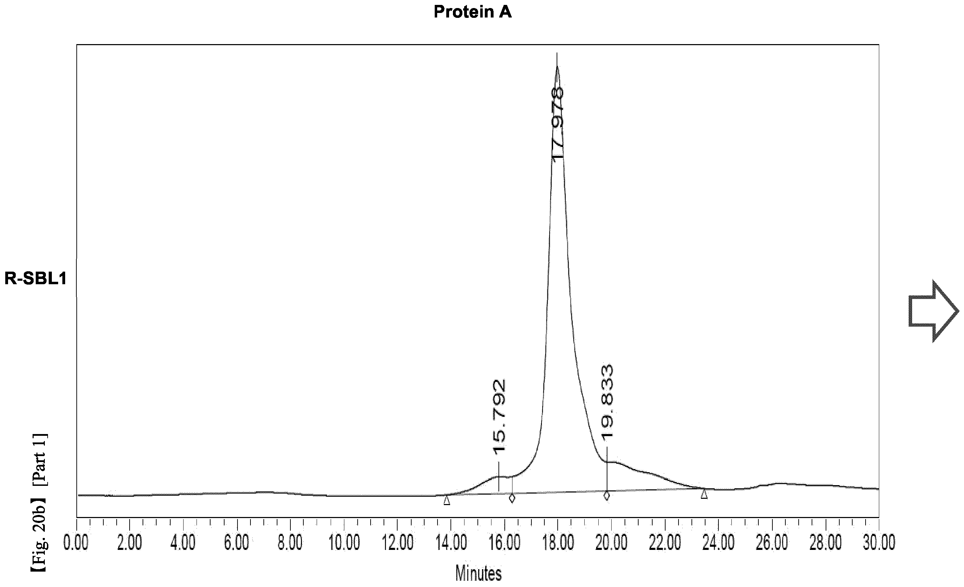

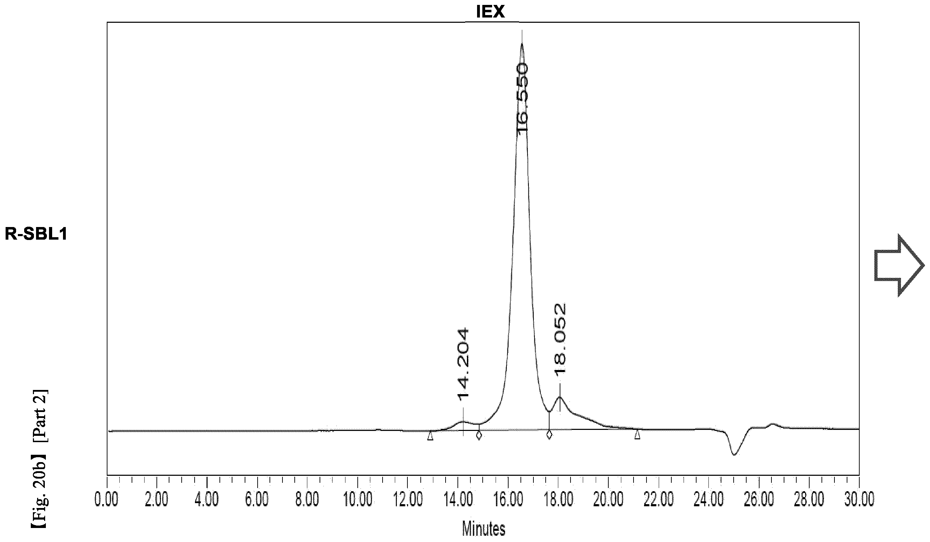

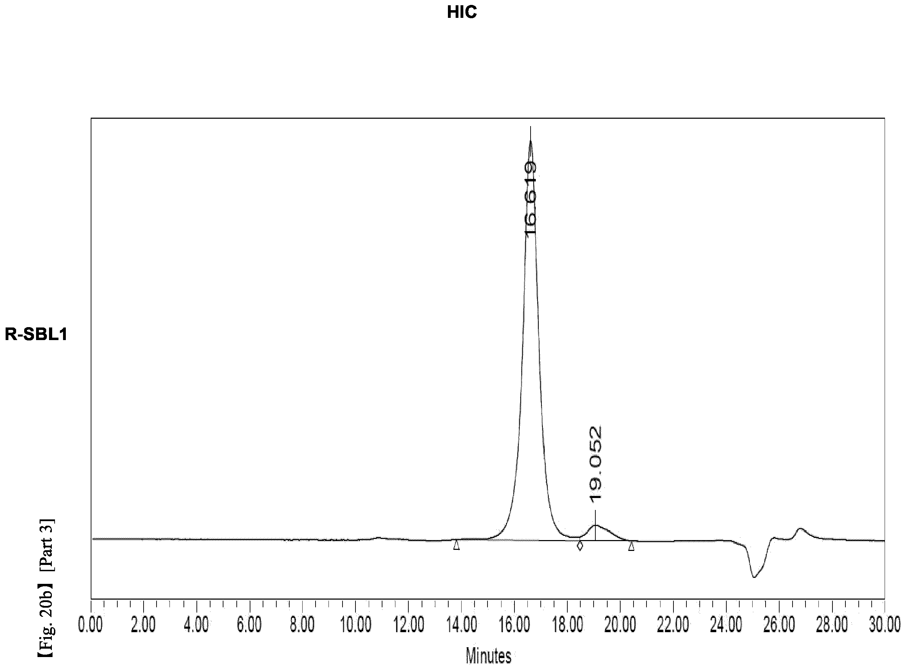

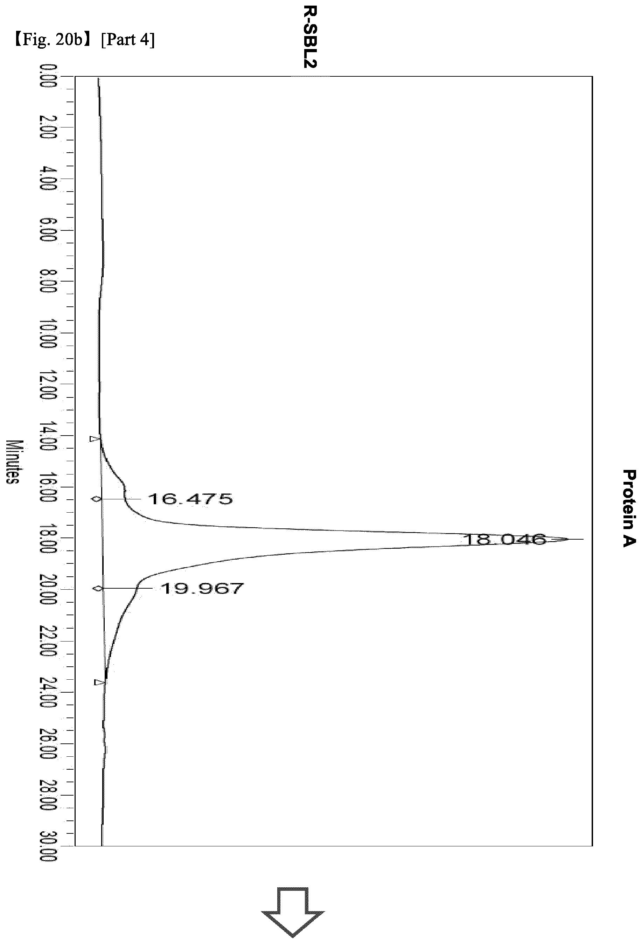

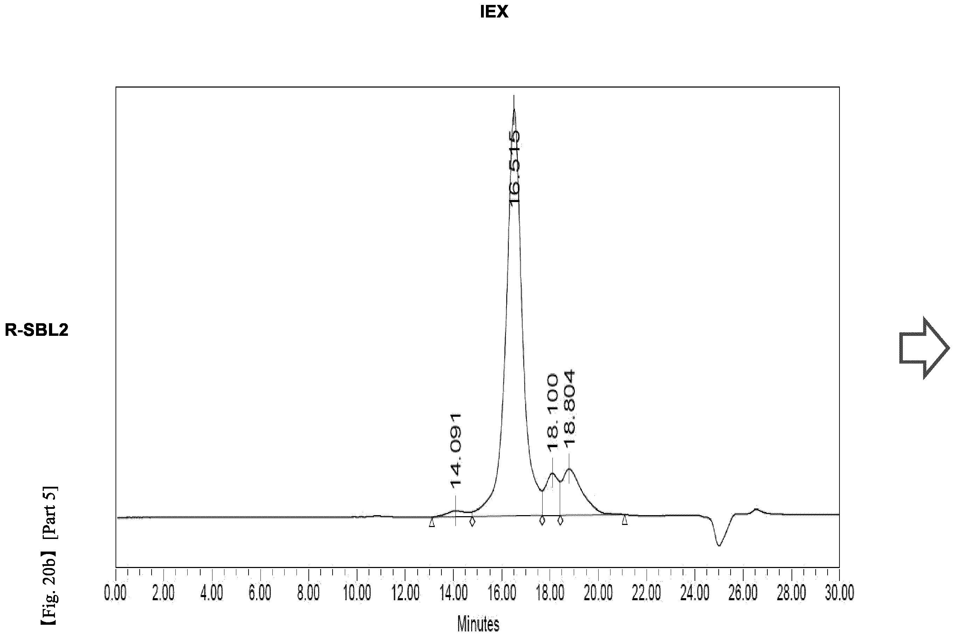

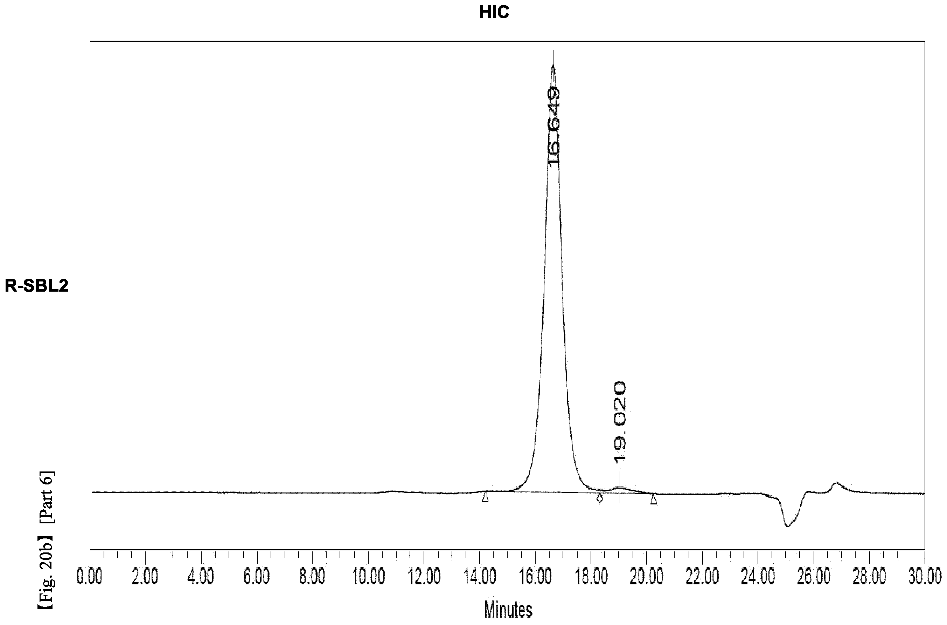

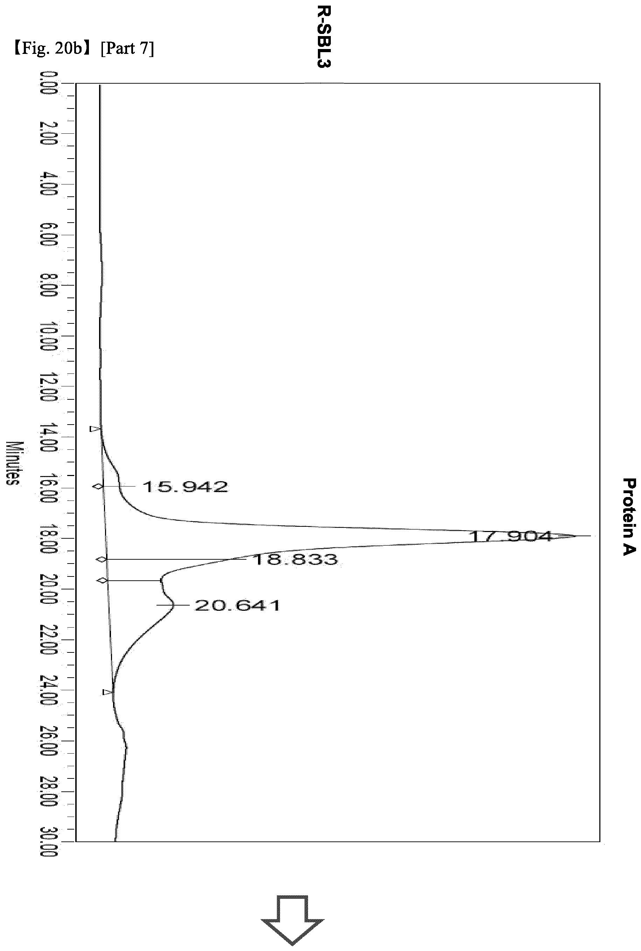

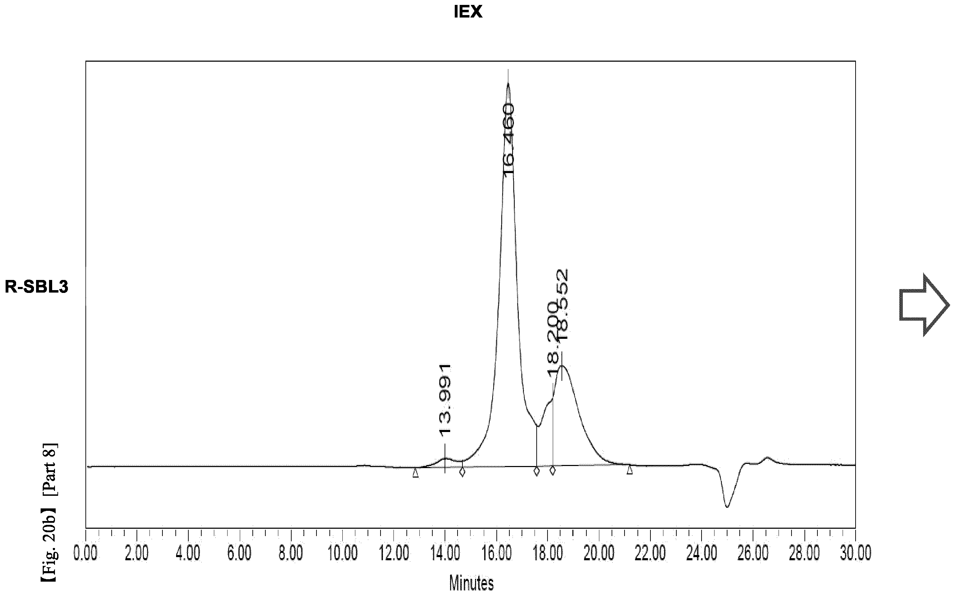

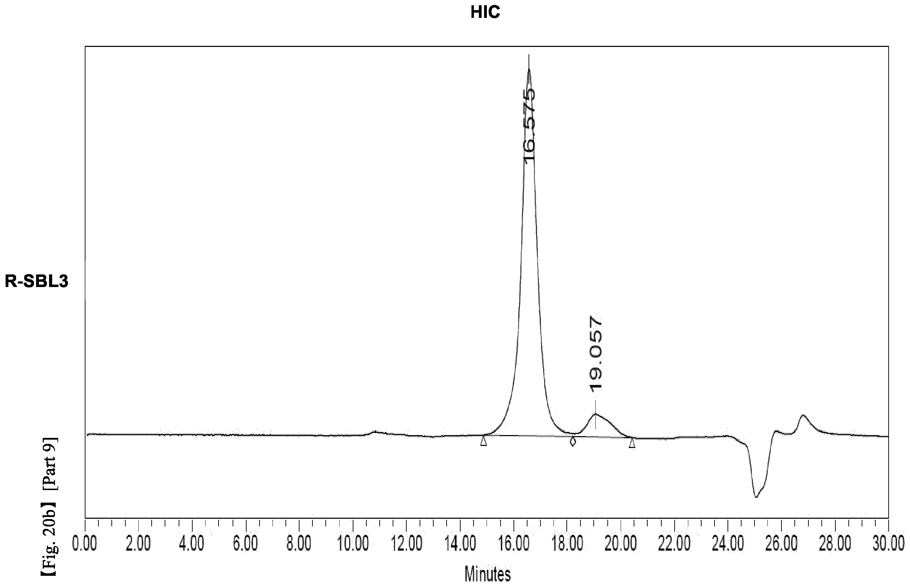

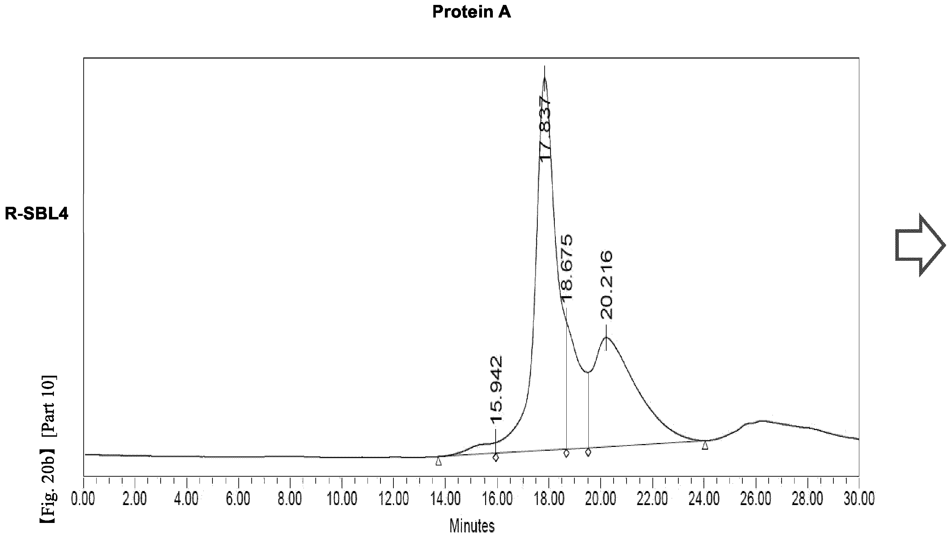

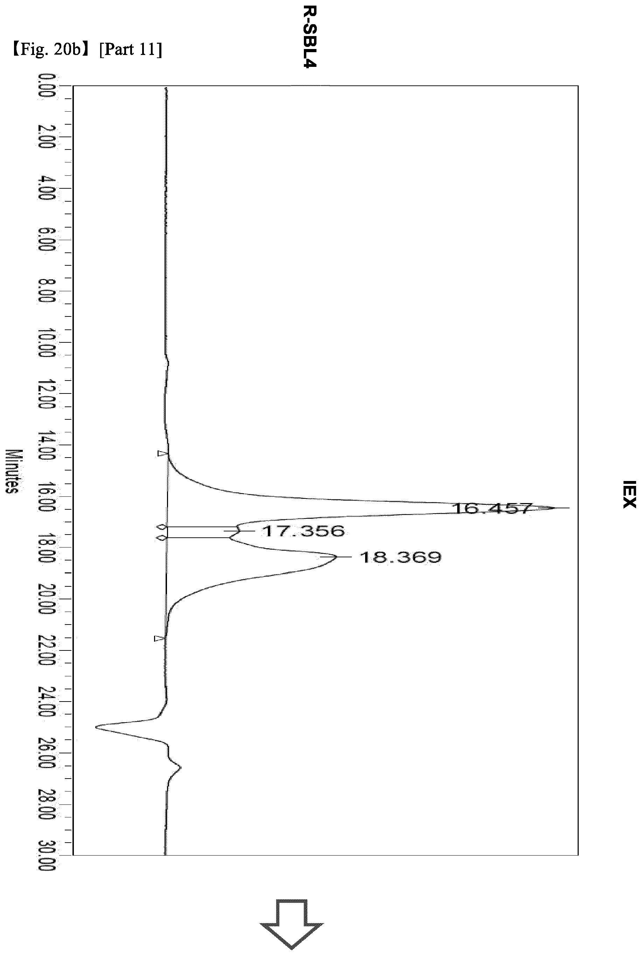

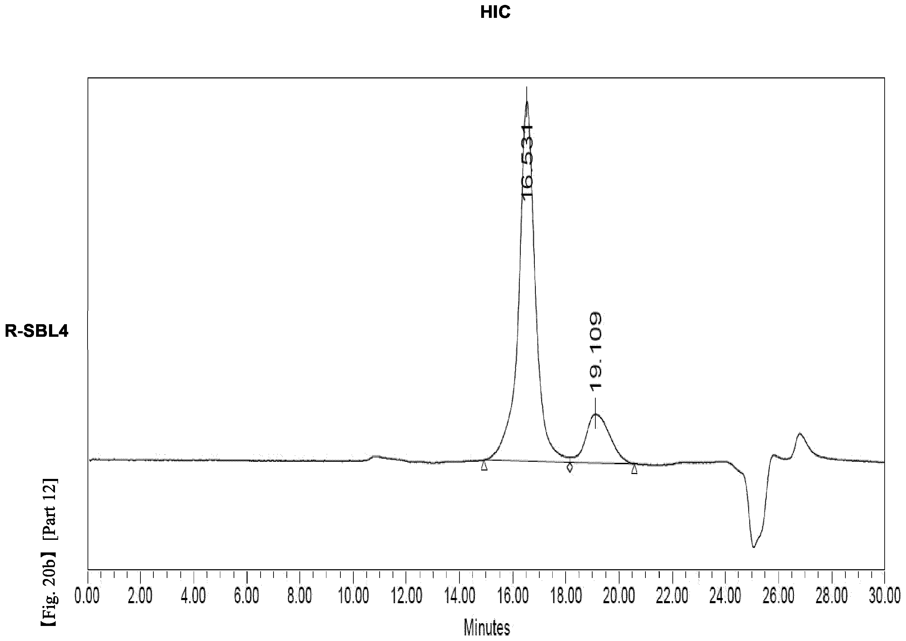

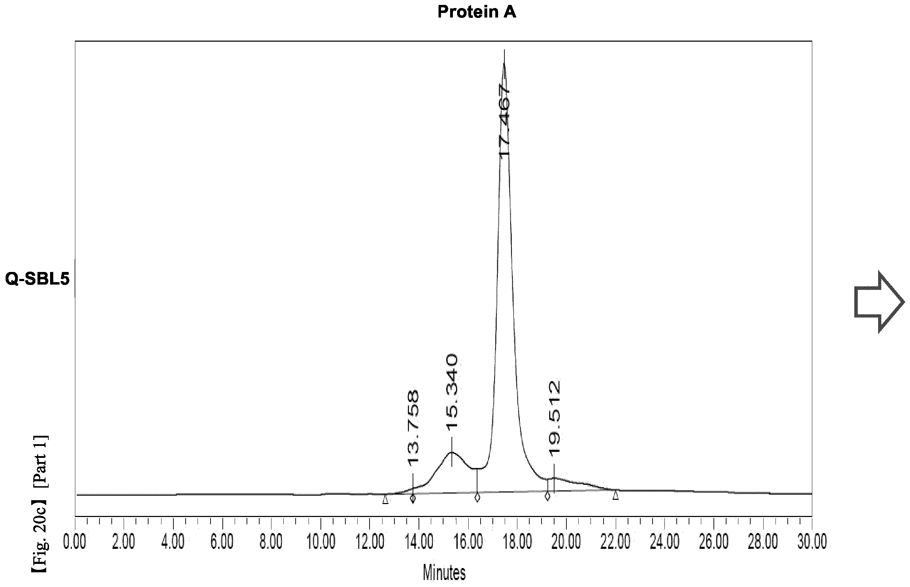

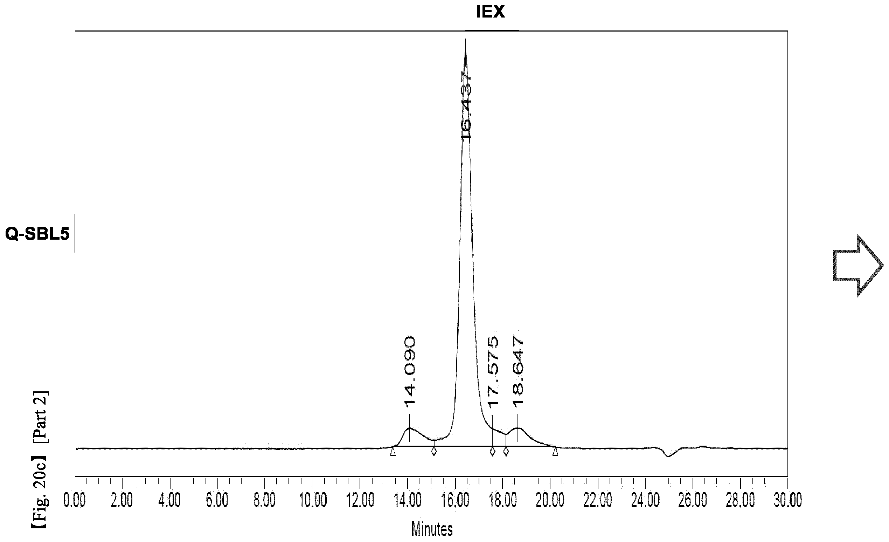

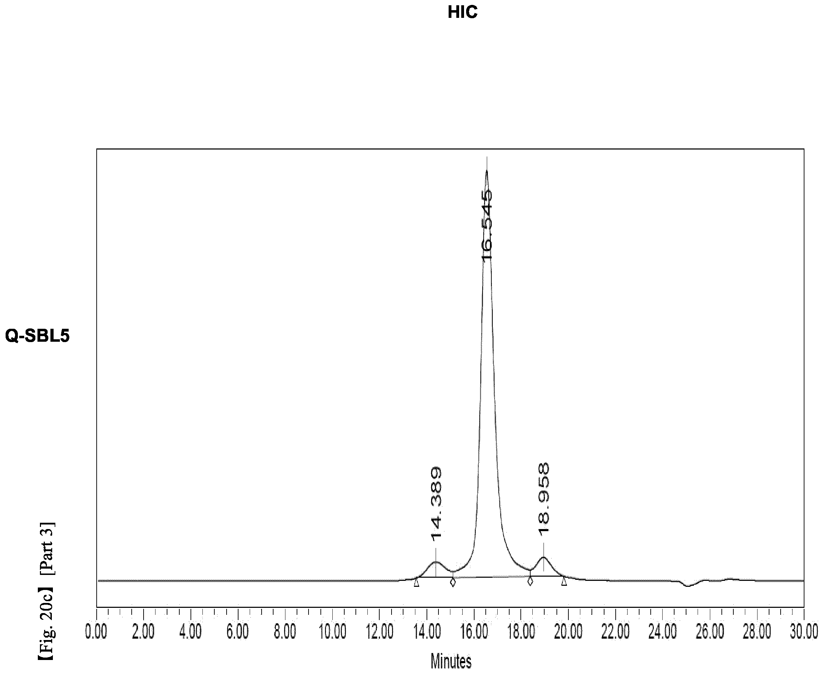

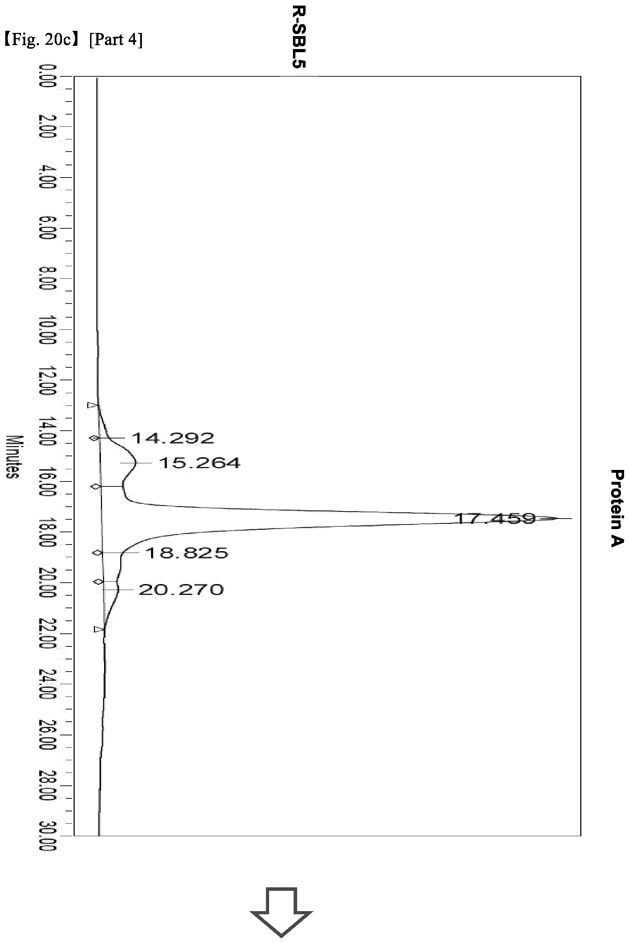

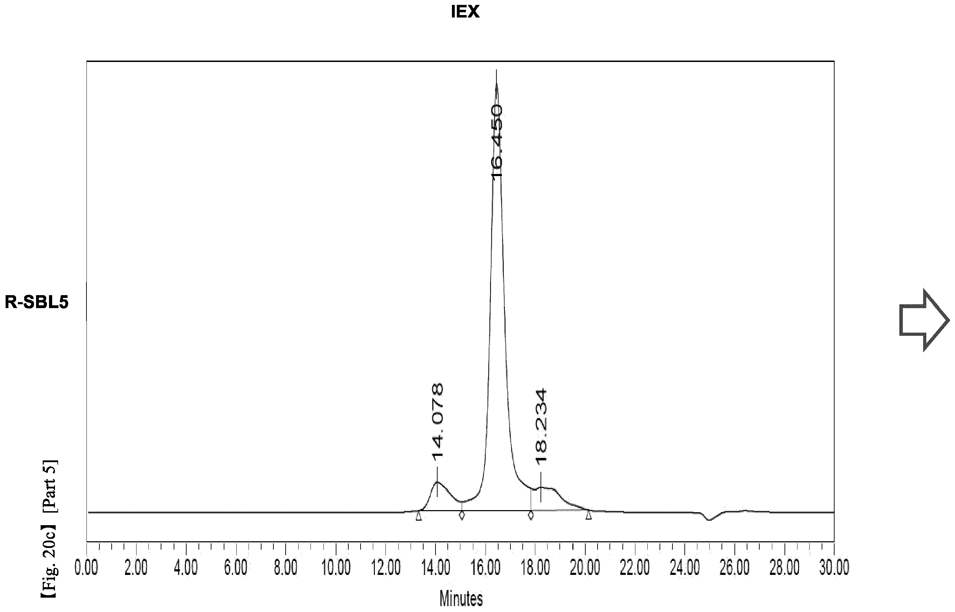

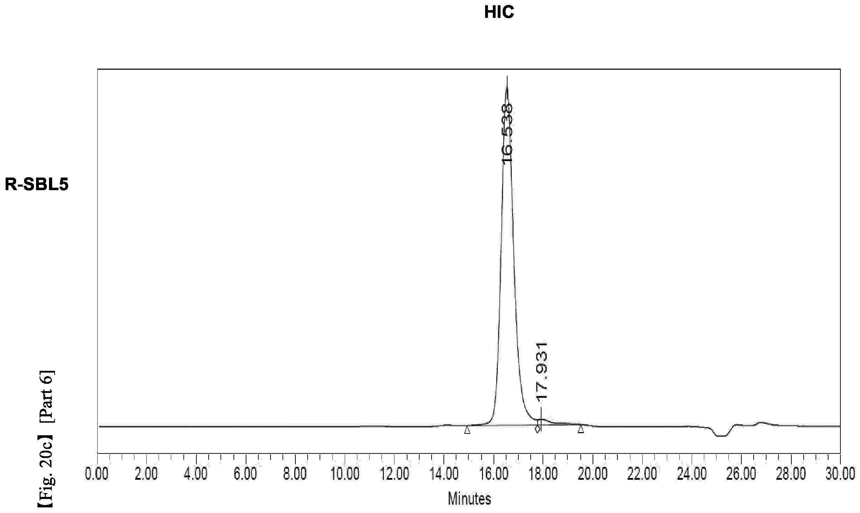

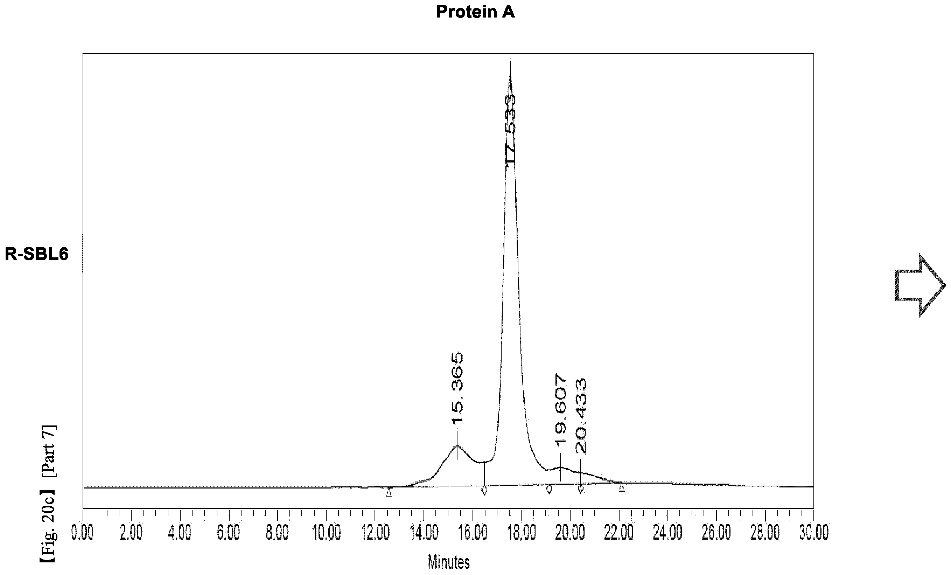

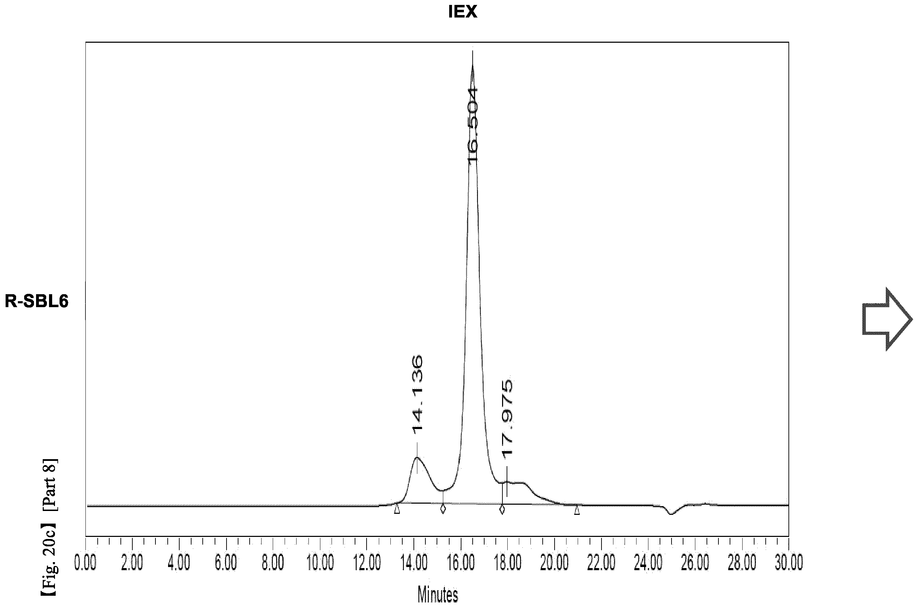

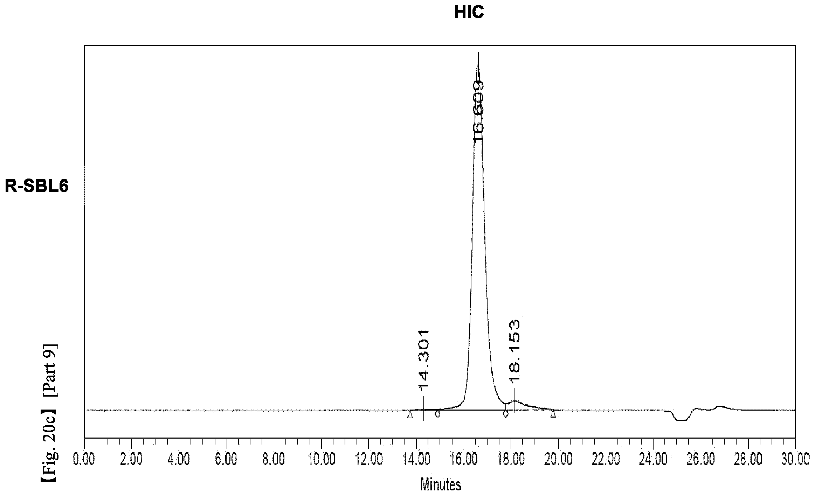

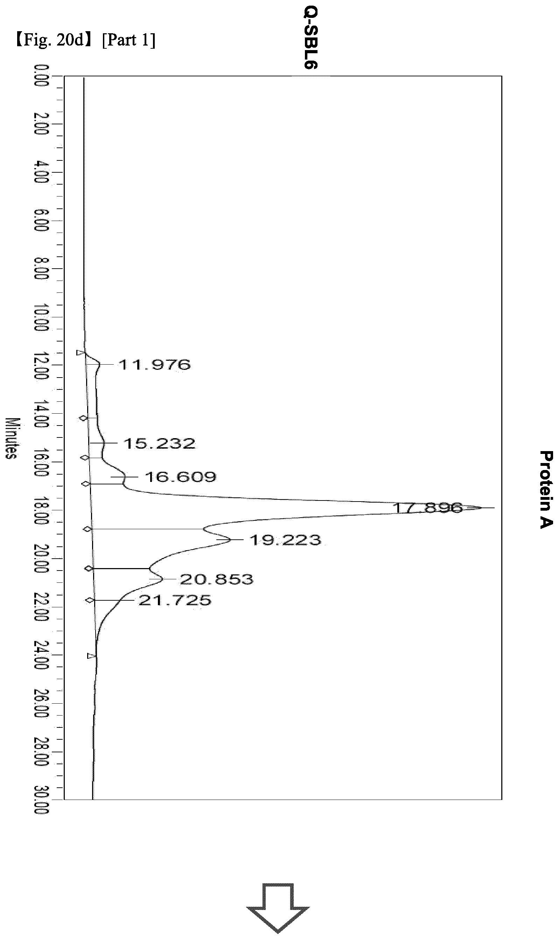

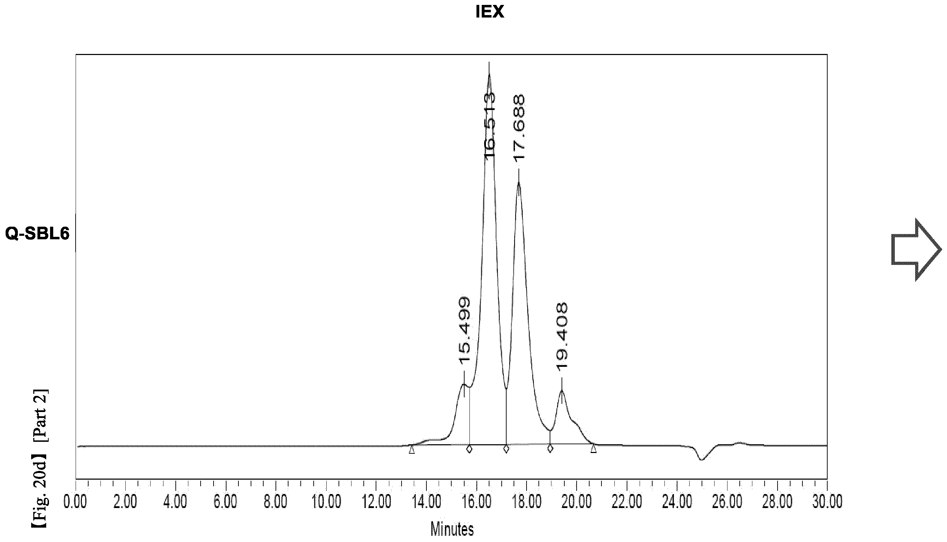

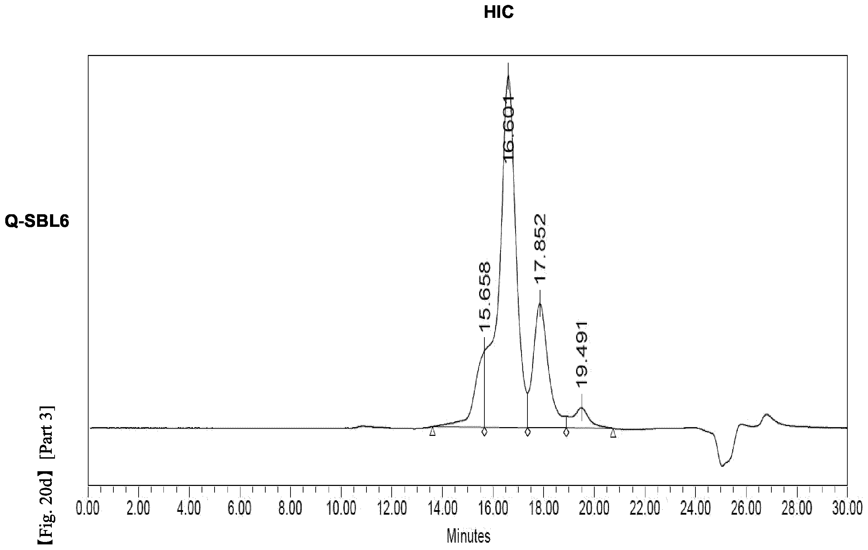

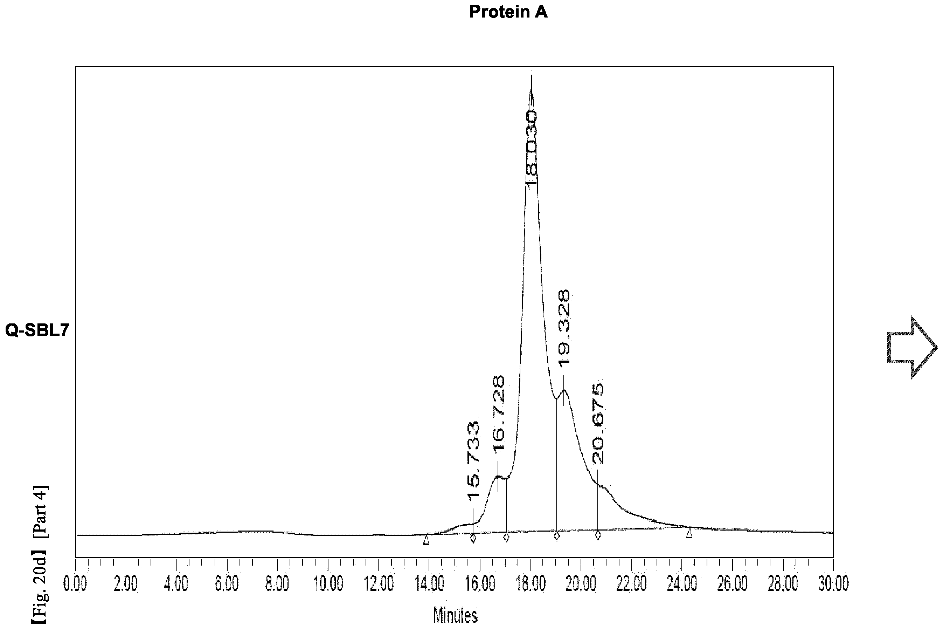

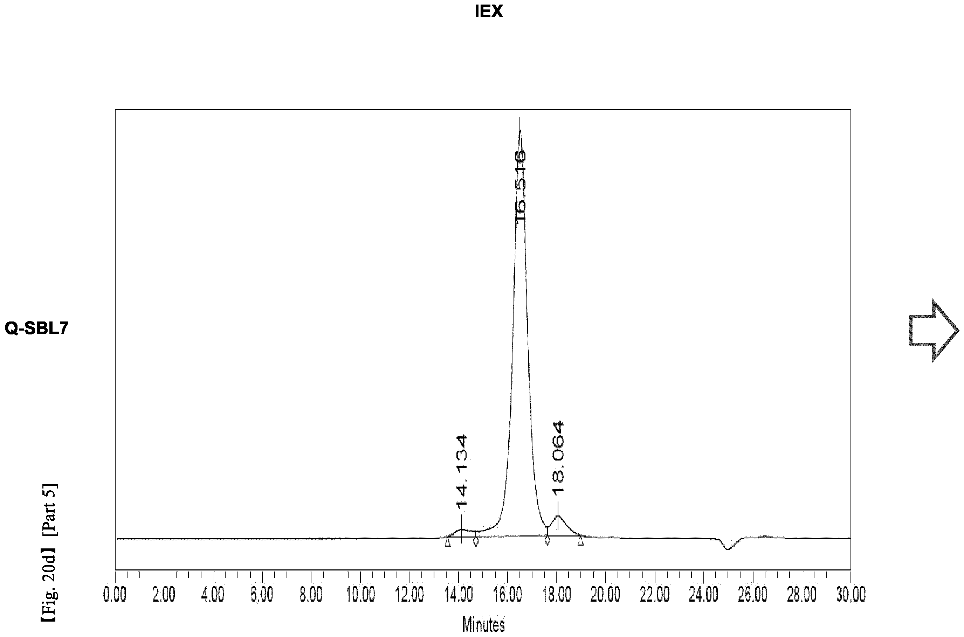

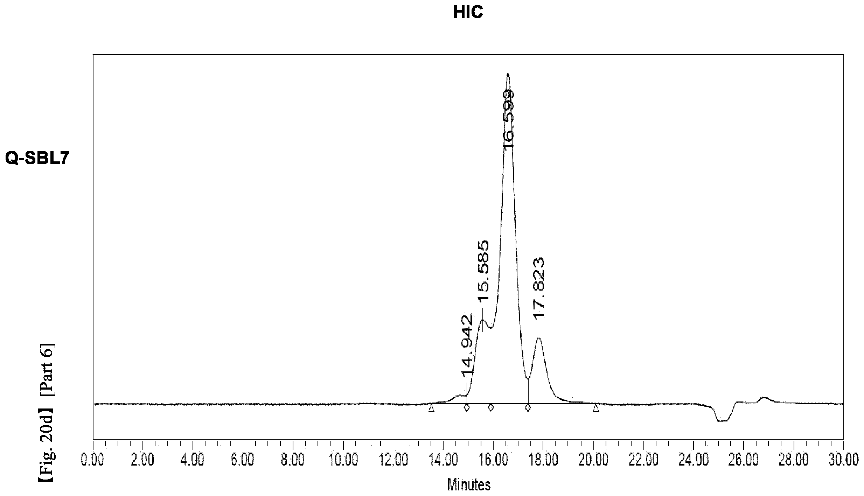

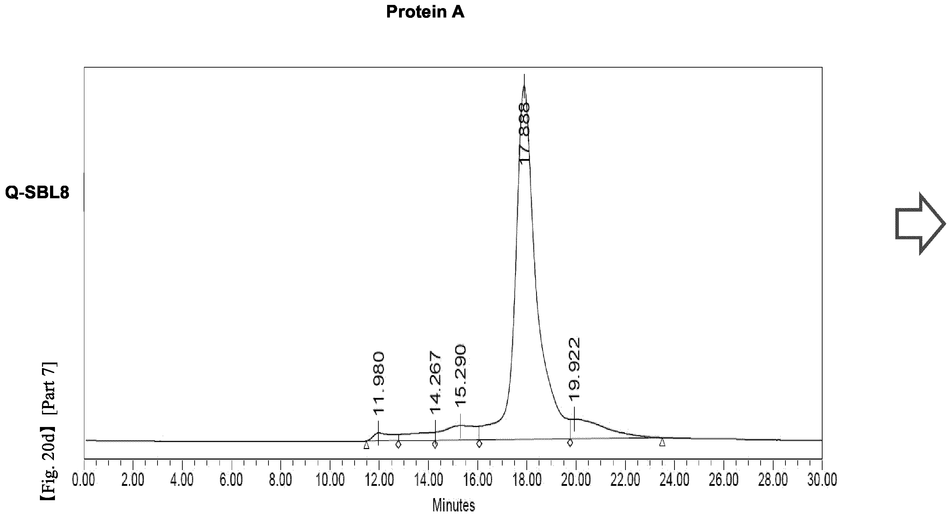

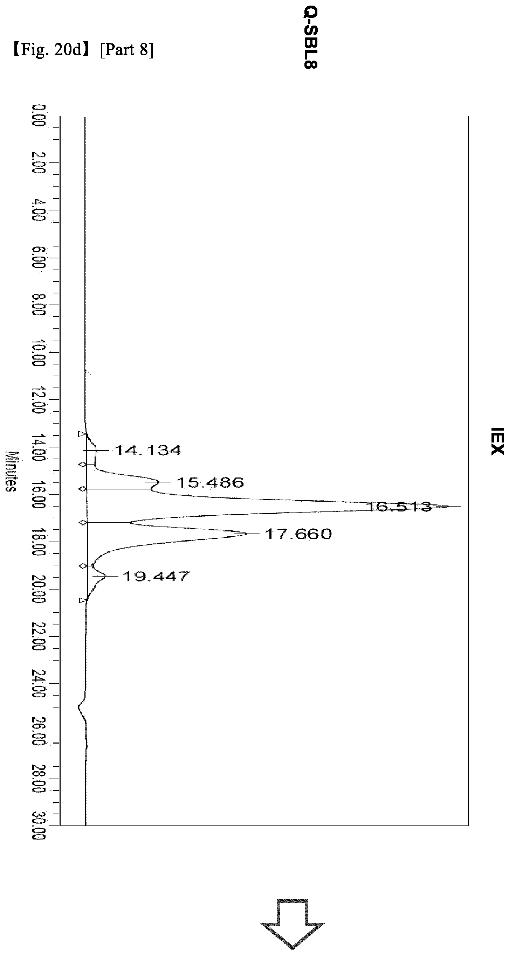

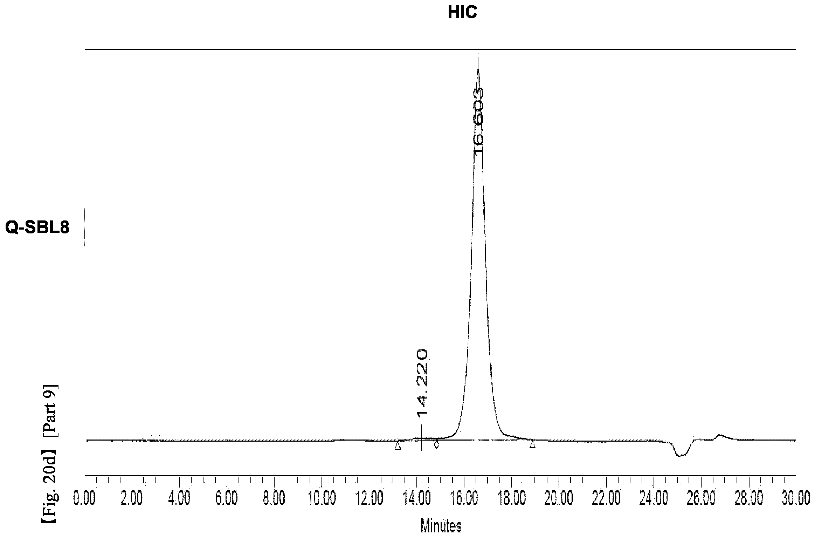

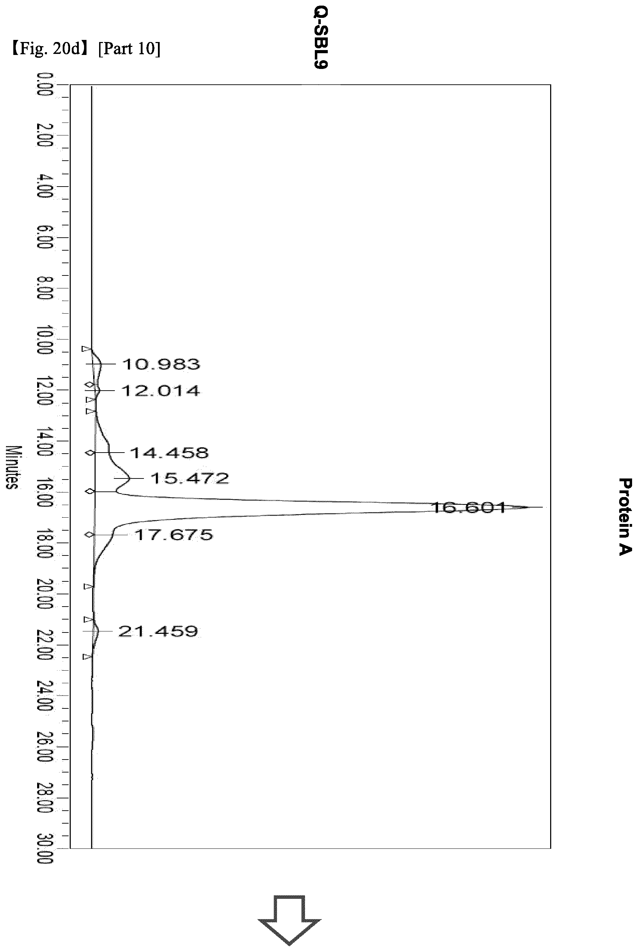

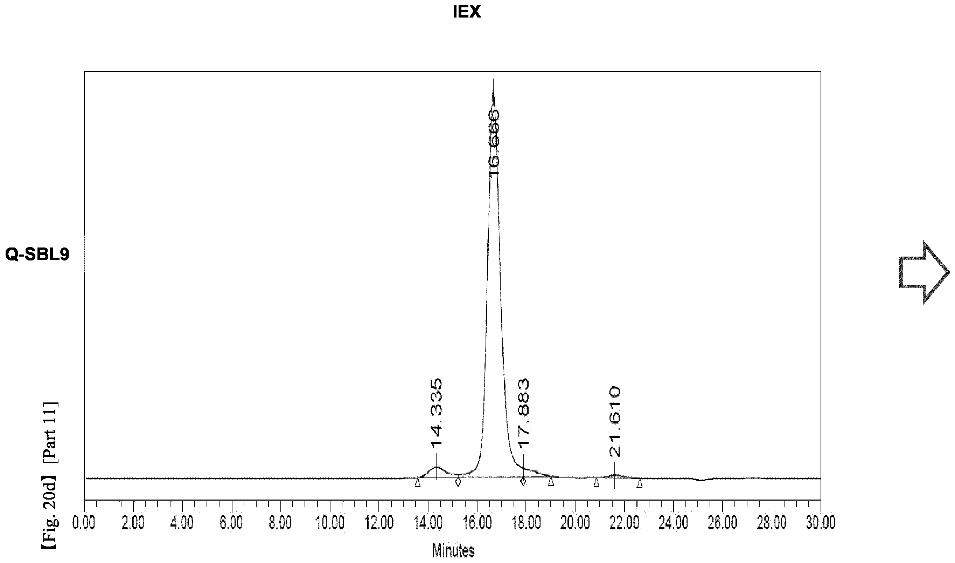

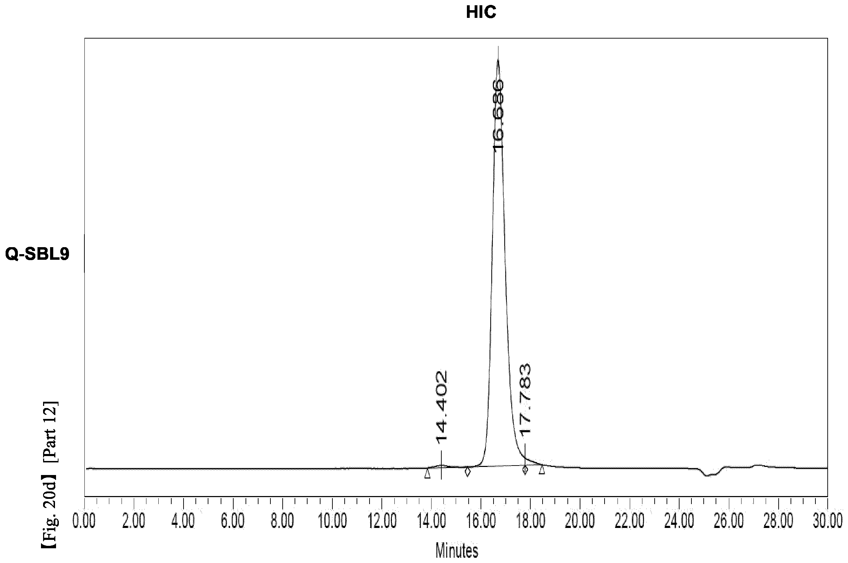

FIGS. 20A, 20B, 20C, and 20D are chromatograms of the bispecific antibody candidates subjected to the final purification of the present invention analyzed using size exclusion high-performance liquid chromatography (SEC-HPLC).

**[Table 27] Analysis of purity of bispecific antibody after rPA purification and three-step purification by size exclusion high performance liquid chromatography (SEC-HPLC)**

| Sample ID | SEC analysis (After 1-step purification) | SEC analysis (After 3-step purification) |
|---|---|---|
| Q-SBL1 | 85 | 96 |
| Q-SBL2 | 80 | 97 |
| Q-SBL3 | 78 | 97 |
| Q-SBL4 | 45 | 58 |
| R-SBL1 | 86 | 95 |
| R-SBL2 | 87 | 98 |
| R-SBL3 | 67 | 92 |
| R-SBL4 | 54 | 84 |
| Q-SBL5 | 70 | 98 |
| R-SBL5 | 84 | 98 |
| R-SBL6 | 79 | 96 |
| Q-SBL6 | 53 | 68 |
| Q-SBL7 | 62 | 99 |
| Q-SBL8 | 83 | 94 |
| Q-SBL9 | 78 | 99 |

### Example 5. Bispecific antibody characterization

### 5-1. CE-SDS analysis

Maurice (Protein Simple) and Maurice CE-SDS PLUS Application Kit (Protein simple) were used for sodium dodecyl sulfate capillary electrophoresis (CE-SDS) to analyze the purity of bispecific antibody candidates under non-reducing or reducing conditions. In one analysis, 25 µL of protein was used at a maximum concentration of 2 mg/mL in the sample analysis after purification using protein A resin, and 25 µL of protein was used at a maximum protein concentration of 1 mg/mL in sample analysis after protein A resin/cation exchange chromatography (CEX)/hydrophobic binding chromatography (HIC) purification. For analysis, each sample was mixed with 2.5 µL of 250 mM iodoacetamide or 14.2 M 2-mercaptoethanol, 2 µL of an internal standard material and 25 µL of a sodium dodecyl sulfate sample buffer, followed by heating at 70°C for 10 minutes. After completion of the analysis, the data was analyzed using Compass for iCE software version 2.2.0 provided by the manufacturer.

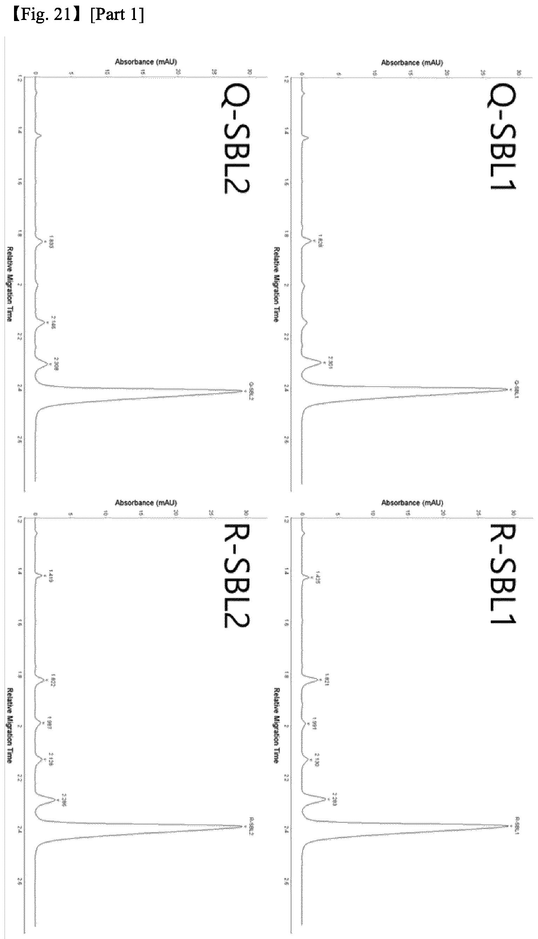

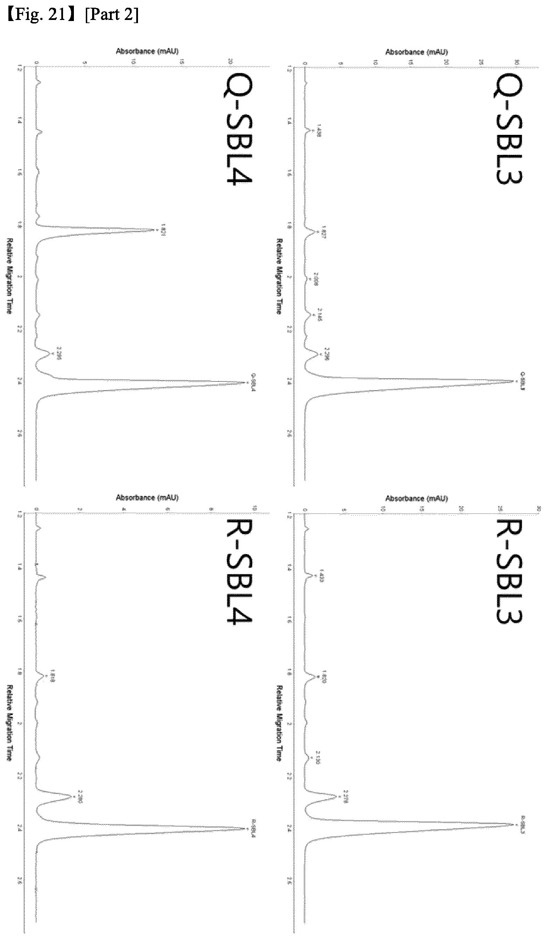

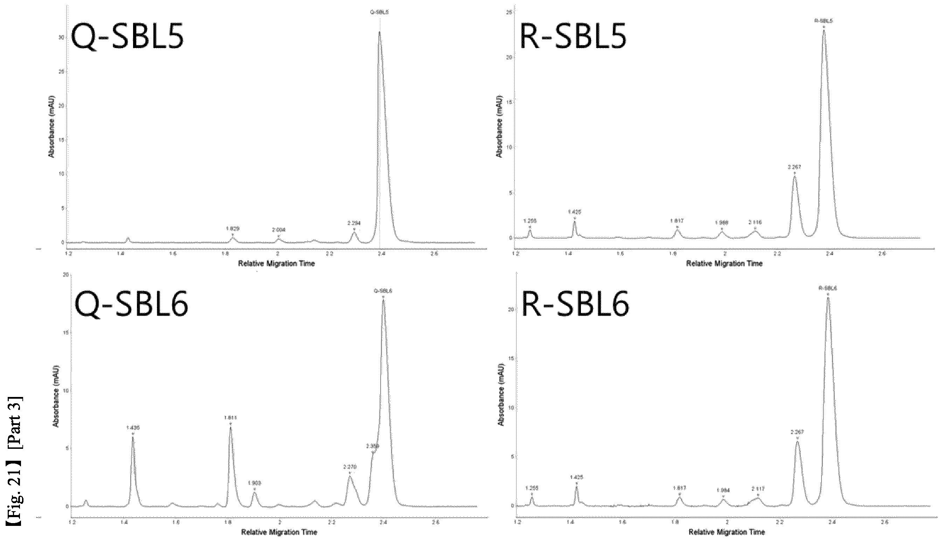

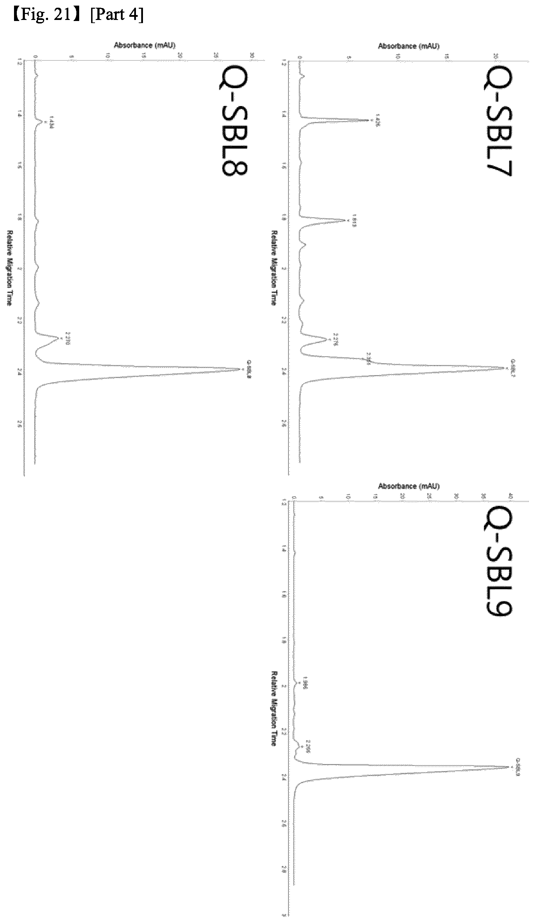

FIG. 21 shows data analyzed on CE-SDS under non-reducing conditions after 3-step (rPA+CEX+HIC) purification. After 3-step (rPA+CEX+HIC) purification of the bispecific antibody candidates, the purity was determined by sodium dodecyl capillary electrophoresis (CE-SDS). As a result, in the candidate group in which the linker length between the CH3 dimer and CH1/CL in the Fab region of the first arm was varied, Q-SBL4 or R-SBL4, a candidate, to which the linker of GGGGSGGGGSGGGGSGGGGS was applied, had the least purity. Second, the purity of all the candidates derived by introducing the IgG1 hinge (EPKSSDKTHTCPPCP) or the IgG4 hinge (ESKYGPPCPPCP) into the hinge region amino acid sequence of the first arm was not improved even after 3-step (rPA + CEX + HIC) purification. Finally, in the candidate group derived by exchanging the positions of the domains mutated to the knob or hole included in the CH3 domain, Q-SBL8 in which knob and hole domain positions were changed had the highest purity. Overall, Q-SBL2 and Q-SBL9 had high purity.

**[Table 28] Purity of bispecific antibodies after 1-step and 3-step purification under non-reducing conditions**

| Sample ID | CE-SDS (1-step purification, Non-reduced, %) | CE-SDS (3-step purification, Non-reduced, %) |
|---|---|---|
| Q-SBL1 | 57.4 | 86.7 |
| Q-SBL2 | 65.0 | 88.9 |
| Q-SBL3 | 53.0 | 88.3 |
| Q-SBL4 | 45.3 | 61.2 |
| R-SBL1 | 48.6 | 83.1 |
| R-SBL2 | 68.4 | 85.6 |
| R-SBL3 | 38.8 | 82.2 |
| R-SBL4 | 38.1 | 79.1 |
| Q-SBL5 | 58.6 | 67.1 |
| R-SBL5 | 62.3 | 72.7 |
| R-SBL6 | 55.6 | 69.8 |
| Q-SBL6 | 47.8 | 49.6 |
| Q-SBL7 | 57.8 | 59.0 |
| Q-SBL8 | 61.2 | 85.2 |
| Q-SBL9 | 76.2 | 96.9 |

### 5-2. Thermal stability

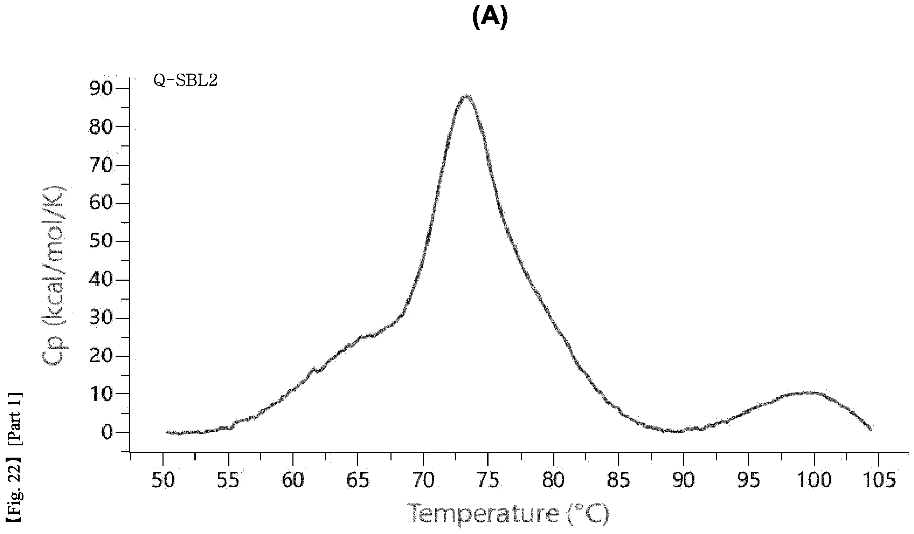

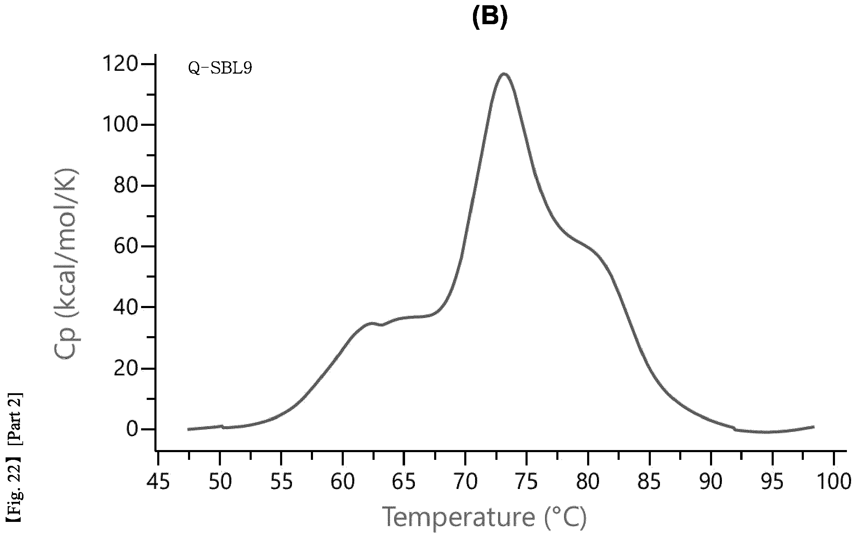

The thermal stability of the bispecific antibody candidates was measured using differential scanning calorimetry (Microcal PEAQ-DSC Automated, Malvern). At this time, the protein concentration used for measurement was up to 1 mg/mL. The sample was heated from 25°C to 110°C at a rate of 200°C/hr. Normalized heat capacity (Cp) data were calibrated with respect to the buffer solution baseline. Data were analyzed with Microcal PEAQ-DSC Automated software version 1.60 provided by the manufacturer. The melting point (Tm) was used to determine the temperature stability of the bispecific antibody under 50 mM acetate pH 5.0 conditions. This is data of the thermal stability of the bispecific antibody candidate group. Only sample groups having a purity of 90% or higher based on the result of SEC analysis were tested. FIG. 22 shows the results of DSC analysis of Q-SBL2 and Q-SBL9 as representative examples.

**[Table 29] DSC measurement of bispecific antibodies**

| Sample ID | Tonset (°C) | Tm1 (°C) | Tm2 (°C) |
|---|---|---|---|
| Q-SBL1 | 59.53 | 65.61 | 73.57 |
| Q-SBL2 | 55.37 | 63.99 | 73.70 |
| Q-SBL3 | 59.49 | 65.39 | 73.37 |
| Q-SBL4 | 59.17 | 65.35 | 73.30 |
| R-SBL1 | 57.09 | 62.71 | 73.80 |
| R-SBL2 | 56.46 | 62.37 | 73.73 |
| R-SBL3 | 56.87 | 62.14 | 73.71 |
| R-SBL4 | 57.69 | 62.40 | 73.86 |
| Q-SBL5 | 52.70 | 61.86 | 73.22 |
| R-SBL5 | 52.94 | 61.14 | 73.31 |
| R-SBL6 | 51.66 | 60.29 | 73.28 |
| Q-SBL6 | 54.75 | 61.36 | 74.16 |
| Q-SBL7 | 55.19 | 62.07 | 73.97 |
| Q-SBL8 | 56.68 | 61.74 | 74.00 |
| Q-SBL9 | 53.82 | 63.47 | 73.03 |

### 5-3. Dual Antigen Binding ELISA

The specific ELISA process is as follows. A 96-well high-adsorption ELISA plate was coated with rhVEGF 165 (R&D Systems) using 1x PBS pH 7.4 and the coating concentration was 0.5 µg/ml (100 ul/well). Coating was performed overnight at 4°C and washed 5 times with 0.05% PBS-T. The result was blocked with 200 pl/well of 2% BSA, incubated at 37°C for 2 hours, and washed 5 times with 0.05% PBS-T. Equal amounts of the bispecific antibody serially diluted (10 to 0.0005 µg/ml) in 2% BSA and rhHER2-his (R&D Systems) diluted to 1 µg/ml were mixed and incubated at 37°C for 1 hour. The microplate was washed 5 times with 0.05% PBS-T, 100 pl of the mixture of the heterodimeric antibody and rhHER2-his sample was added to each well and incubated at 37°C for 2 hours, followed by washing 5 times with 0.05% PBS-T. Then, HRP-conjugated anti-his antibody (Abeam) diluted 1:10000 with PBS containing 2% BSA was added in an amount of 100 pl/well, incubated at 37°C for 1 hour, and washed 5 times with 0.05% PBS-T. The colorimetric substrate TMB (Bio-Rad) was added at 100 pl/well and was allowed to develop color at room temperature for 5 minutes. 1M H₂SO₄ was added at 100 pl/well and color development was terminated. Absorbance was measured at a wavelength of 450 nm using a SpectraMax ABS Plus (Molecular Devices) instrument. The results of the comparison in EC₅₀ between Q-SBL1, Q-SBL2, Q-SBL3, and Q-SBL4 and of the comparison in EC₅₀ between R-SBL1, R-SBL2, R-SBL3, and R-SBL4 are as follows. The EC₅₀ of Q-SBL5, R-SBL5, and R-SBL6 with changes in the hinge region, compared to Q-SBL1 and R-SBL1, are as follows. Finally, the result of comparison in EC₅₀ between Q-SBL6, Q-SBL7, and Q-SBL8, which are candidates depending on the location of the knob/hole, and Q-SBL1, is shown as follows. Only sample groups having a purity of 90% or higher based on the result of SEC analysis were tested. FIGS. 14A and 14B are 4-parameter fitting graphs of the dual antigen binding affinity assay.

**[Table 30] EC₅₀ of bispecific antibodies in dual antigen binding ELISA**

| Sample ID | EC₅₀ (pg/ml) | R² |
|---|---|---|
| Q-SBL1 | 0.018 | 0.996 |
| Q-SBL2 | 0.019 | 1.000 |
| Q-SBL3 | 0.019 | 0.996 |
| R-SBL1 | 0.019 | 0.992 |
| R-SBL2 | 0.016 | 0.999 |
| R-SBL3 | 0.020 | 0.996 |
| Q-SBL5 | 0.017 | 0.996 |
| R-SBL5 | 0.016 | 0.998 |
| R-SBL6 | 0.017 | 0.996 |
| Q-SBL6 | 0.026 | 0.997 |
| Q-SBL7 | 0.023 | 0.999 |
| Q-SBL8 | 0.017 | 0.999 |
| Q-SBL9 | 0.029 | 0.999 |

### 5-4. HUVEC proliferation assay

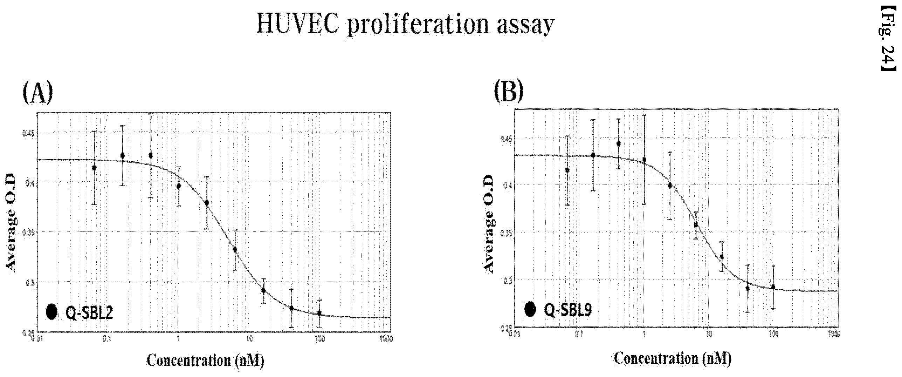

In order to determine the cell proliferation inhibitory effect of the bispecific antibodies, human umbilical vein endothelial cells (HUVECs) were purchased from Lonza and used in experiments. HUVEC (Lonza) cell culture was performed using EBM-2 (Lonza) containing EGM-2 Single Quot (Lonza) and the HUVEC cells used for test were cells of passage 5 or less. The cells were subcultured in a 37°C, 5% CO₂ incubator and the cell confluence was controlled within 80% in a 25-T flask. To analyze the proliferation inhibitory effect of vascular endothelial cells, the vascular endothelial cells were cultured in EBM-2 medium containing 0.25% FBS (Lonza) at a density of 4,000 cells/well on a 96-well plate for 6 hours. Antibodies of various concentrations were pre-treated with VEGF on a 96-well plate and allowed to react at room temperature for 15 minutes. The culture medium in the 96-well plate containing HUVEC cells was replaced with the EBM-2 culture medium containing 0.25% fetal calf serum. Then, each plate well was treated with various concentrations of antibody and 20 ng/ml of VEGF. After culturing for 70 hours, the result was treated with WST-8 (DOJINDO) for 5 hours and absorbance was measured at a wavelength of 450 nm to compare the degree of cell proliferation under respective conditions (FIG. 24).

**[Table 31] IC₅₀ of bispecific antibodies in HUVEC proliferation assay**

| Sample ID | IC₅₀ (nM) |
|---|---|
| Q-SBL1 | 5.347 |
| Q-SBL2 | 5.014 |
| Q-SBL3 | 3.472 |
| R-SBL1 | 6.535 |
| R-SBL2 | 5.032 |
| R-SBL3 | 7.772 |
| Q-SBL5 | 6.437 |
| R-SBL5 | 7.988 |
| R-SBL6 | 6.770 |
| Q-SBL9 | 5.481 |

When the cultured human vascular endothelial cell was treated with VEGF alone or in combination with a bispecific antibody, the growth rate of vascular endothelial cells decreased compared to the only VEGF treatment group, and nine bispecific antibodies excluding the bispecific antibody R-SBL3 had an IC₅₀ of 10 nM or less. All sample groups were tested only when the purity was 90% or higher based on the result of SEC analysis.

### 5-5. C1q binding assay

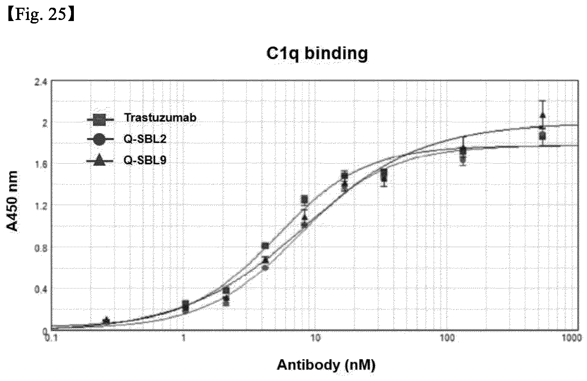

The Fc region of IgG1 interacts with Fcγ receptors (FcγR, Fcγ Receptor) and complement proteins (C1q, Complement component 1q) to induce an immune effector function. This plays an important role in increasing the efficacy by removing target cells through antibody-dependent cell-mediated cytotoxicity (ADCC), antibody-dependent cellular phagocytosis (ADCP), or complement-dependent cytotoxicity (CDC). Therefore, an ELISA experiment was conducted to determine the C1q binding activity of the bispecific antibody candidate group including the IgG1 backbone. The specific ELISA process was as follows. A 96-well high-adsorption ELISA plate was coated with 100 pl/well of the bispecific antibody serially diluted in 1xPBS pH 7.4 (540.5 - 0.3 nM) . Coating was performed overnight at 4°C and washed 5 times with 0.05% PBS-T. The result was blocked with 200 pl/well of 5% BSA (PBS), incubated at 25°C for 2 hours, and washed 5 times with 0.05% PBS-T. The C1q protein was diluted to 5 µg/ml with 5% BSA (PBS-T), 100 pl of the result was added to each well and incubated at 25°C for 2 hours. The microplate was washed 5 times with 0.05% PBS-T, HRP-conjugated anti-his antibody (Abeam) diluted 1:2000 with PBS containing 2% BSA was added in an amount of 100 pl/well, and incubated at 25°C for 1 hour. The result was washed 5 times with 0.05% PBS-T and the colorimetric substrate TMB (Bio-Rad) was added at 100 pl/well and allowed to develop color for 5 minutes at room temperature. 1M H₂SO₄ was added at 100 pl/well and color development was terminated. Absorbance was measured at a wavelength of 450 nm using a SpectraMax ABS Plus (Molecular Devices) instrument. The result of comparison in EC₅₀ between Trastuzumab, Q-SBL2, and Q-SBL9 is as follows. Only sample groups having a purity of 90% or higher based on the result of SEC analysis were tested. FIG. 25 is a 4-parameter fitting graph of the C1q binding ELISA.

**[Table 32] EC₅₀ of bispecific antibodies in C1q binding ELISA**

| **Sample ID** | **EC₅₀ (nM)** | **R²** |
|---|---|---|
| Trastuzumab | 5.115 | 0.991 |
| Q-SBL2 | 7.289 | 0.990 |
| Q-SBL9 | 8.270 | 0.987 |

Although specific configurations of the present invention have been described in detail, those skilled in the art will appreciate that this description is provided to set forth preferred embodiments for illustrative purposes and should not be construed as limiting the scope of the present invention. Therefore, the substantial scope of the present invention is defined by the accompanying claims and equivalents thereto.

### [Industrial applicability]

The novel bispecific or multispecific antibody format according to the present invention is capable of simultaneously binding to two or more targets and of inhibiting or improving the activity of the desired targets, thus being highly effective in treating or diagnosing diseases compared to a single-target antibody.

In addition, the novel bispecific or multispecific antibody format according to the present invention forms a heterodimer in a state in which non-specific binding between heavy and light chains is rarely present and forms almost no homodimer. Therefore, the novel bispecific or multispecific antibody format is highly expressed in animal cells and the purification process thereof is not much different from that of monoclonal antibodies. The novel bispecific or multispecific antibody exhibits superior or comparable stability to general monoclonal antibodies. The bispecific antibody format is useful for development of various bispecific antibody drugs and the treatment of patients with complicated diseases.

## Claims

1. A bispecific antibody comprising:
a first arm binding to a first antigen and comprising VHl-CHa-Fcl and VLl-CLb; and
a second arm binding to a second antigen and comprising VH2-CH1-Fc2 and VL2-CL,
wherein the VH1 and the VH2 are each heavy-chain variable regions including the same or different antigen-binding regions,
the VL1 and VL2 are each light-chain variable regions including the same or different antigen-binding regions,
the CHa comprises i) an IgG heavy-chain constant region or an IgD heavy-chain constant region CH1, and an IgG heavy-chain constant region CH2 or CH3, or ii) an IgM heavy-chain constant region CH3,
the CLb comprises i) a CL1 including an IgG light-chain constant region λ or κ, and at least one selected from the group consisting of IgG heavy-chain constant regions CH1, CH2, and CH3, or ii) an IgM heavy-chain constant region CH3,
the CH1 is an IgG heavy-chain constant region CH1 and CL is an IgG light-chain constant region CL, and
the Fc1 of the first arm is linked to the Fc2 of the second arm to form a heavy-chain constant region dimer.

2. The bispecific antibody according to claim 1, wherein the CHa and the CLb are derived from IgG1, IgG2, IgG3, IgG4, IgD or IgM.

3. The bispecific antibody according to claim 1, wherein, in the first arm, the CHa comprises a heavy-chain constant region CH1 derived from IgG1, IgG2, IgG3, IgG4, IgD or IgM, and a heavy-chain constant region CH3 derived from IgG1, IgG2, IgG3, IgG4, IgD or IgM in order from an N-terminus to a C-terminus.

4. The bispecific antibody according to claim 1, wherein, in the first arm, the CHa comprises a heavy-chain constant region CH3 derived from IgG1, IgG2, IgG3, IgG4, IgD or IgM, and a heavy-chain constant region CH1 derived from IgG1, IgG2, IgG3, IgG4, IgD or IgM in order from the N-terminus to the C-terminus.

5. The bispecific antibody according to claim 1, wherein the CHa of the first arm comprises a heavy-chain constant region CH3 derived from IgM.

6. The bispecific antibody according to claim 1, wherein, in the first arm, the CLb comprises a light-chain constant region CL1 derived from IgG1, IgG2, IgG3, IgG4, IgD or IgM, and a heavy-chain constant region CH3 derived from IgG1, IgG2, IgG3, IgG4, IgD or IgM in order from the N-terminus to the C-terminus.

7. The bispecific antibody according to claim 1, wherein, in the first arm, the CLb comprises a CH3 derived from IgG1, IgG2, IgG3, IgG4, IgD or IgM, and a CL1 derived from IgG1, IgG2, IgG3, IgG4, IgD or IgM in order from the N-terminus to the C-terminus.

8. The bispecific antibody according to claim 1, wherein, in the first arm, the CLb comprises a heavy-chain constant region CH3 derived from IgM.

9. The bispecific antibody according to claim 1, wherein the CH1 of CHa and CL1 of CLb are linked without a disulfide bond.

10. The bispecific antibody according to claim 1, wherein the CHa and CLb each comprise CH3 and the CH3 forms a dimer through a disulfide bond.

11. The bispecific antibody according to claim 1, wherein the CHa and CLb each comprise CH3, and one of the dimers formed by bonding CH3 comprises at least one selected from the group consisting of Y349C, S354C, T366S, T366W, L368A, and Y407V, and comprises at least one selected from the group consisting of S354C, Y349C, T366W, T366S, L368A and Y407V, to form a knob-in-hole structure.

12. The bispecific antibody according to claim 1, wherein the CH1 of CHa and the CL1 of CLb are linked without a disulfide bond, and i) any one of CH3 of CHa and CH3 of CLb comprises at least one selected from the group consisting of Y349C, T366S, L368A and Y407V, and the other comprises S354C and/or T366W, or ii) any one of CH3 of CHa and CH3 of CLb comprises at least one selected from the group consisting of S354C, T366S, L368A and Y407V, and the other comprises Y349C and/or T366W.

13. The bispecific antibody according to claim 1, wherein one of the CH3 dimers of the Fc comprises at least one selected from the group consisting of Y349C, S354C, T366S, T366W, L368A, and Y407V, and at least one selected from the group consisting of S354C, Y349C, T366W, T366S, L368A and Y407V, to form a knob-in-hole structure in the dimer.

14. A bispecific antibody comprising:
a first arm binding to a first antigen and comprising VH1-CHa-Fc1 and VL1-CLb; and
a second arm binding to a second antigen and comprising VH2-CH1-Fc2 and VL2-CL,
wherein the VH1 and the VH2 are each heavy-chain variable regions including the same or different antigen-binding regions,
the VL1 and VL2 are each light-chain variable regions including the same or different antigen-binding regions,
the CHa comprises an IgG heavy-chain constant region CH3 and an IgG heavy-chain constant region CH1,
the CLb comprises CL1 including an IgG light-chain constant region λ or κ, and an IgG heavy-chain constant region CH3,
the CH1 is an IgG heavy-chain constant region CH1 and CL is an IgG light-chain constant region CL, and
the Fc1 of the first arm is linked to the Fc2 of the second arm to form a heavy-chain constant region dimer.

15. The bispecific antibody according to claim 14, wherein the IgG heavy-chain constant region CH1 is derived from IgG1, IgG2, IgG3, IgG4, IgD or IgM.

16. The bispecific antibody according to claim 14, wherein the CH1 of CHa and CL1 of CLb are linked without a disulfide bond.

17. The bispecific antibody according to claim 14, wherein the CH3 of CHa and CL3 of CLb form a dimer through a disulfide bond.

18. The bispecific antibody according to claim 14, wherein one of the dimers formed by bonding the CH3 of CHa to the CH3 of CLb comprises at least one selected from the group consisting of T366W, S354C, and Y349C, and the other comprises at least one selected from the group consisting of S354C, Y349C, T366S, L368A, and Y407V, to form a knob-in-hole structure.

19. The bispecific antibody according to claim 14, wherein one of the dimers formed by bonding the CH3 of CHa to the CH3 of CLb comprises at least one selected from the group consisting of S354C, T366S, L368A, and Y407V, and the other comprises Y349C and T366W, to form a knob-in-hole structure.

20. The bispecific antibody according to claim 14, wherein the CH3 of CHa or CH3 of CLb comprises a sequence represented by SEQ ID NOS. 8 to 13.

21. The bispecific antibody according to claim 14, wherein the CH1 of CHa and the CL1 of CLb are linked without a disulfide bond, and i) any one of the CH3 of CHa and CH3 of CLb comprises at least one selected from the group consisting of Y349C, T366S, L368A and Y407V, and the other comprises S354C and/or T366W, or ii) any one of the CH3 of CHa and CH3 of CLb comprises at least one selected from the group consisting of S354C, T366S, L368A and Y407V, and the other comprises Y349C and/or T366W.

22. The bispecific antibody according to claim 14, wherein the CH3 and CH1 of Cha, and CH3 and CL1 of CLb are linked via a linker.

23. The bispecific antibody according to claim 22, wherein the linker has a 5 to 10 aa residue.

24. The bispecific antibody according to claim 22, wherein monomers of the CH3 dimer formed by the Fc1 of first arm and the Fc2 of second arm are linked through a disulfide bond or without a disulfide bond.

25. The bispecific antibody according to claim 14, wherein the first arm and the second arm are linked via a hinge.

26. The bispecific antibody according to claim 14, wherein the first arm and the second arm are linked through a hinge including one or more sequences selected from the group consisting of:
DKTHTCPPCP;
EPKSSDKTHTCPPCP; and
ESKYGPPCPPCP.

27. A multispecific antibody comprising the bispecific antibody according to any one of claims 1 to 26.

28. The multispecific antibody according to claim 27, wherein an antigen-binding fragment binding to an additional antigen is further included at an N-terminus of the first arm or the second arm in the bispecific antibody or an antigen-binding fragment binding to an additional antigen is further included at an Fc1 terminus of the first arm or an Fc2 terminus of the second arm in the bispecific antibody.
